(19) Europäisches Patentamt
European Patent Office
Office européen des brevets

(11) EP 2 272 867 A1

(12) **EUROPEAN PATENT APPLICATION**
published in accordance with Art. 153(4) EPC

(43) Date of publication:
**12.01.2011 Bulletin 2011/02**

(21) Application number: **09718914.6**

(22) Date of filing: **10.03.2009**

(51) Int Cl.:
***C07K 14/795*** *(2006.01)*

(86) International application number:
**PCT/JP2009/054569**

(87) International publication number:
**WO 2009/113551 (17.09.2009 Gazette 2009/38)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **11.03.2008 JP 2008061373**

(71) Applicant: **Osaka University**
**Suita-shi**
**Osaka 565-0871 (JP)**

(72) Inventors:
- **HAYASHI, Takashi**
  **Sulta-shi, Osaka 565-0871 (JP)**
- **KITAGISHI, Hiroaki**
  **Sulta-shi, Osaka 565-0871 (JP)**
- **OOHORA, Koji**
  **Sulta-shi, Osaka 565-0871 (JP)**
- **ONODA, Akira**
  **Sulta-shi, Osaka 565-0871 (JP)**
- **KAKIKURA, Yasuaki**
  **Sulta-shi, Osaka 565-0871 (JP)**

(74) Representative: **Matthews, Derek Peter**
**Dehns**
**St Bride's House**
**10 Salisbury Square**
**London EC4Y 8JD (GB)**

# (54) PROTEIN MONOMER, PROTEIN POLYMER OBTAINED FROM SAID MONOMER, AND DEVICE THAT CONTAINS THEM

(57) A protein polymer having a larger molecular weight is provided by regularly arranging a protein having a large molecular weight. The protein polymer having a large molecular weight can be obtained using a protein monomer represented by formula (I) or a salt thereof

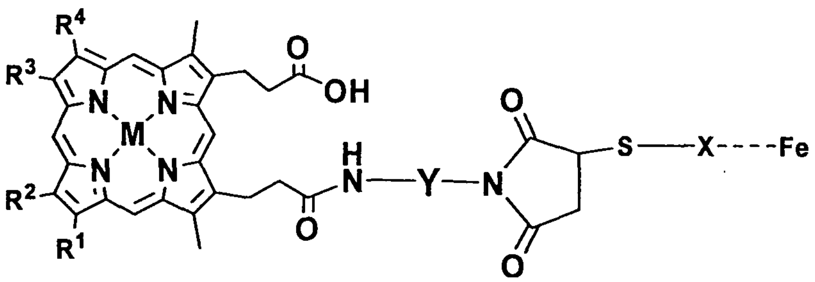

(I)

wherein $R^1$, $R^2$, $R^3$, $R^4$, Y, and X are as defined in the specification.

EP 2 272 867 A1

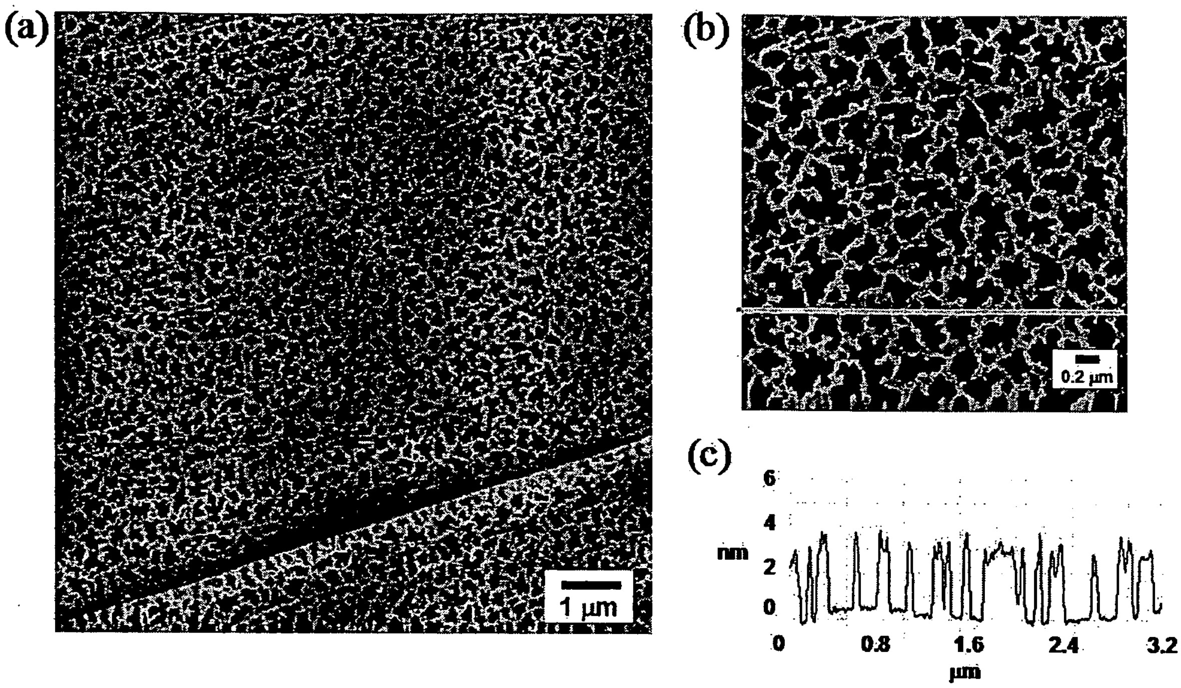


FIG. 6

## Description

Technical Field

The present invention relates to a protein monomer and a protein polymer obtained from the monomer. More specifically, the present invention relates to a protein monomer, a protein polymer having the monomer as a monomer unit, and a device containing them.

Background Art

Production of large-molecularweight organic compounds have been attempted conventionally by regularly arranging an small-molecular-weight organic compound using various interactions such as coordination bond, covalent bond, and ionic bond (see, for example, Non-Patent Document 1). It is hoped that a large-molecularweight organic compound produced in such a manner will be used as a multi-function nanodevice.

It is, however, very difficult to arrange regularly a large-molecular-weight organic compound, such as protein, using coordination bond, covalent bond, ionic bond, or other interactions and produce an organic compound that has an even larger molecular weight. The difficulty lies in the two points; 1) that it is difficult to chemically and suitably modify the functional group on the surface of a protein that has a higher-order structure and 2) that it is difficult to develop a system that allows such chemically modified proteins to interact with each other.

Non-Patent Document 1: J. M. Lehn, "Supramolecular Chemistry: Concepts and Perspectives" VCH Publication, Weinheim, Germany, 1995

Disclosure of the Invention

Problems to Be Solved by the Invention

In this regard, an object of the present invention is to provide a protein polymer that has an even larger molecular weight obtained by regularly arranging a protein that has a large molecular weight.

Means for Solving Problem

The present invention is directed to a protein monomer represented by formula (I) or a salt thereof.

[Chemical Formula 9]

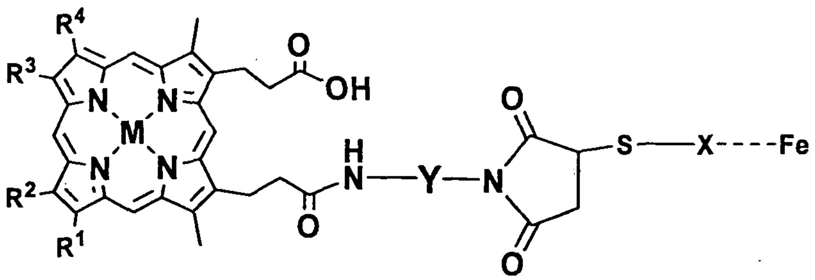

(I)

In formula (I) above,

$R^1$, $R^2$, $R^3$, and $R^4$ each independently represent a hydrogen atom, a lower alkyl group, a halogen-substituted lower alkyl group, or a lower alkenyl group; Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;

M is selected from the group consisting of Fe, Zn, and Co; and

X represents X that is present in a hemoprotein mutant, and the hemoprotein mutant is represented by the formula (V).

[Chemical Formula 10] HS-X----Fe (V)

The mutant is a protein that has the same amino acid sequence as a native hemoprotein except that one amino acid residue is replaced with a cysteine residue. In the mutant, -SH represents a side-chain thiol group of the cysteine residue, and Fe is bonded to the four nitrogen atoms of a porphyrin group contained in the hemoprotein.

The present invention also is directed to a protein monomer represented by formula (II) or a salt thereof.

[Chemical Formula 11]

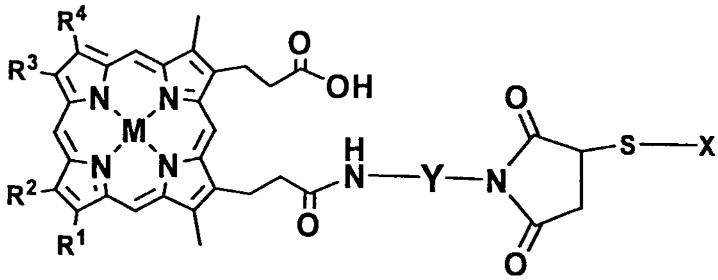

(II)

In formula (II) above,
$R^1$, $R^2$, $R^3$, and $R^4$ each independently represent a hydrogen atom, a lower alkyl group, a halogen-substituted lower alkyl group, or a lower alkenyl group;
Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
X represents X that is present in a hemoprotein mutant, and the hemoprotein mutant is represented by the formula (V).

[Chemical Formula 12] HS-X----Fe (V)

The mutant is a protein that has the same amino acid sequence as a native hemoprotein except that one amino acid residue is replaced with a cysteine residue. In the mutant, -SH represents a side-chain thiol group of the cysteine residue, and Fe is bonded to the four nitrogen atoms of a porphyrin group contained in the hemoprotein.

The present invention also is directed to a protein polymer or a salt thereof that contains as a monomer unit the protein monomer represented by formula (II) or the salt thereof.

The protein polymer represented by formula (I) or the salt thereof as well as the protein monomer represented by formula (II) or the salt thereof are of use as monomers for the production of protein polymers.

Effects of the Invention

The protein monomer or the salt thereof of the present invention has a large molecular weight. Regularly arranging the protein monomer or the salt thereof using a heme (including one that has Zn in place of Fe) allows a protein polymer that has a larger molecular weight to be produced.

Brief Description of Drawings

[Fig. 1] Fig. 1a shows a UV-vis spectrum of native myoglobin.
[Fig. 1b] Fig. 1b shows a UV-vis spectrum of the protein polymer (III-4) produced in Example 4.
[Fig. 1c] Fig. 1c shows a UV-vis spectrum of the protein polymer (III-3) produced in Example 3.
[Fig. 1d] Fig. 1d shows a UV-vis spectrum of the protein polymer (III-5) produced in Example 5.
[Fig. 1e] Fig. 1e shows a UV-vis spectrum of an oxygen complex of native myoglobin.
[Fig. 1f] Fig. 1f shows a UV-vis spectrum of an oxygen complex of the protein polymer (III-5) produced in Example 5.
[Fig. 2a] Fig. 2a shows size exclusion chromatograms of the protein polymers obtained in Examples 3 to 5.
[Fig. 2b] Fig. 2b shows size exclusion chromatograms of the protein polymer (III-1) obtained in Example 1 and the protein polymer (III-1) obtained in Example 2.

[Fig. 2c] Fig. 2c shows a size exclusion chromatogram of the protein polymer (III-6) obtained in Example 10.
[Fig. 3] Fig. 3 shows a size exclusion chromatogram of the protein polymer (III-5) obtained in Example 5 measured under various concentrations.
[Fig. 4a] Fig. 4a shows an AFM image of the protein polymer (III-4) obtained in Example 4.
[Fig. 4b] Fig. 4b shows an AFM image of the protein polymer (III-3) obtained in Example 3.
[Fig. 4c] Fig. 4c shows an AFM image of the protein polymer (III-5) obtained in Example 5.
[Fig. 4d] Fig. 4d shows an AFM image of the protein polymer (III-2) obtained in Example 2.
[Fig. 5] Fig. 5(a) shows an ESI-TOF-MS spectrum of the protein monomer (II-5) obtained in Example 4 and Fig. 5 (b) shows a deconvoluted ESI-TOF-MS spectrum of the protein polymer (II-5). Fig. 5(c) shows an ESI-TOF-MS spectrum of the protein monomer (II-4) obtained in Example 3 and Fig. 5(d) shows a deconvoluted ESI-TOF-MS spectrum of the protein monomer (II-4). Fig. 5(e) shows an ESI-TOF-MS spectrum of the protein monomer (II-3) obtained in Example 5 and Fig. 5(f) shows a deconvoluted ESI-TOF-MS spectrum of the protein monomer (II-3).
[Fig. 6] Fig. 6 shows AFM images of the assembly obtained in Example 6.
[Fig. 7] Fig. 7 shows anAFM image of the assembly obtained in Example 7.
[Fig. 8] Fig. 8 shows an AFM image of the assembly obtained in Example 8.
[Fig. 9] Fig. 9 shows AFM images of the assembly obtained in Example 9.
[Fig. 10] Fig. 10 shows an AFM image of compound (IV-1) (triad).
[Fig.11(a)] Fig. 11 (a) shows a cyclic voltammogram of hemin-modified gold electrode (A).
[Fig. 11(b)] Fig. 11 (b) shows a cyclic voltammogram of protein monomer-modified gold electrode (B).
[Fig. 11(c)] Fig. 11 (c) shows a cyclic voltammogram of protein polymer-modified gold electrode (C).
[Fig. 12(a)] Fig. 12(a) shows cyclic voltammograms of hemin-modified gold electrode (A) obtained at different scan rates.
[Fig. 12(b)] Fig. 12(b) shows cyclic voltammograms of protein monomer-modified gold electrode (B) obtained at different scan rates.
[Fig. 12(c)] Fig. 12(c) shows cyclic voltammograms of protein polymer-modified gold electrode (C) obtained at different scan rates.
[Fig. 12(d)] Fig. 12(d) shows cyclic voltammograms of hemin-modified gold electrode (D) obtained at different scan rates.
[Fig. 12(e)] Fig. 12(b) shows cyclic voltammograms of protein polymer-modified gold electrode (E) obtained at different scan rates.
[Fig. 13(a)] Fig. 13(a) is a graph showing the peak current obtained using hemin-modified gold electrode (A).
[Fig. 13(b)] Fig. 13(b) is a graph showing the peak current obtained using protein monomer-modified gold electrode (B).
[Fig. 13(c)] Fig. 13(c) is a graph showing the peak current obtained using protein polymer-modified gold electrode (C).
[Fig. 13(d)] Fig. 13(d) is a graph showing the peak current obtained using hemin-modified gold electrode (D).
[Fig. 13(e)] Fig. 13(e) is a graph showing the peak current obtained using protein polymer-modified gold electrode (E).
[Fig. 14(a)] Fig. 14(a) shows the results of DPV obtained using hemiri-modified gold electrode (A).
[Fig. 14(b)] Fig. 14(b) shows the results of DPV obtained using protein monomer-modified gold electrode (B).
[Fig. 14(c)] Fig. 14(c) shows the results of DPV obtained using protein polymer-modified gold electrode (C).
[Fig. 14(d)] Fig. 14(d) shows the results of DPV obtained using hemin-modified gold electrode (D).
[Fig. 14(e)] Fig. 14(e) shows the results of DPV obtained using protein polymer-modified gold electrode (E).
[Fig. 15] Fig. 15 shows the resistances of hemin-modified gold electrode (A), protein monomer-modified gold electrode (B), and protein polymer-modified gold electrode (C).

Best Mode of Carrying Out the Invention

The present invention is directed to, as stated above, the protein monomer represented by formula (I) or the salt thereof.

The present invention also is directed to, as stated above, the protein monomer represented by formula (II) or the salt thereof.

The present invention also is directed to, as stated above, a protein polymer or a salt thereof that contains as a monomer unit the protein monomer represented by formula (II) or the salt thereof. Note that the protein monomer represented by formula (II) or the salt thereof constituting the protein polymer or the salt thereof may be a single kind, a mixture of two or more kinds, or a mixture with positional isomers. Furthermore, the protein polymer is preferably a random protein polymer represented by formula (III).

[Chemical Formula 13]

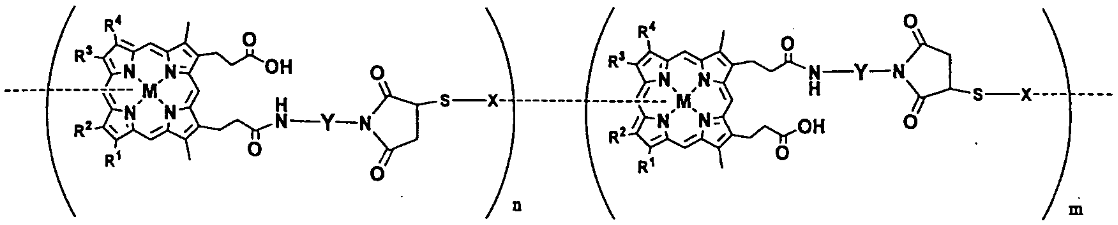

(III)

In formula (III) above,
$R^1$, $R^2$, $R^3$, and $R^4$ each independently represent a hydrogen atom, a lower alkyl group, a halogen-substituted lower alkyl group, or a lower alkenyl group; Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
X represents X that is present in a hemoprotein mutant, and the hemoprotein mutant is represented by the formula (V).

[Chemical Formula 14] HS-X----Fe (V)

The mutant is a protein that has the same amino acid sequence as a native hemoprotein except that one amino acid residue is replaced with a cysteine residue. In the mutant, -SH represents a side-chain thiol group of the cysteine residue, and Fe is bonded to the four nitrogen atoms of a porphyrin group contained in the hemoprotein.

The present invention also is directed to a protein assembly or a salt thereof containing a triad represented by formula (IV) or a salt thereof and the protein monomer represented by formula (II) or the salt thereof. The protein monomer represented by formula (II) or the salt thereof may be a single kind, a mixture of two or more kinds, or a mixture with positional isomers. In addition, the protein monomer represented by formula (II) or the salt thereof may be in the form of a protein polymer when considered as a monomer unit. Moreover, the protein assembly may contain one or more triads.

[Chemical Formula 15]

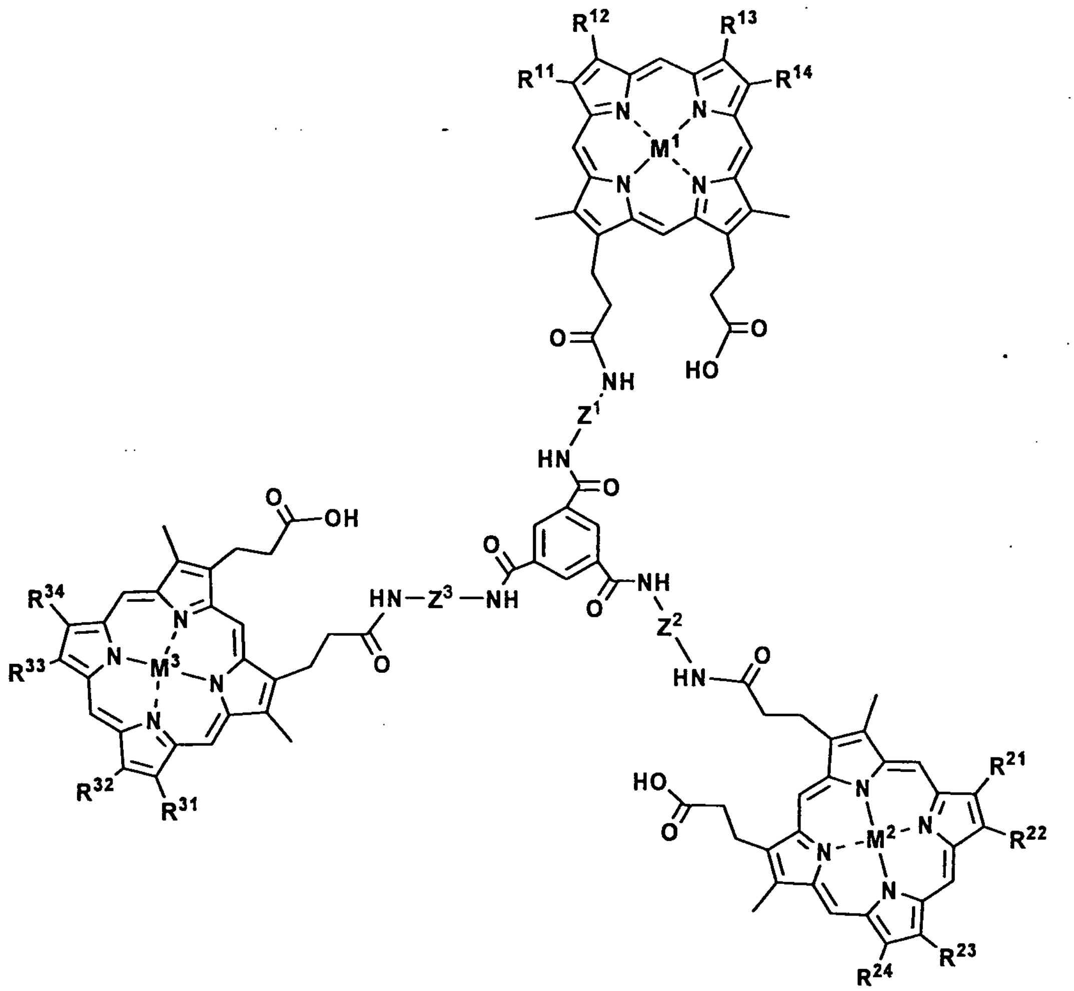

(IV)

In formula (IV) above,

$R^{11}$, $R^{12}$, $R^{13}$, $R^{14}$, $R^{21}$, $R^{22}$, $R^{23}$, $R^{24}$, $R^{31}$,$R^{32}$, $R^{33}$, and $R^{34}$ each independently represent a hydrogen atom, a lower alkyl group, a halogen-substituted lower alkyl group, or a lower alkenyl group;

$M^1$, $M^2$, and $M^3$ are each independently selected from the group consisting of Fe, Zn, and Co; and

$Z^1$, $Z^2$, and $Z^3$ each independently represent a group represented by a formula $-(CH_2)_{m1}-$, $-(CH_2)_{m1}-O-(CH_2)_{m2}-$, $-(CH_2)_{m1}-O-(CH_2)_{m2}-O-(CH_2)_{m3}-$, or $-(CH_2)_{m1}-O-(CH_2)_{m2}-O-(CH_2)_{m3}-O-(CH_2)_{m4}-$, wherein m1, m2, m3, and m4 each independently represent an integer of 1 to 20.

The present invention also is directed to a nanosheet containing the protein assembly or the salt thereof.

The present invention also is directed to a device containing at least one selected from the group consisting of a protein monomer, a protein monomer salt, a protein polymer, a protein polymer salt, a protein assembly, and a protein assembly salt. The protein monomer or the protein monomer salt is the protein monomer represented by formula (I) or the salt thereof of the present invention or the protein monomer represented by formula (II) or the salt thereof of the present invention; the protein polymer or the protein polymer salt is a protein polymer containing the protein monomer represented by formula (II) or the salt thereof as a monomer unit, or is the protein polymer represented by formula (III) or the salt thereof of the present invention; and the protein assembly or the protein assembly salt is the protein assembly or the protein assembly salt of the present invention.

The present invention also is directed to a substrate modified with at least one selected from the group consisting of a protein monomer, a protein monomer salt, a protein polymer, and a protein polymer salt. The protein monomer or the protein monomer salt is the protein monomer represented by formula (II) (provided that M is Fe in formula (II)) or the salt thereof of the present invention; and the protein polymer or the protein polymer salt is a protein polymer containing the protein monomer represented by formula (II) (provided that M is Fe in formula (II)) or the salt thereof as a monomer unit, or the protein polymer represented by formula (III) (provided that M is Fe in formula (III)) or the salt thereof of the present invention.

In the present invention, the term "lower" indicates 1 to 6 carbon atoms and preferably 1 to 4 carbon atoms

unless specified otherwise.

The term "lower alkyl group" as found in the "lower alkyl group" and the "halogen-substituted lower alkyl group" refers to a residue of a linear or branched alkane having 1 to 6 carbon atoms, such as methyl, ethyl, propyl, isopropyl, butyl, isobutyl, t-butyl, pentyl, neopentyl and hexyl. Preferable examples of lower alkyl groups include alkyls having 1 to 5 carbon atoms. Preferable alkyls having 1 to 5 carbon atoms include methyl, ethyl, propyl, isopropyl, butyl, isobutyl, tert-butyl, pentyl, neopentyl, and the like.

The term "lower alkenyl group" refers to a linear or branched alkenyl group having 2 to 6 carbon atoms, such as vinyl, allyl, isopropenyl, 1-, 2-, or 3-butenyl, 1-, 2-, 3-, or 4-pentenyl, and 1-, 2-, 3-, 4-, or 5-hexenyl. A preferable lower alkenyl group is vinyl.

The term "halogen atom" includes fluorine, chlorine, bromine, and iodine, and fluorine is preferable.

An example of the "halogen-substituted lower alkyl group" is a lower alkyl group in which one or more hydrogen atoms of an aforementioned lower alkyl group are replaced with aforementioned halogen atoms. Preferable examples of such halogen-substituted lower alkyl groups include methyl iodide, dichloromethyl, trichloromethyl, and trifluoromethyl. A preferable halogen-substituted lower alkyl group is trifluoromethyl.

The monomer, the polymer, the assembly, and the salts thereof of the present invention may take solvate forms, and such solvate forms are also encompassed within the scope of the invention. Solvates preferably include hydrates and ethanolic solvates.

M contained in the monomer, the polymer, the assembly, and the salts thereof of the present invention is, as stated above, selected from the group consisting of Fe, Zn, and Co. When M is Fe, M includes $Fe^{2+}$ and $Fe^{3+}$; when M is Zn, M includes $Zn^{2+}$; and when M is Co, M includes $Co^{2+}$ and $Co^{3+}$.

In the protein monomer represented by formula (I) or the salt thereof, the protein monomer represented by formula (II) or the salt thereof, and the protein polymer represented by formula (III) or the salt thereof, the protein is not particularly limited as long as it is a protein having one or more hemes (including those in which Zn is substituted for Fe), and examples include cytochrome, hemoglobin, myoglobin, and peroxidase. Examples of cytochrome include cytochrome $b_{562}$, cytochrome $b_5$, and cytochrome $P450_{CAM}$, and cytochrome $b_{562}$ is preferable. Examples of hemoglobin include hemoglobin (human), hemoglobin (bovine), hemoglobin (equine), and hemoglobin (rat), and hemoglobin (human) is particularly preferable. Examples of myoglobin include myoglobin (*sus scrofa*), myoglobin (equine), myoglobin (human), and myoglobin (sperm whale), and myoglobin (sperm whale) and myoglobin (*sus scrofa*) are preferable. Examples of peroxidase include horseradish peroxidase, chloroperoxidase, and catalase, and horseradish peroxidase is preferable.

As for the protein monomer represented by formula (I) or the salt thereof, it is preferable that in formula (I), $R^1$, $R^2$, $R^3$, and $R^4$ each independently represent a lower alkyl group or a lower alkenyl group;
Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20; and
M represents Fe or Zn.

In the present invention, the hemoprotein mutant represented by formula (V):

[Chemical Formula 16] HS-X----Fe (V)

is a protein that has the same amino acid sequence as the aforementioned hemoprotein except that one amino acid residue is replaced with a cysteine residue. The hemoprotein mutant refers, in the case of being a cytochrome, for example, to (a) a protein that has an amino acid sequence identical to SEQ ID NO. 1 except that one amino acid is replaced with cysteine, or (b) a protein that has an amino acid sequence identical to SEQ ID NO. 1 except that one amino acid is replaced with cysteine and one or two amino acids are deleted, replaced, or added and that functions as a cytochrome. Note that the amino acid sequence having SEQ ID NO.1 is an amino acid sequence of native cytochrome $b_{562}$. The amino acid sequence of native cytochrome $b_{562}$ is not limited to the amino acid sequence having SEQ ID NO. 1, and a suitable sequence may be selected from the Protein Data Bank. Specifically, an example is a sequence having ID NO. 1QPU (dated June 2, 1999), and the amino acid sequence having SEQ ID NO. 1 corresponds to that of ID NO. 1QPU. Said one amino acid residue replaced with cysteine is preferably an amino acid residue that is located on the exterior of the structure when the cytochrome is depicted in a three-dimensional configuration. In addition, it is necessary that the replacement with the cysteine residue does not affect the structure of the heme contained in the cytochrome. To attain such replacement, for example, in the amino acid sequence of SEQ ID NO. 1, one of the 1st to 106th amino acid residues, preferably one of the 60, 62, 63, 66, 67, 70, 73, 74, 76, 78, 89, 90, 96, 99, and 100th amino acid residues, and more preferably one of the 60, 63, 66, 67, and 100th amino acid residues may be replaced with a cysteine residue.

Moreover, the hemoprotein mutant refers, in the case of being a hemoglobin, for example, to (a) a protein that has an amino acid sequence identical to SEQ ID NO. 9 or 10 except that one amino acid is replaced with cysteine, or (b) a protein that has an amino acid sequence identical to SEQ ID NO. 9 or 10 except that one amino acid is replaced with cysteine and one or two amino acids are deleted, replacement, or added and that functions as a hemoglobin (human). The amino acid sequence having SEQ ID NO. 9 is an amino acid sequence of the $\alpha$-subunit of a native hemoglobin and

the amino acid sequence having SEQ ID NO. 10 is an amino acid sequence of the β-subunit of a native hemoglobin. The amino acid sequence of a native hemoglobin is not limited to the amino acid sequences having SEQ ID NOs. 9 and 10, and a suitable sequence may be selected from the Protein Data Bank. Specifically, an example is a sequence having ID NO. 1GZX (dated July 8, 2002), and the amino acid sequences having the aforementioned SEQ ID NOs. 9 and 10 correspond to that of ID NO. 1GZX. Said one amino acid residue replaced with cysteine is preferably an amino acid residue that is located on the exterior of the structure when the hemoglobin is depicted in a three-dimensional configuration. In addition, it is necessary that the replacement with the cysteine residue does not affect the structure of the heme contained in the hemoglobin.

Moreover, the hemoprotein mutant refers, in the case of being a myoglobin, for example, to (a) a protein that has an amino acid sequence identical to SEQ ID NO. 2 except that one amino acid is replaced with cysteine, or (b) a protein that has an amino acid sequence identical to SEQ ID NO. 2 except that one amino acid is replaced with cysteine and one or two amino acids are deleted, replaced, or added and that functions as a myoglobin. The amino acid sequence having SEQ ID NO. 2 is identical to an amino acid sequence of a wild-type myoglobin (sperm whale), but the amino acid sequence having SEQ ID NO. 2 further contains Met as an amino acid residue before Val that is the first amino acid residue in the native amino acid sequence (the amino acid sequence of a protein actually extracted from a whale). In addition, the 122nd amino acid residue of the native amino acid sequence, i.e., aspartic acid, corresponds to the 123rd amino acid residue of the amino acid sequence having the SEQ ID NO. 2, but the amino acid residue thereof is replaced with asparagine. The amino acid sequence of a wild-type myoglobin is not limited to the amino acid sequence having SEQ ID NO. 2, and a suitable sequence may be selected from the Protein Data Bank. Specifically, an example is the sequence having ID NO. 2MBW (dated June 20, 1996), and the amino acid sequence having SEQ ID NO. 2 corresponds to that of ID NO. 2MBW. Said one amino acid residue replaced with cysteine is preferably an amino acid residue that is located on the exterior of the structure when the myoglobin is depicted in a three-dimensional configuration. In addition, it is necessary that the replacement with the cysteine residue does not affect the structure of the heme contained in the myoglobin. To attain such replacement, for example, in the amino acid sequence of SEQ ID NO. 2, one of the 1st to 154th amino acid residues, preferably one of the 8, 9, 12, 13, 16, 17, 53, 54, 102, 103, 106, 107, 113, 114, 117, 118, 121, 122, 125, 126, 127, 133, 134, 140, 141, 147, and 148th amino acid residues, and more preferably one of the 125, 126, 133, and 134th amino acid residues may be replaced with a cysteine residue.

Moreover, the hemoprotein mutant refers, in the case of being a horseradish peroxidase, for example, to (a) a protein that has an amino acid sequence identical to SEQ ID NO. 3 except that one amino acid is replaced with cysteine, or (b) a protein that has an amino acid sequence identical to SEQ ID NO. 1 except that one amino acid is replaced with cysteine and one or two amino acids are deleted, replaced, or added and that has horseradish peroxidase activity. Note that the amino acid sequence having SEQ ID NO. 3 is an amino acid sequence of a native horseradish peroxidase. The amino acid sequence of a native horseradish peroxidase is not limited to the amino acid sequence having SEQ ID NO. 3, and a suitable sequence may be selected from the Protein Data Bank. Specifically, an example is the sequence having ID NO. 1ATJ (dated February 4, 1998), and the amino acid sequence having SEQ ID NO. 3 corresponds to that of ID NO. 1ATJ. Said one amino acid residue replaced with cysteine is preferably an amino acid residue that is located on the exterior of the structure when the horseradish peroxidase is depicted in a three-dimensional configuration. In addition, it is necessary that the replacement with the cysteine residue does not affect the structure of the heme contained in the horseradish peroxidase.

In the mutant, as stated above, -SH represents a side-chain thiol group of the cysteine residue, and Fe is bonded to the four nitrogen atoms of a porphyrin group contained in the hemoprotein.

As for the protein monomer represented by formula (I) or the salt thereof, it is more preferable that in formula (I), $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

Moreover, as for the protein monomer represented by formula (I) or the salt thereof, it is more preferable that in formula (I), $R^2$ and $R^4$ each independently represent a lower alkyl group and $R^1$ and $R^3$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

As for the protein monomer represented by formula (I) or the salt thereof, it is more preferable that in formula (I), $R^1$ and $R^3$ each independently represent a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Moreover, as for the protein monomer represented by formula (I) or the salt thereof, it is more preferable that

in formula (I), $R^2$ and $R^4$ each independently represent a methyl group and $R^1$ and $R^3$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Moreover, as for the protein monomer represented by formula (II) or the salt thereof, it is preferable that in formula (II), $R^1$, $R^2$, $R^3$, and $R^4$ each independently represent a lower alkyl group or a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20; and M represents Fe or Zn.

As for the protein monomer represented by formula (II) or the salt thereof, it is more preferable that in formula (II), $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

Moreover, as for the protein monomer represented by formula (II) or the salt thereof, it is more preferable that in formula (II), $R^2$ and $R^4$ each independently represent a lower alkyl group and $R^1$ and $R^3$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

As for the protein monomer represented by formula (II) or the salt thereof, it is more preferable that in formula (II), $R^1$ and $R^3$ each independently represent a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Moreover, as for the protein monomer represented by formula (II) or the salt thereof, it is more preferable that in formula (II), $R^2$ and $R^4$ each independently represent a methyl group and $R^1$ and $R^3$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Moreover, as for the protein polymer or the salt thereof containing a protein monomer or a salt thereof of the present invention as a monomer unit, it is preferable that the protein monomer is a protein monomer represented by formula (II) wherein $R^1$, $R^2$, $R^3$, and $R^4$ each independently represent a lower alkyl group or a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20; and M represents Fe or Zn.

As for the protein polymer or the salt thereof containing a protein monomer or a salt thereof of the present invention as a monomer unit; more preferable is a protein polymer or a salt thereof containing as a monomer unit at least one of

a protein monomer represented by formula (II) or a salt thereof wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin; and

a protein monomer represented by formula (II) or a salt thereof wherein $R^2$ and $R^4$ each independently represent a lower alkyl group and $R^1$ and $R^3$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

As for the protein polymer or the salt thereof containing a protein monomer or a salt thereof of the present invention as a monomer unit, it is more preferable that the protein monomer is a protein monomer represented by formula (II) wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

As for the protein polymer or the salt thereof containing a protein monomer or a salt thereof of the present

invention as a monomer unit, it is more preferable that the protein monomer is a protein monomer represented by formula (II) wherein $R^2$ and $R^4$ each independently represent a lower alkyl group and $R^1$ and $R^3$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

As for the protein polymer or the salt thereof containing a protein monomer or a salt thereof of the present invention as a monomer unit, more preferable is a protein polymer or a salt thereof containing as a monomer unit at least one of:

a protein monomer represented by formula (II) or a salt thereof wherein $R^1$ and $R^3$ each independently represent a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue; and

a protein monomer represented by formula (II) or a salt thereof wherein $R^2$ and $R^4$ each independently represent a methyl group and $R^1$ and $R^3$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

As for the protein polymer or the salt thereof containing a protein monomer or a salt thereof of the present invention as a monomer unit, it is more preferable that the protein monomer is a protein monomer represented by formula (II) wherein $R^1$ and $R^3$ each independently represent a methyl group and $R^2$ and $R^4$ each independently represent a vinyl group; Y is a group represented by a formula $-(CH_2)_2-$,- $(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Moreover, as for the protein polymer or the salt thereof containing a protein monomer or a salt thereof of the present invention as a monomer unit, it is more preferable that the protein monomer is a protein monomer represented by formula (II) wherein $R^2$ and $R^4$ each independently represent a methyl group and $R^1$ and $R^3$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; and the hemoprotein is a cytochrome that has an amino acid sequence having SEQ ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Moreover, as for the protein polymer represented by formula (III) or the salt thereof, preferable is a protein polymer or a salt thereof containing a protein monomer represented by formula (II) or a salt thereof as a monomer unit wherein $R^1$, $R^2$, $R^3$, and $R^4$ each independently represent a lower alkyl group or a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20; and M represents Fe or Zn.

Moreover, as for the protein polymer represented by formula (III) or the salt thereof, more preferable is a protein polymer or a salt thereof containing as a monomer unit at least one of

a protein monomer represented by formula (II) or a salt thereof wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin; and

a protein monomer represented by formula (II) or a salt thereof wherein $R^2$ and $R^4$ each independently represent a lower alkyl group and $R^1$ and $R^3$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

Moreover, as for the protein polymer represented by formula (III) or the salt thereof, more preferable is one or

more selected from the group consisting of:

a protein polymer or a salt thereof containing as a monomer unit a protein monomer represented by formula (II) or a salt thereof wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin; and

a protein polymer or a salt thereof containing as a monomer unit a protein monomer represented by formula (II) or a salt thereof wherein $R^2$ and $R^4$ each independently represent a lower alkyl group and $R^1$ and $R^3$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 3; M represents Fe or Zn; and the hemoprotein is a cytochrome or a myoglobin.

As for the protein polymer represented by formula (III) or the salt thereof, further preferable is a protein polymer or a salt thereof containing as a monomer unit at least one of:

a protein monomer represented by formula (II) or a salt thereof wherein $R^1$ and $R^3$ are each independently a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue; and

a protein monomer or a salt thereof represented by formula (II) wherein $R^2$ and $R^4$ are each independently a methyl group and $R^1$ and $R^3$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

As for the protein polymer represented by formula (III) or the salt thereof, further preferable is one or more selected from the group consisting of:

a protein polymer or a salt thereof containing as a monomer unit a protein monomer represented by formula (II) or a salt thereof wherein $R^1$ and $R^3$ are each independently a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue, and

a protein polymer or a salt thereof containing as a monomer unit a protein monomer or a salt thereof represented by formula (II) wherein $R^2$ and $R^4$ are each independently a methyl group and $R^1$ and $R^3$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Examples of the salt of the protein monomer represented by formula (I), the salt of the protein monomer represented by formula (II), the salt of the protein polymer represented by formula (III), the triad represented by formula (IV) or the salt thereof, and the protein assembly or the salt thereof include salts of bases or acids such as: salts of alkali metals such as sodium and potassium; salts of alkaline earth metals such as calcium and magnesium; salts of inorganic bases such as ammonium; salts of organic amines such as triethylamine, pyridine, picoline, ethanolamine, triethanolamine, dicyclohexylamine, and *N,N*-dibenzylethyleneamine; salts of inorganic acids such as hydrochloric acid, hydrobromic acid, sulfuric acid, and phosphoric acid; salts of organic carboxylic acid such as formic acid, acetic acid, trifluoroacetic acid, maleic acid, and tartaric acid; acid addition salts of sulfonic acids such as methanesulfonic acid, benzenesulfonic acid, and p-toluenesulfonic acid; and salts and acid addition salts of bases such as basic and acidic amino acids, e.g., arginine, aspartic acid, and glutamic acid, formed with the protein monomer represented by formula (I), the protein monomer represented by formula (II), the protein polymer represented by formula (III), the triad represented

by formula (IV), and the protein assembly.

As for the protein assembly or the salt thereof, preferable is a protein assembly or a salt thereof obtained by treating under a neutral condition one or more triads represented by formula (IV) or salts thereof wherein $R^{11}$, $R^{12}$, $R^{13}$, $R^{14}$, $R^{21}$, $R^{22}$, R23, $R^{24}$, $R^{31}$,$R^{32}$, $R^{33}$, and $R^{34}$ each independently represent a lower alkyl group or a lower alkenyl group; $Z^1$, $Z^2$, and $Z^3$ each independently represent a group represented by a formula $-(CH_2)_{m1}-$, $-(CH_2)_{m1}-O-(CH_2)_{m2}-O-(CH_2)_{m3}-$, or $-(CH_2)_{m1}-O-(CH_2)_{m2}-O-(CH_2)_{m3}-O-(CH_2)_{m4}-$ wherein m1, m2, m3, and m4 each independently represent an integer of 1 to 20; and $M^1$, $M^2$, and $M^3$ each independently represent Fe or Zn, with

one or more protein monomers represented by formula (II) or salts thereof wherein $R^1$, $R^2$, $R^3$, and $R^4$ each independently represent a lower alkyl group or a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$ wherein n1, n2, n3, and n4 each independently represent an integer of 1 to 20; and M represents Fe or Zn.

As for the protein assembly or the salt thereof, more preferable is a protein assembly or a salt thereof obtained by treating under a neutral condition one or more triads represented by formula (IV) or salts thereof wherein, $R^{11}$ and $R^{13}$ each represent a lower alkenyl group and $R^{12}$ and $R^{14}$ each represent a lower alkyl group, or $R^{11}$ and $R^{13}$ each represent a lower alkyl group and $R^{12}$ and $R^{14}$ each represent a lower alkenyl group;

$R^{21}$ and $R^{23}$ each represent a lower alkenyl group and $R^{22}$ and $R^{24}$ each represent a lower alkyl group, or $R^{21}$ and $R^{23}$ each represent a lower alkyl group and $R^{22}$ and $R^{24}$ each represent a lower alkenyl group;

$R^{31}$ and $R^{33}$ each represent a lower alkenyl group and $R^{32}$ and $R^{34}$ each represent a lower alkyl group, or $R^{31}$ and $R^{33}$ each represent a lower alkyl group and $R^{32}$ and $R^{34}$ each represent a lower alkenyl group;

$Z^1$, $Z^2$, and $Z^3$ each independently represent a group represented by a formula $-(CH_2)_{m1}-$, $-(CH_2)_{m1}-O-(CH_2)_{m2}-O-(CH_2)_{m3}-$, or $-(CH_2)_{m1}-O-(CH_2)_{m2}-O-(CH_2)_{m3}-O-(CH_2)_{m4}-$ wherein m1, m2, m3, and m4 each independently represent an integer of 1 to 20; $M^1$, $M^2$, and $M^3$ each independently represent Fe or Zn, with at least one of a protein monomer represented by formula (II) or a salt thereof wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$wherein n1, n2, n3, and n4 each independently represent an integer of 1 to 3; M represents Fe or Zn; and the hemoprotein is a cytochrome or a myoglobin, and

a protein monomer represented by formula (II) or a salt thereof wherein $R^1$ and $R^3$ each independently represent a lower alkenyl group and $R^2$ and $R^4$ each independently represent a lower alkyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$ wherein n1, n2, n3, and n4 each independently represent an integer of 1 to 3; M represents Fe or Zn; and the hemoprotein is a cytochrome or a myoglobin.

As for the protein assembly or the salt thereof, more preferable is a protein assembly or a salt thereof obtained by treating under a neutral condition one or more triads represented by formula (IV) or salts thereof wherein $R^{11}$ and $R^{13}$ each represent a lower alkenyl group and $R^{12}$ and $R^{14}$ each represent a lower alkyl group, or $R^{11}$ and $R^{13}$ each represent a lower alkyl group and $R^{12}$ and $R^{14}$ each represent a lower alkenyl group;

$R^{21}$ and $R^{23}$ each represent a lower alkenyl group and $R^{22}$ and $R^{24}$ each represent a lower alkyl group, or $R^{21}$ and $R^{23}$ each represent a lower alkyl group and $R^{22}$ and $R^{24}$ each represent a lower alkenyl group;

$R^{31}$ and $R^{33}$ each represent a lower alkenyl group and $R^{32}$ and $R^{34}$ each represent a lower alkyl group, or $R^{31}$ and $R^{33}$ each represent a lower alkyl group and $R^{32}$ and $R^{34}$ each represent a lower alkenyl group;

$Z^1$, $Z^2$, and $Z^3$ each independently represent a group represented by a formula $-(CH2)_{ml}-$, $-(CH_2)_{ml}-O-(CH_2)_{m2}-O-(CH_2)_{m3}-$ or $-(CH_2)_{m1}-O-(CH_2)_{m2}-O-(CH_2)_{m3}-O-(CH_2)_{m4}-$ wherein ml, m2, m3, and m4 each independently represent an integer of 1 to 20; $M^1$, $M^2$, and $M^3$ each independently represent Fe or Zn, with

a protein monomer represented by formula (II) or a salt thereof wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$ wherein n1, n2, n3, and n4 each independently represent an integer of 1 to 3; M represents Fe or Zn; and the hemoprotein is a cytochrome or a myoglobin, and

a protein monomer represented by formula (II) or a salt thereof wherein $R^1$ and $R^3$ each independently represent a lower alkenyl group and $R^2$ and $R^4$ each independently represent a lower alkyl group; Y is a group represented by a formula $-(CH2)_{n1}-$, $-(CH_2)_{n1}-O-(CH_2)_{n2}-O-(CH_2)_{n3}-$ or $-(CH_2)_{n1}-O-(CH_2)_{n2}-O-(CH_2)_{n3}-O-(CH_2)_{n4}-$ wherein n1, n2, n3, and n4 each independently represent an integer of 1 to 3; M represents Fe or Zn; and the hemoprotein is a cytochrome or a myoglobin.

As for the protein assembly or the salt thereof, more preferable is a protein assembly or a salt thereof obtained by treating under a neutral condition a triad represented by formula (IV) or a salt thereof wherein $R^{11}$ and $R^{13}$, $R^{21}$ and $R^{23}$, and $R^{31}$ and $R^{33}$ each independently represent a lower alkyl group and $R^{12}$ and $R^{14}$, $R^{22}$ and R2, and $R^{32}$ and $R^{34}$ each independently represent a lower alkenyl group; $Z^1$, $Z^2$, and $Z^3$ each independently represent a group represented by a formula $-(CH_2)_{m1}-$, $-(CH2)_{m1}-O-(CH_2)_{m2}-O-(CH_2)_{m3}-$, or $-(CHO_{m1}-O-(CH_2)_{m2}-O-(CH_2)_{m3}-O-(CH_2)_{m4}-$ wherein m1,

m2, m3, and m4 each independently represent an integer of 1 to 20; and $M^1$, $M^2$, and $M^3$ each independently represent Fe or Zn, with

a protein monomer represented by formula (II) or a salt thereof wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$ wherein n1, n2, n3, and n4 each independently represent an integer of 1 to 3; M represents Fe or Zn; and the hemoprotein is a cytochrome or a myoglobin.

As for the protein assembly or the salt thereof, more preferable is a protein assembly or a salt thereof obtained by treating under a neutral condition a triad represented by formula (IV) or a salt thereof wherein $R^{11}$ and $R^{13}$, $R^{21}$ and $R^{23}$, and $R^{31}$ and $R^{33}$ each independently represent a lower alkenyl group and $R^{12}$ and $R^{14}$, $R^{22}$ and $R^{24}$, and $R^{32}$ and $R^{34}$ each independently represent a lower alkyl group; $Z^1$, $Z^2$, and $Z^3$ each independently represent a group represented by a formula $-(CH_2)_{m1}-$, $-(CH_2)_{m1}-O-(CH_2)_{m2}-O-(CH_2)_{m3}-$, or $-(CH_2)_{m1}-O-(CH_2)_{m2}-O-(CH_2)_{m3}-O-(CH_2)_{m4}-$, wherein m1, m2, m3, and m4 each independently represent an integer of 1 to 20; and $M^1$, $M^2$, and $M^3$ each independently represent Fe, or Zn, with

a protein monomer represented by formula (II) or a salt thereof wherein $R^1$ and $R^3$ each independently represent a lower alkyl group and $R^2$ and $R^4$ each independently represent a lower alkenyl group; Y is a group represented by a formula $-(CH_2)_{n1}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$ wherein n1, n2, n3, and n4 each independently represent an integer of 1 to 3; M represents Fe or Zn; and the hemoprotein is a cytochrome or a myoglobin.

As for the protein assembly or the salt thereof, further preferable is a protein assembly or a salt thereof obtained by treating under a neutral condition one or more triads represented by formula (IV) or salts thereof wherein $R^{11}$ and $R^{13}$ each represent a vinyl group and $R^{12}$ and $R^{14}$ each represent a methyl group, or $R^{11}$ and $R^{13}$ each represent a methyl group and $R^{12}$ and $R^{14}$ each represent a vinyl group;

$R^{21}$ and $R^{23}$ each represent a vinyl group and $R^{22}$ and $R^{24}$ each represent a methyl group, or $R^{21}$ and $R^{23}$ each represent a methyl group and $R^{22}$ and $R^{24}$ each represent a vinyl group;

$R^{31}$ and $R^{33}$ each represent a vinyl group and $R^{32}$ and $R^{34}$ each represent a methyl group, or $R^{31}$ and $R^{33}$ each represent a methyl group and $R^{32}$ and $R^{34}$ each represent a vinyl group; $Z^1$, $Z^2$, and $Z^3$ each independently represent a group represented by a formula $-(CHO_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; and $M^1$, $M^2$, and $M^3$ each independently represent Fe or Zn, with at least one of

a protein monomer represented by formula (II) or a salt thereof wherein $R^1$ and $R^3$ each independently represent a methyl group; $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEX ID NO.1 and said one amino acid residue is the histidine of the 63rd amino acid residue, or a myoglobin that has an amino acid sequence having SEQ ID NO. 2 and said one amino acid residue is the alanine of the 125th or 126th amino acid residue, and

a protein monomer represented by formula (II) or a salt thereof wherein $R^1$ and $R^3$ each independently represent a vinyl group; $R^2$ and $R^4$ each independently represent a methyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

As for the protein assembly or the salt thereof, further preferable is a protein assembly or a salt thereof obtained by treating under a neutral condition one or more triads represented by formula (TV) or salts thereof wherein $R^{11}$ and $R^{13}$ each represent a vinyl group and $R^{12}$ and $R^{14}$ each represent a methyl group, or $R^{11}$ and $R^{13}$ each represent a methyl group and $R^{12}$ and $R^{14}$ each represent a vinyl group;

$R^{21}$ and $R^{23}$ each represent a vinyl group and $R^{22}$ and $R^{24}$ each represent a methyl group, or $R^{21}$ and $R^{23}$ each represent a methyl group and $R^{22}$ and $R^{24}$ each represent a vinyl group;

$R^{31}$ and $R^{33}$ each represent a vinyl group and $R^{32}$ and $R^{34}$ each represent a methyl group, or $R^{31}$ and $R^{33}$ each represent a methyl group and $R^{32}$ and $R^{34}$ each represent a vinyl group; $Z^1$, $Z^2$, and $Z^3$ each independently represent a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; and $M^1$, $M^2$, and $M^3$ each independently represent Fe or Zn, with

a protein monomer represented by formula (II) or a salt thereof wherein $R^1$ and $R^3$ are each independently a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue, and

a protein monomer represented by formula (II) or a salt thereof wherein $R^1$ and $R^3$ are each independently a vinyl group

and $R^2$ and $R^4$ each independently represent a methyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

As for the protein assembly or the salt thereof, further preferable is a protein assembly or a salt thereof obtained by treating under a neutral condition a triad represented by formula (IV) or a salt thereof wherein $R^{11}$ and $R^{13}$, $R^{21}$ and $R^{23}$, and $R^{31}$ and $R^{33}$ each independently represent a methyl group and $R^{12}$ and $R^{14}$, $R^{22}$ and $R^{24}$, and $R^{32}$ and $R^{34}$ each independently represent a vinyl group; $Z^1$, $Z^2$, and $Z^3$ each independently represent a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; and $M^1$, $M^2$, and $M^3$ each independently represent Fe or Zn, with

a protein monomer represented by formula (II) or a salt thereof wherein $R^1$ and $R^3$ each independently represent a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

As for the protein assembly or the salt thereof, further preferable is a protein assembly or a salt thereof obtained by treating under a neutral condition a triad represented by formula (IV) or a salt thereof wherein $R^{11}$ and $R^{13}$, $R^{21}$ and R23, and $R^{31}$ and $R^{33}$ each independently represent a vinyl group and $R^{12}$ and $R^{14}$, $R^{22}$ and $R^{24}$, and $R^{32}$ and $R^{34}$ each independently represent a methyl group; $Z^1$, $Z^2$, and $Z^3$ each independently represent a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; and $M^1$, $M^2$, and $M^3$ each independently represent Fe or Zn, with

a protein monomer represented by formula (II) or a salt thereof wherein $R^1$ and $R^3$ each independently represent a methyl group and $R^2$ and $R^4$ each independently represent a vinyl group; Y is a group represented by a formula $-(CH_2)_2-$, $-(CH_2)_2-O-(CH_2)_2-O-(CH_2)_2-$, or $-(CH_2)_3-O-(CH_2)_2-O-(CH_2)_2-O-(CH_2)_3-$; M represents Fe or Zn; the hemoprotein is a cytochrome that has an amino acid sequence having SEQ ID NO. 1 and said one amino acid residue is the histidine of the 63rd amino acid residue, or a myoglobin that has an amino acid sequence having SEQ ID NO. 2 and said one amino acid residue is the alanine of the 125th or 126th amino acid residue.

Next, an example of a method for producing the protein monomer represented by formula (I) or the salt thereof of the present invention shall be described.

A feature of the production method is to react a porphyrin linker represented by formula (VI) with a hemoprotein represented by formula (V) to give the protein monomer of formula (I) or the salt thereof (see scheme 1).

[Chemical Formula 17]

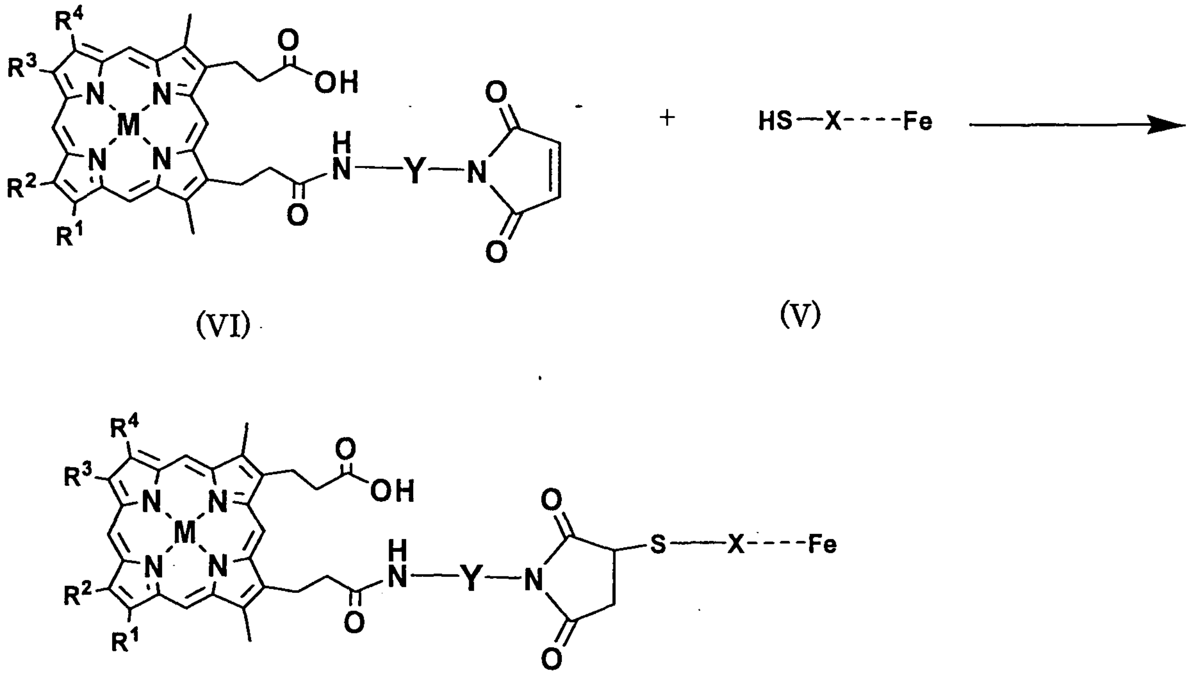

(VI) (V)

(I)

Scheme 1

In formulas (I) and (VI) above,
$R^1$, $R^2$, $R^3$, and $R^4$ each independently represent a hydrogen atom, a lower alkyl group, a halogen-substituted lower alkyl group, or a lower alkenyl group;
Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
in formulas (I) and (V),
X represents X that is present in a hemoprotein mutant, and the hemoprotein mutant is represented by the formula (V):
[Chemical Formula 18] **HS-X----Fe** (V)

The mutant is a protein that has the same amino acid sequence as a native hemoprotein except that one amino acid residue is replaced with a cysteine residue. In the mutant, -SH represents a side-chain thiol group of the cysteine residue, and Fe is bonded to the four nitrogen atoms of a porphyrin group contained in the hemoprotein.
In formula (VI) above, the lower alkyl group and the lower alkenyl group are as defined above.
In the production method, the reaction between the porphyrin linker represented by formula (VI) and the hemoprotein represented by formula (V) may be performed in a solvent in the presence of a catalyst under an inert gas (e.g., argon and nitrogen) atmosphere. Examples of the catalyst include weak acids and weak bases. The weak acids include citric acid, hydrochloric acid, sulfuric acid, nitric acid, toluenesulfonic acid, acetic acid, and the like. The weak bases include histidine, sodium hydrogencarbonate, tris(hydroxymethyl) aminomethane, and the like.
In the production method, the reaction solvent is not limited, and examples include polar solvents such as alcohols (e.g., methanol and ethanol), water, dimethyl sulfoxide and dimethylformamide, and buffers (e.g., Tris-HCl buffers, phosphate buffers, borate buffers, and carbonate buffers). The reaction solvent may contain an additive to enhance the solubility of the porphyrin linker represented by formula (VI) and/or the hemoprotein represented by formula (V). The additive includes histidine and the like. Such solvents may be used singly or as a mixture of two or more. It is preferable that the solvent is selected from dimethyl sulfoxide and buffers that are mentioned above as examples, and mixtures of dimethyl sulfoxide and Tris-HCl, histidine-containing water, and the like are more preferable.

In the production method, the reaction temperature is not limited, and it may be, for example, 0 to 100°C, preferably 10 to 60°C, and more preferably 20 to 30°C.

In the production method, the reaction time is not limited, and it may be, for example, 30 minutes to 24 hours, preferably 2 to 10 hours, and more preferably 5 to 8 hours.

In the production method, the molar ratio of the porphyrin linker represented by formula (VI) relative to the hemoprotein represented by formula (V) (porphyrin linker represented by formula (VI):hemoprotein represented by formula (V)) is, for example, 100:1 to 5:1, preferably 50:1 to 10:1, and more preferably 20:1 to 10:1.

In the production method, the porphyrin linker represented by formula (VI) may be commercially available, and it may be produced privately in reference to a known document.

In the production method, the hemoprotein represented by formula (V) can be produced, for example, as described below.

A gene coding for the hemoprotein represented by formula (V) may be cloned using the guinea pig total RNA or the like, or a DNA may be chemically synthesized using the phosphoroamidite method based on the base sequence of a gene coding for a native hemoprotein. The cloning method is not particularly limited and may be performed using, for example, a commercially available cloning kit or the like. Moreover, a gene coding for the hemoprotein represented by formula (V) may be transferred to a host cell.

Examples of the host cell include animal cells, plant cells, insect cells, yeasts, and bacteria. Examples of the gene transfer technique include the lithium acetate method, calcium phosphate method, methods that use liposome, electroporation, methods that use a viral vector, and micropipette injection method. Alternatively, a gene may be transferred using integration into the host chromosome, an artificial chromosome or a plasmid that can replicate and partition autonomously.

The gene to be introduced in the host cell preferably is operably linked to a necessary regulatory sequence so that it is expressed constitutively or randomly in the host cell. The term "regulatory sequence" refers to a base sequence that is necessary for the expression of the gene operably linked in a host cell, and examples of regulatory sequences suitable for use in eukaryotic cells include promoters, polyadenylation signals, and enhancers. The phrase "operably linked" means that respective components are juxtaposed such that they can function.

Hereinbelow, an example of a method for producing the protein monomer represented by formula (II) or the salt thereof of the present invention shall be described.

A feature of the production method is to treat the protein monomer represented by formula (I) or the salt thereof with an acid to obtain the protein monomer represented by formula (II) or the salt thereof (see scheme 2).

[Chemical Formula 19]

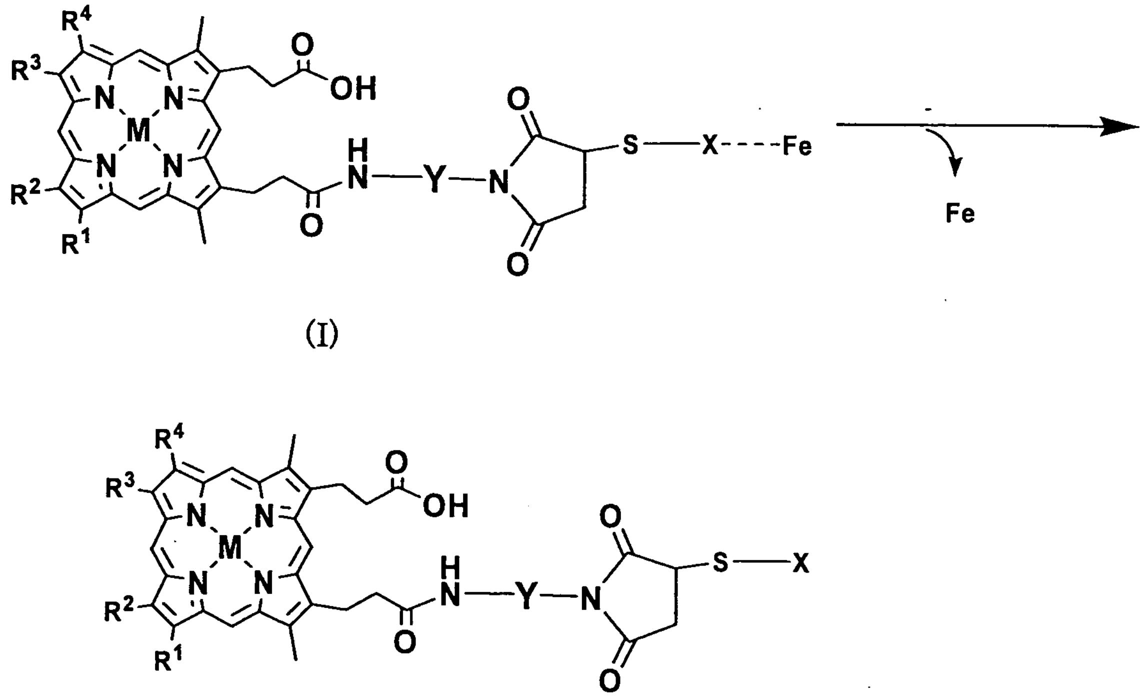


(I)

(II)

Scheme 2

In formulas (I) and (II) above,
$R^1$, $R^2$, $R^3$, and $R^4$ each independently represent a hydrogen atom, a lower alkyl group, a halogen-substituted lower alkyl group, or a lower alkenyl group; Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
X represents X that is present in a hemoprotein mutant, and the hemoprotein mutant is represented by the formula (V):
[Chemical Formula 20] **HS-X----Fe** (V)

The mutant is a protein that has the same amino acid sequence as a native hemoprotein except that one amino acid residue is replaced with a cysteine residue. In the mutant, -SH represents a side-chain thiol group of the cysteine residue, and Fe is bonded to the four nitrogen atoms of a porphyrin group contained in the hemoprotein.
In the production method, the acid treatment reaction of the protein monomer represented by formula (I) or the salt thereof may be carried out by, for example, treatment with hydrochloric acid, sulfuric acid, nitric acid, or the like.
In the production method, the reaction solvent is not limited, and examples include polar solvents such as alcohols (e.g., methanol and ethanol), water, dimethyl sulfoxide and dimethylformamide, and buffers (e.g., Tris-HCl buffers, phosphate buffers, borate buffers, and carbonate buffers). Such solvents may be used singly or as a mixture of two or more. It is preferable that the solvent is selected from dimethyl sulfoxide and buffers, and mixtures of dimethyl sulfoxide and Tris-HCl, phosphate buffers, and the like are more preferable.
In the production method, the reaction temperature is not limited, and it may be, for example, 0 to 30°C, preferably 0 to 10°C, and more preferably 3 to 5°C.
In the production method, the acid treatment may be carried out at, for example, a pH of 0.5 to 3.0, preferably 1.5 to 2.5, and more preferably 1.8 to 2.1.
In the method for producing the protein monomer represented by formula (I) or the salt thereof as well as the method for producing the protein monomer represented by formula (II) or the salt thereof, the hemoprotein preferably is a cytochrome, a hemoglobin, a myoglobin, or a peroxidase.

Hereinbelow, an example of the method for producing a protein polymer or a salt thereof of the present invention shall be described.

A feature of the production method is to treat the protein monomer represented by formula (II) or the salt thereof under a neutral condition to obtain a protein polymer or a salt thereof.

In the production method, the reaction solvent is not limited, and examples include polar solvents such as alcohols (e.g., methanol and ethanol), water, dimethyl sulfoxide and dimethylformamide, and buffers (e.g., Tris-HCl buffers, phosphate buffers, borate buffers, and carbonate buffers). Such solvents may be used singly or as a mixture of two or more. It is preferable that the solvent is selected from phosphate buffers, and Tris-HCl buffers, phosphate buffers, and the like are more preferable.

In the production method, the reaction temperature is not limited, and it may be, for example, 0 to 30°C, preferably 0 to 10°C, and more preferably 3 to 5°C.

In the production method, the treatment under a neutral condition may be carried out at, for example, a pH of 6.0 to 10.0, preferably 6.5 to 8.5, and more preferably 7.0 to 8.0.

In the method for producing a protein polymer or a salt of the present invention, the protein polymer or the salt thereof preferably is the protein polymer represented by formula (III) or the salt thereof (see scheme 3).

[Chemical Formula 21]

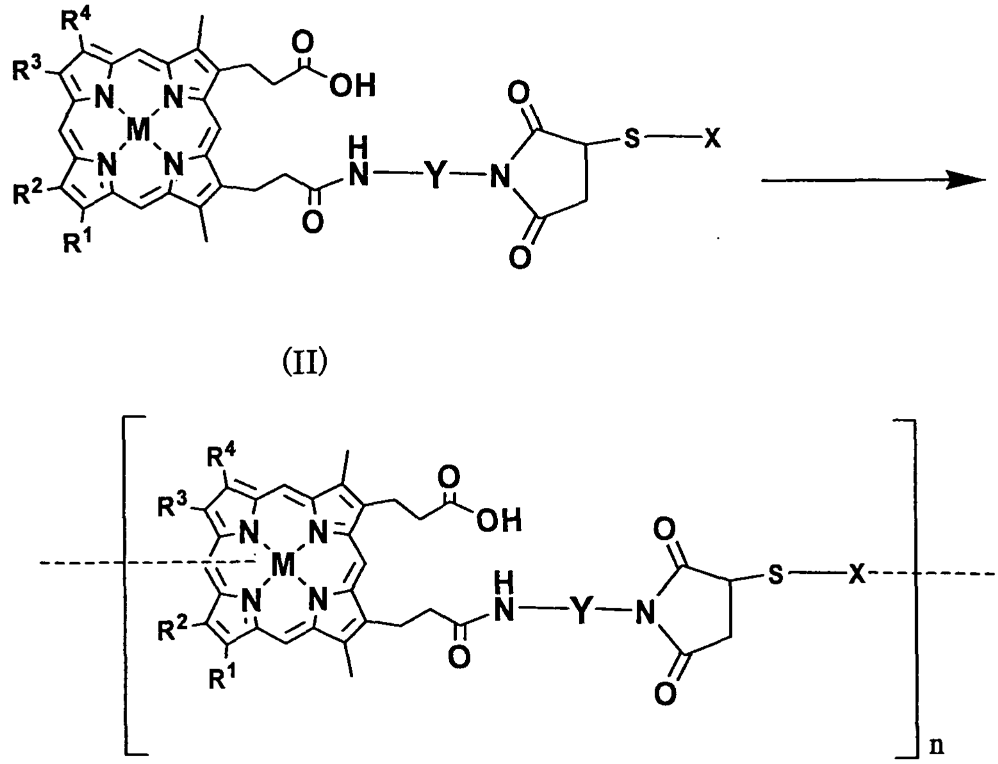

(II)

(III)

Scheme 3

In formulas (II) and (III) above,
$R^1$, $R^2$, $R^3$, and $R^4$ each independently represent a hydrogen atom, a lower alkyl group, a halogen-substituted lower alkyl group, or a lower alkenyl group;
Y is a group represented by a formula $-(CH_2)_{n1}-$, $-(CH_2)_{n1}-O-(CH_2)_{n2}-$, $-(CHO_{n1}-O-(CH2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
X represents X that is present in a hemoprotein mutant, and the hemoprotein mutant is represented by the formula (V):

[Chemical Formula 22] **HS-X----Fe** (V)

The mutant is a protein that has the same amino acid sequence as a native hemoprotein except that one amino acid residue is replaced with a cysteine residue. In the mutant, -SH represents a side-chain thiol group of the cysteine residue, and Fe is bonded to the four nitrogen atoms of a porphyrin group contained in the hemoprotein.

In the production method, the protein monomer represented by formula (II) or the salt thereof may be a compound composed solely of a protein monomer represented by formula (II) or a salt thereof or may be a mixture in which a compound that is a positional isomer of the protein monomer or a salt thereof is concomitantly present. In the case of a mixture of a protein monomer represented by formula (II) or a salt thereof with a compound, or a salt thereof, that is a positional isomer of the protein monomer represented by formula (II) or the salt thereof is used, the resulting protein polymer represented by formula (III) is also a mixture with a positional isomer.

In the method for producing a protein polymer or a salt thereof of the present invention, the hemoprotein preferably is a cytochrome, a hemoglobin, a myoglobin, or a peroxidase.

The protein polymer or the salt thereof of the present invention that contains the protein monomer represented by formula (II) or the salt thereof as a monomer unit is of use as an oxygen-storing biopolymer when the hemoprotein is a cytochrome, a hemoglobin, or a myoglobin. Moreover, when the hemoprotein is a peroxidase, the protein polymer or a salt thereof is of use as an enzyme assembly.

The protein polymer or the salt thereof of the present invention that contain the protein monomer represented by formula (II) or the salt thereof as a monomer unit can be decomposed into the protein monomer represented by formula (II) or the salt thereof under an acidic or basic condition. Therefore, the protein polymer or the salt thereof of the present invention is of use as a pH-responsive protein polymer of which assembly state is controlled by pH.

Hereinbelow, an example of a method for producing the protein assembly or the salt thereof of the present invention shall be described.

A feature of the production method is to treat the triad represented by (IV) or the salt thereof with the protein monomer represented by (II) or the salt thereof under a neutral condition to obtain the protein assembly or the salt thereof of the present invention.

A feature of the production method is to treat the triad represented by (IV) or the salt thereof with the protein monomer represented by (II) or the salt thereof under a neutral condition to obtain a protein assembly or a salt thereof.

In the production method, the reaction solvent is not limited, and examples include polar solvents such as alcohols (e.g., methanol and ethanol), water, dimethyl sulfoxide and dimethylformamide, and buffers (e.g., Tris-HCl buffers, phosphate buffers, borate buffers, and carbonate buffers). Such solvents may be used singly or as a mixture of two or more. Such solvents preferably are selected from buffers, and Tris-HCl buffers are more preferable.

In the production method, the reaction temperature is not limited, and it may be, for example, 0 to 30°C, preferably 0 to 10°C, and more preferably 3 to 5°C.

In the production method, the treatment under a neutral condition may be carried out at, for example, a pH of 6.0 to 10.0, preferably 6.5 to 8.5, and more preferably 7.0 to 8.0.

In the method for producing the protein assembly or the salt of the present invention, the protein assembly or the salt thereof preferably is a protein assembly represented by formula (VII) or a salt thereof (see scheme 4).

[Chemical Formula 23]

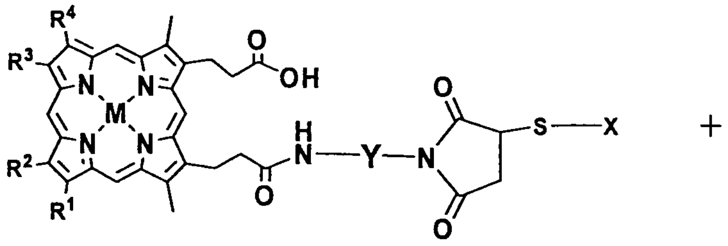

(II)

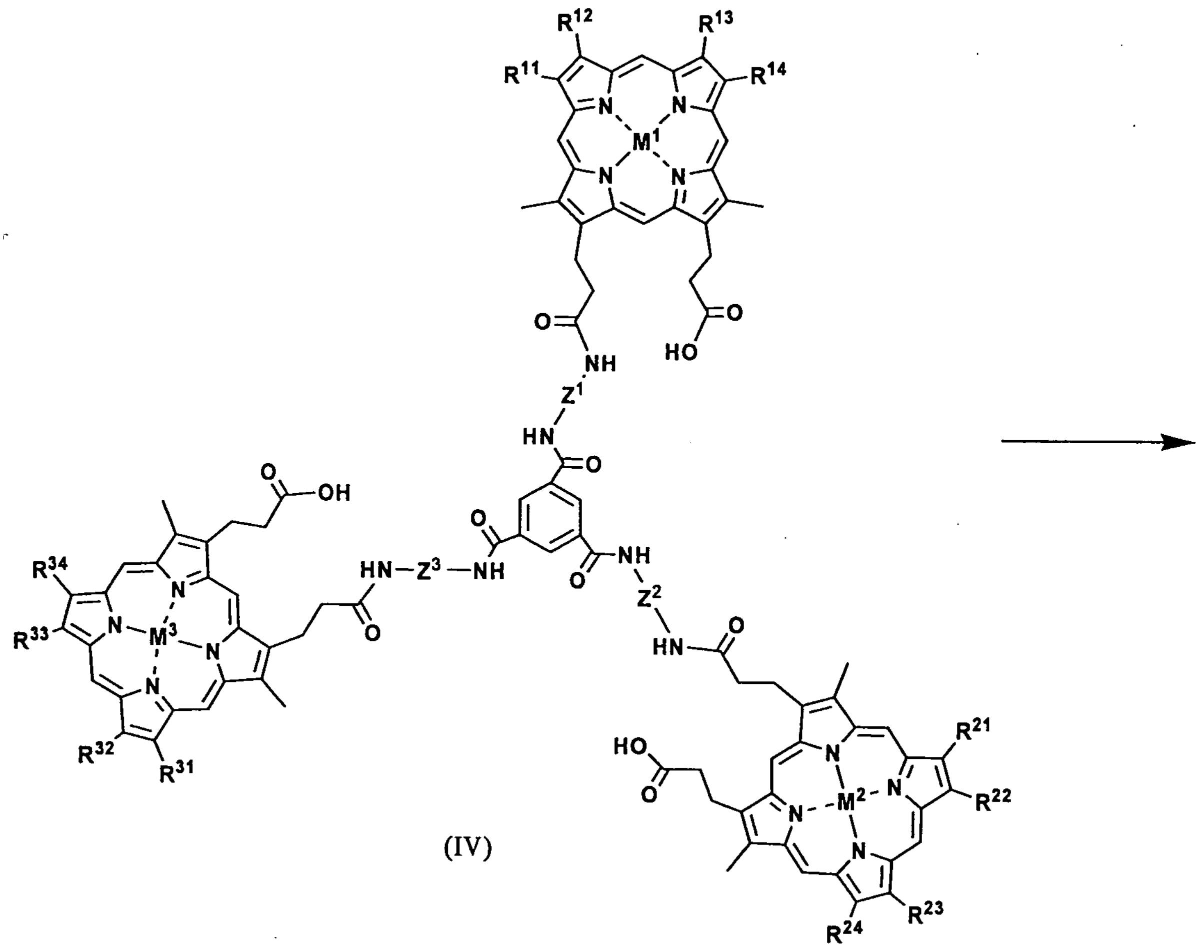

(IV)

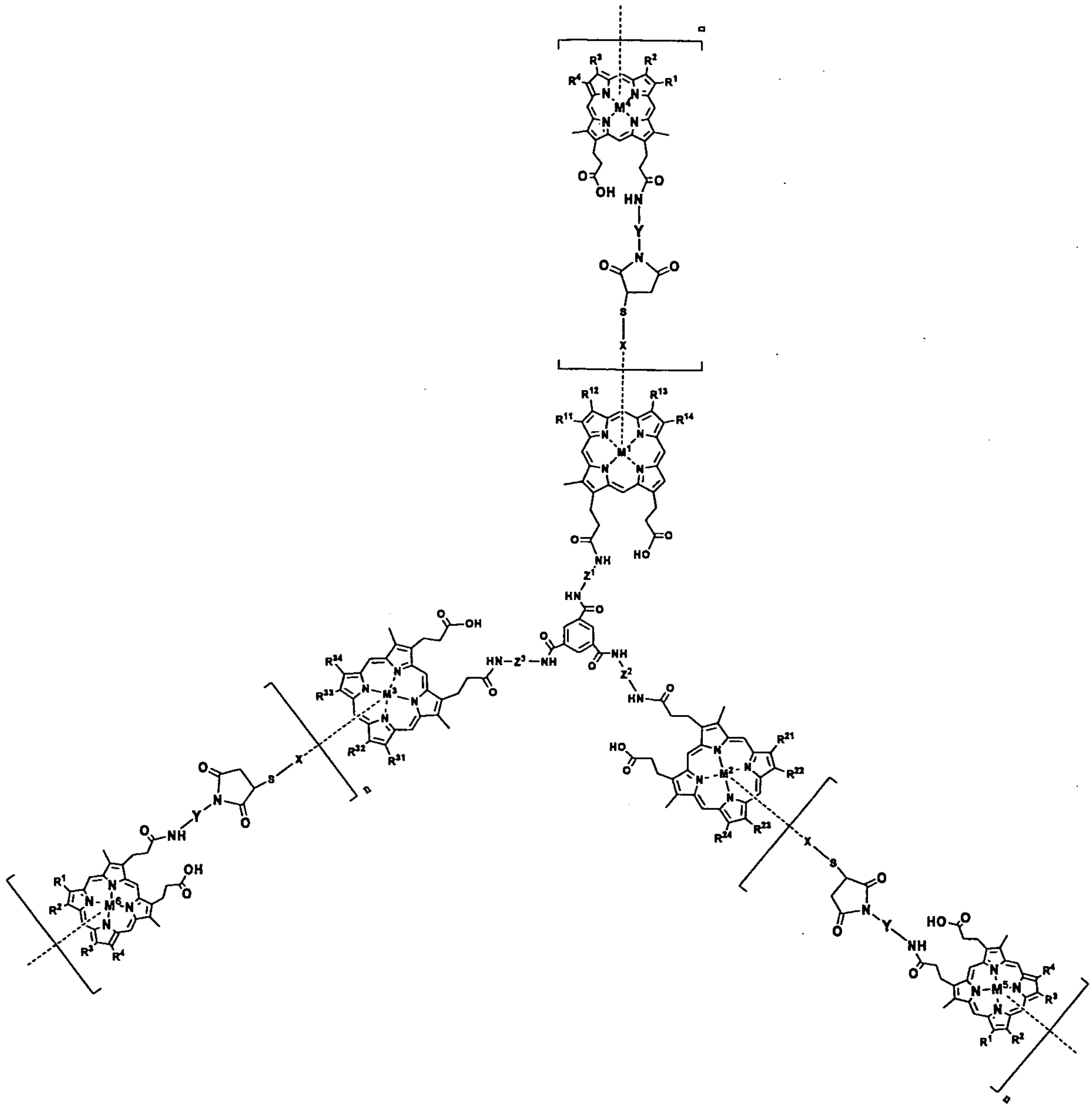

Scheme 4

In formulas (II), (IV), and (VII) above,

$R^1$, $R^2$, $R^3$, and $R^4$ each independently represent a hydrogen atom, a lower alkyl group, a halogen-substituted lower alkyl group, or a lower alkenyl group;

$R^{11}$, $R^{12}$, $R^{13}$, $R^{14}$, $R^{21}$, $R^{22}$, $R^{23}$, $R^{24}$, $R^{31}$,$R^{32}$, $R^{33}$, and $R^{344}$ each independently represent a hydrogen atom, a lower alkyl group, a halogen-substituted lower alkyl group, or a lower alkenyl group;

$Z^1$, $Z^2$, and $Z^3$ each independently represent a group represented by a formula $-(CH_2)_{m1}-$, $-(CH_2)_{m1}-O-(CH_2)_{m2}-$, $-(CH_2)_{m1}-O-(CH_2)_{m2}-O-(CH_2)_{m3}-$, or $-(CH_2)_{m1}-O-(CH_2)_{m2}-O-(CH_2)_{m3}-O-(CH_2)_{m4}-$, wherein m1, m2, m3, and m4 each independently represent an integer of 1 to 20;

Y is a group represented by a formula $-(CH_2)_{n1}$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20; and

M1, M2, M3, M4, M5, and M6 are each independently selected from the group consisting of Fe, Zn, and Co.

X represents X that is present in a hemoprotein mutant, and the hemoprotein mutant is represented by the formula (V).

[Chemical Formula 24] **HS-X----Fe** (V)

The mutant is a protein that has the same amino acid sequence as a native hemoprotein except that one amino acid residue is replaced with a cysteine residue. In the mutant, -SH represents a side-chain thiol group of the cysteine residue, and Fe is bonded to the four nitrogen atoms of a porphyrin group contained in the hemoprotein.

In the production method, the protein monomer represented by formula (II) or the salt thereof may be a compound composed solely of a protein monomer represented by formula (II) or a salt thereof or may be a mixture in which a compound that is a positional isomer of the protein monomer or a salt thereof is concomitantly present. In the case of a mixture of the protein monomer represented by formula (II) or the salt thereof with a compound, or a salt thereof, that is a positional isomer of the protein monomer represented by formula (II) or the salt thereof is used, the resulting protein assembly represented by formula (VII) or a salt thereof is also a mixture with a positional isomer.

In the production method, the molar ratio of the triad represented by formula (IV) or the salt thereof relative to the protein monomer represented by formula (II) or the salt thereof (the triad represented by formula (IV) or the salt thereof: the protein monomer represented by formula (II) or the salt thereof) is, for example, 1:1 to 1:1000, preferably 1:5 to 1:500, and more preferably 1:10 to 1:100.

In the method for producing the protein polymer or the salt thereof of the present invention, the hemoprotein preferably is a cytochrome, a hemoglobin, a myoglobin, or a peroxidase.

The protein assembly or the salt thereof of the present invention that contains as a monomer unit the protein monomer represented by formula (II) or the salt thereof is of use as an oxygen-storing biomaterial when the hemoprotein is a cytochrome, a hemoglobin, or a myoglobin. Moreover, when the hemoprotein is a peroxidase, the protein assembly or the salt thereof is of use as an enzyme assembly. In the biomaterial, M in the protein assembly or the salt thereof that contains as a monomer unit the protein monomer represented by formula (II) or the salt thereof is preferably $Fe^{2+}$.

The present invention also is directed to, as stated above, a nanosheet containing the protein assembly or the salt thereof. The thickness of the nanosheet is, for example, 1 to 10 nm and preferably 2 to 5 nm. It is preferable that the nanosheet has a mesh structure. The protein assembly or the salt thereof that contains as a monomer unit the protein monomer represented by formula (II) or the salt thereof wherein the hemoprotein is a cytochrome, a hemoglobin, or a myoglobin is of use as an oxygen-storing biomaterial, a catalyst fiber, a sensor fiber, or the like. In the nanosheet, M in the protein assembly preferably is $Fe^{2+}$.

The present invention also is directed to, as stated above, a device containing one or more selected from the group consisting of the protein monomer of formula (I), the salt of the protein monomer of formula (I), the protein monomer of formula (II), the salt of the protein monomer of formula (II), the protein polymer containing as a monomer unit the protein monomer of formula (II), the salt of the protein polymer containing as a monomer unit the protein monomer of formula (II), the protein polymer of formula (III), the salt of the protein polymer of formula (III), the protein assembly of formula (VII), and the salt of the protein assembly of formula (VII). The device is for use as an oxygen sensor, an oxygen adsorber, or the like. When the device is used in such a way, M present in the protein monomer, the protein monomer salt, the protein polymer, the protein polymer salt, the protein assembly, and the protein assembly salt contained in the device is preferably $Fe^{2+}$.

The present invention also is directed to, as stated above, a substrate modified with one or more selected from the group consisting of the protein monomer of formula (II), the salt of the protein monomer of formula (II), the protein polymer that contains as a monomer unit the protein monomer of formula (II), the salt of the protein polymer that contains as a monomer unit the protein monomer of formula (II), the protein polymer of formula (III), and the salt of the protein polymer of formula (III) (provided that in the formulas (II) and (III), M is Fe). Since the protein monomer as well as the other products have an ability to store oxygen, the application of an electric current to the substrate allows oxygen to be stored in the protein monomer as well as in the other products and the termination of an electric current allows oxygen to be released from the protein monomer and like materials that have stored oxygen, thus enabling the substrate to be used as an oxygen storing electrode. When the substrate is used in such a way, M present in the protein monomer, the protein monomer salt, the protein polymer, or the protein polymer salt that modifies the substrate is preferably $Fe^{2+}$.

The aforementioned substrate may be produced, for example, as described below. First, a substrate is provided. This substrate may be a metal plate or a plate that is made of a plastic or other materials and the surface of which is coated with a metal. The substrate is not limited to being in the form of a plate. Initially, a heme-containing linker is fixed covalently to the surface of the substrate. Specifically, for example, a linker that contains a reactive group is bonded to the substrate first. Thereafter, a hemin is coupled to the reactive group and then the substrate is subjected to a reaction with one or more selected from the group consisting of the protein monomer represented by formula (II), the salt of the protein monomer represented by formula (II), the protein polymer that contains as a monomer unit the protein monomer of formula (II), the salt of the protein polymer that contains as a monomer unit the protein monomer of formula (II), the protein polymer represented by formula (III), and the salt of the protein polymer represented by formula (III) Consequently, the heme contained in the hemin present on the surface of the substrate reacts with one of more selected from the group consisting of the protein monomer represented by formula (II), the salt of the protein monomer represented by formula

(II), the protein polymer that contains as a monomer unit the protein monomer of formula (II), the salt of the protein polymer that contains as a monomer unit the protein monomer of formula (II), the protein polymer represented by formula (III), and the salt of the protein polymer represented by formula (III), thereby giving a substrate modified with one or more selected from the group consisting of the protein monomer represented by formula (II), the salt of the protein monomer represented by formula (II), the protein polymer that contains as a monomer unit the protein monomer of formula (II), the salt of the protein polymer that contains as a monomer unit the protein monomer of formula (II), the protein polymer represented by formula (III), and the salt of the protein polymer represented by formula (III). Note that a substrate modified with one or more selected from the group which consisting of the protein polymer of formula (III) and the salt of the protein polymer of formula (III) may be obtained as a result of polymerization that occurs on a substrate when the protein monomer of formula (II) or the salt thereof is reacted. The method of bonding the linker that contains a reactive group to the substrate may be performed in reference to, for example, a paper by A. Das et al. (J. Biophysical Chemistry, 2006, Vol. 123, pp.102-112). The length of the linker is not particularly limited.

The present invention shall be described more specifically hereinbelow by way of examples; however, the examples are not to limit the scope of the invention. Various spectra were measured with the following instruments. Nuclear magnetic resonance (NMR) spectra were measured with a JEOL EX270 nuclear magnetic resonance spectrometer (270 MHz) manufactured by JEOL Ltd., or a Bruker DPX-400 nuclear magnetic resonance spectrometer (400 MHz), with the remaining signal of the measurement solvent being used as an internal reference. Electrospray-ionisation time-of-flight mass spectrometry (ESI-TOF-MS) was carried out with an Applied Biosystems Mariner API-TOF workstation. UV-visible absorption spectra were measured with a spectrophotometer UV-2550 or UV-3150 manufactured by Shimadzu Corporation. The pH of aqueous solutions was measured with a pH meter F-52 manufactured by Horiba Ltd. For size-exclusion chromatography (SEC), measurement was carried out with a Superdex 200 10/300GL column (exclusion limit: $1.3\times10^6$ Da) connected to an $AKTA_{FPLC}$ system manufactured by GE Healthcare, using a UPC-900 detector for detection. Measurement was carried out with an atomic force microscope (AFM) Nanoscope V manufactured by Digital Instruments.

The starting materials were produced in reference to the following documents: Protoporphyrin IX mono-t-butyl ester 2: T. Matsuo, T. Hayashi, Y Hisaeda; J. Am. Chem. Soc. 124, 11234 (2002).
Mono-N-Boc-protected diamines: M. Trester-Zedlitz, K. Kamada, S.K Burley, D. Fenyo, B.T. Chait, T.W Muir; J. Am. Chem. Soc. 125, 2416 (2003), and R. Schneider, F. Schmitt, C. Frochot, Y Fort, N. Lourette, F. Guillemin, J.F. Mueller, M. Barberi-Heyob; Bioorg. Med., Chem. 13, 2799 (2005).
*N*-methoxycarbonylmaleimide: O. Keller, J. Rudinger; Helv Chim. Acta. 58, 531 (1975).
For other general reagents, commercially available products were used without modification.

Example 1

(1) Production of compound represented by formula 1(8)

The compound represented by formula 1(8) was produced according to scheme 5 below.

[Chemical Formula 25]

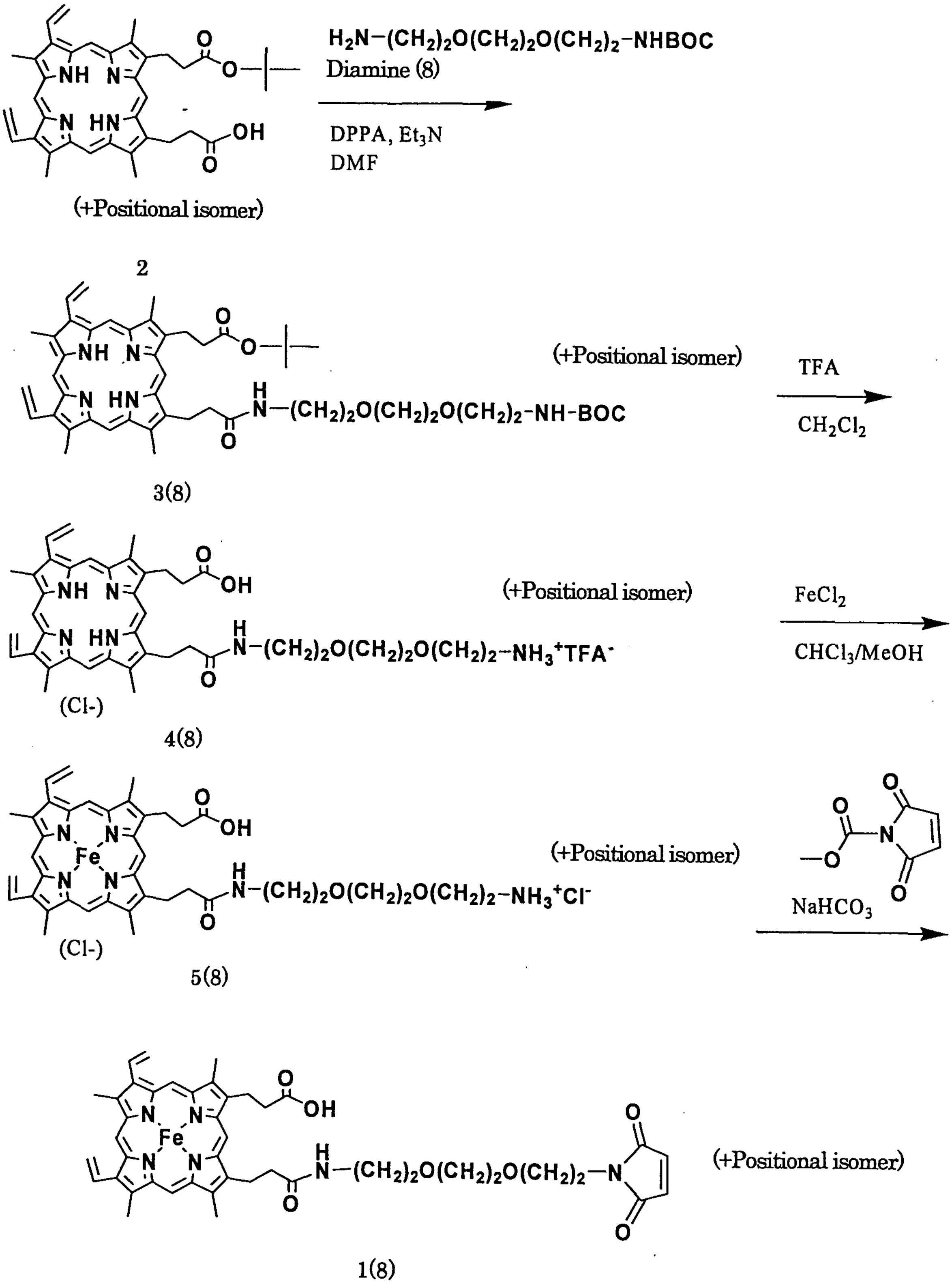


2

3(8)

4(8)

5(8)

1(8)

Scheme 5

(j) Production of compound represented by formula 3(8)

Under a nitrogen atmosphere, protoporphyrin IX mono-*t*-butyl ester 2 (255 mg, $4.1\times10^{-4}$ mol), *N*-Boc-1,2-bis (2-aminoethoxy)ethane (diamine (8), 208 mg, $8.4\times10^{-4}$ mol), and DMF (25 mL) were added to a 50 mL recovery flask

and dissolved. The solution was cooled in an ice bath, and a DMF solution (1 mL) of diphenylphosphoryl azide (DPPA, 290,mg, $1.1\times10^{-3}$ mol) and a DMF solution (1 mL) of triethylamine ($Et_3N$, 170 mg, $1.7\times10^{-3}$ mol) were each added thereto. The solution was stirred under protection from light at room temperature for 4 hours, and DMF solutions of DPPA and $Et_3N$ each in the same amount as above were added. Stirring was performed for 2 more hours, the solvent was distilled off under reduced pressure, and the residue was purified by silica gel chromatography (chloroform/acetone = 5/1). The resulting solids were dissolved in a minimum amount of chloroform, the precipitate formed by adding hexane to the solution was recovered, and thus a mixture of the title compound 3(8) and a positional isomer thereof was obtained (218 mg, 63%, purple, solid).

$^1$H NMR (270 MHz, pyridine-$d_5$) δ:10.55 (s, 1H), 10.46 (s, 1H), 10.41(s, 0.5H), 10.35 (s, 0.5H), 10.29 (s, 0.5H), 10.22 (s, 0.5H) (due to the presence of the positional isomer, the meso-proton signal was split into six; the abundance ratio based on the peak intensity was 1:1.), 8.56-8.42 (m, 2H), 6.44 (m, 2H), 6.19 (m, 2H), 4.59-4.49 (m, 4H), 3.68-3.46 (m, 16H), 3.36 (m, 2H), 3.11 (m, 2H), 2.96 (m, 2H), 2.89 (m, 2H), 2.45 (m, 2H), 2.36 (m, 2H), 1.40 (s, 9H), 1.38 (s, 9H)-3.27 (s, 2H).
ESI-TOF-MS (positive mode) m/z: found 850.07 (M+H)+, calculated for $C_{49}H_{64}N_6O_7$, 850.08.
UV-vis ($CHCl_3$) λmax/nm (absorption): 630 (0.042), 579 (0.056), 543 (0.090), 507 (0.11), 408 (1.27).

(ii) Production of compound represented by formula 4(8)

Under a nitrogen atmosphere, the compound 3(8) (205 mg, $2.4\times10^{-4}$ mol), dichloromethane (10 mL), and trifluoroacetic acid (4 mL) were added to a 50 mL recovery flask while cooling in an ice bath. Thereafter, the reaction solution was returned to room temperature and stirred. Seven hours later, the solvents were distilled off under reduced pressure, and the resulting residue was dissolved by adding a minimum amount of methanol. The precipitate formed by adding diethyl ether to the solution was recovered, and thus a mixture of the title compound 4(8) and a positional isomer thereof was obtained (139 mg, 83%, purple, solid).

$^1$H NMR (270 MHz, pyridine-$d_5$) δ:10.47 (s, 1H), 10.23 (s, 0.5H), 10.20 (s, 1H), 10.15 (s, 1H), 10.05 (s, 0.5H), 9.98 (s, 0.5H) (Due to the presence of the positional isomer, the meso-proton signal split into six. The abundance ratio based on the peak intensity was 1:1.), 8.45-8.31 (m, 2H), 6.42 (m, 2H), 6.17 (m, 2H), 4.63-4.55 (m, 4H), 3.59-3.45 (m, 16H), 3.34 (m, 2H), 3.05-2.96 (m, 6H), 2.47 (m, 2H), 2.39 (m, 2H)-3.80 (s, 2H).
ESI-TOF-MS (positive mode) m/z: found 693.97 (M-TEA)+, calculated for $C_{40}H_{49}N_6O_5$, 693.85.
UV-vis (MeOH) λmax/nin (absorption): 628 (0.018), 574 (0.042), 538 (0.055), 504 (0.057), 399 (0.93).

(iii) Production of compound represented by formula 5(8)

The compound 4(8) (109 mg, $1.4\times10^{-4}$ mol), iron(II) chloride monohydrate (630 mg), and sodium hydrogencarbonate (40 mg) were added to a 50 mL recovery flask, dissolved in a mixed solvent of chloroform and methanol (chloroform/methanol = 10/1, 20 mL) saturated with nitrogen, and heated to reflux. Four hours later, the reaction mixture was cooled to room temperature, and the solvent was distilled off under reduced pressure. The residue was dissolved in a mixed solvent of chloroform and methanol (chloroform/methanol = 2/1) and washed with 0.05 M hydrochloric acid. The organic layer was separated and then dried over anhydrous sodium sulfate, and the solvent was distilled off under reduced pressure. The resulting residue was dissolved by adding a minimum amount of methanol, the precipitate formed by adding diethyl ether to the solution was recovered, thoroughly washed with water and dried, and thus a mixture of the title compound 5(8) and a positional isomer thereof was obtained (92 mg, 80%, purple, solid).

ESI-TOF-MS (positive mode) m/z: found, 746.81 (M-Cl-HCl)+, calculated for $C_{40}H_{46}FeN_6O_5$, 746.68.
UV-vis (MeOH) λmax/nm (absorption): 627 (0.021), 538 (0.035, sh), 502 (0.053), 397 (0.91).

(iv) Production of compound represented by formula 1(8)

The compound 5(8) (40 mg, $5.4\times10^{-5}$ mol) and *N*-methoxycarbonylmaleimide (100 mg, $6.45\times10^{-4}$ mol) were dissolved in a mixed solvent of acetone and a saturated aqueous sodium hydrogen carbonate solution (acetone/saturated aqueous sodium hydrogen carbonate solution = 2/1, 10 mL) in a 100 mL recovery flask. After the solution was stirred at room temperature for 2 hours, water (40 mL) was added and stirring was performed for 1 more hour. To the mixture were added an aqueous 0.1 N HCl solution and then chloroform and the mixture was extracted with chloroform. The combined organic layers were dried over anhydrous sodium sulfate and the solvent was distilled off under reduced pressure. The resulting residue was purified by silica gel chromatography (chloroform/methanol = 5/1). The resulting solids were dissolved in a minimum amount of chloroform, the precipitate formed by adding hexane to the solution was recovered, and thus a mixture of the title compound 1(8) and a positional isomer thereof was obtained (25 mg, 57%, deep purple, solid).

ESI-TOF-MS (positive mode) m/z: found, 826.30 $(M-Cl-)^+$, calculated for $C_{44}H_{46}FeN_6O_7$, 826.28.

UV-vis ($CHCl_3$/MeOH =1/1, v/v) λmax/nm (absorption): 597 (0.058), 482 (0.090), 399 (0.99).

(2) Preparation of cytochrome $b_{562}$ mutant (H63C)

A site-specific mutant was generated by a polymerase chain reaction (PCR) using an LA PCR in vitro mutagenesis kit manufactured by Takara Bio Inc., according to the accompanying protocol. *E. coli* TG1 having an expression plasmid (pUC118) for wild-type cytochrome $b_{562}$ (hereinafter abbreviated as $b_{562}$) was mass-cultured to prepare a plasmid that was used as a template for the preparation of an H63C mutant. Using a primer for introducing a mutation site (SEQ ID NO. 5):

[Chemical Formula 26] 5'-AAGATTTCCGCTGCGGTTTC-3'

(the underlined portion indicates a mismatched base pair) and an M13 primer M4 (SEQ ID NO. 6) (5'-GTTTTCCCAGTCACGAC-3') as well as an M13 primer RV SEQ ID NO. 7 (5'-CAGGAAACAGCTATGAC-3') and an MUT4 primer SEQ ID NO. 8 (5'-GGCCAGTGCCTAGCTTACAT3'), a first-stage DNA amplification was carried out by PCR in the respective systems. A heteroduplex DNA was prepared from the two first-stage PCR products, and a second-stage amplification was carried out by the PCR of the heteroduplex DNA using an M13 primer RV and an M13 primer M4. ADNA fragment having a base sequence for a $b_{562}$ mutant was excised with restriction enzymes EcoRI and HindIII and specifically connected to the EcoRI/HindIII sites of the pUC118 vector. Thereafter, an *E. coli* (*Escherichia coli*) strain TG1 was transformed by the expression plasmid thus obtained. The base sequence of the H63C mutant was determined by DNA sequencing (SEQ ID NO. 4). At this time, mutation was found also at the position ofAla37 but it was a silent mutation (GCC mutated to GCG). The mutant protein was expressed in large amounts using the *E. coli* strain TG1 according to a paper (Y Kawamata, S. Machida, T. Ogawa, K. Horie, T. Nagamune; J. Lumin. 98, 141 (2002)) in the same manner as in the expression of the wild-type $b_{562}$. The expressed protein was purified with a cation-exchange column (CM-52, 2.7×18 cm) and a gel filtration column (Sephadex G-50, 1.5×100 cm). The fraction having Rz =$A_{418}/A_{280}$ ≥ 6.0 (stock solution of H63C) was collected and used in the following experiment.

(3) Production of protein monomer and protein polymer containing the monomer as a monomer unit (see scheme 6)

[Chemical Formula 27]

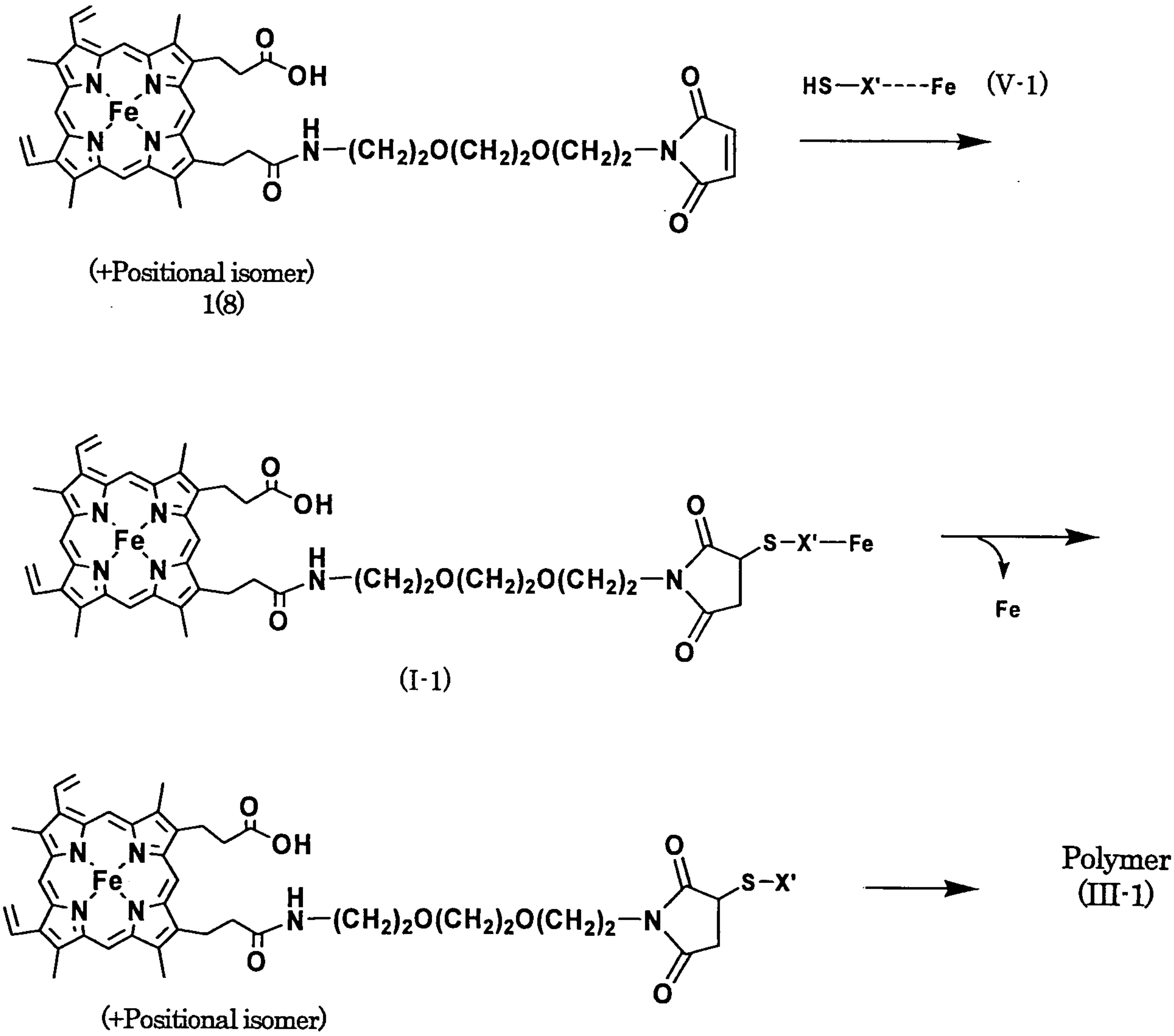


(+Positional isomer)
1(8)

(I-1)

(+Positional isomer)

(II-1)

Polymer
(III-1)

Scheme 6

Formula (V-1):

[Chemical Formula 28] **HS-X'----Fe** (V-1)

indicates the protein mutant expressed in Example 1(2), namely cytochrome $b_{562}$ mutant (H63C), having the same amino acid sequence as native cytochrome $b_{562}$ except that the histidine residue at the 63rd position is replaced with a cysteine residue. In the mutant, -SH represents a side-chain thiol group of the cysteine residue, and Fe is bonded to the four nitrogen atoms of a porphyrin group contained in the hemoprotein.

Under a nitrogen atmosphere, 1.9 mL of a nitrogen-saturated Tris-HCl buffer (0.05 M, pH 7.3) and a DMSO solution (0.6 mL) of the compound 1(8) ($2.6\times10^{-6}$ mol) were added to a 30 mL 2-neck flask and stirred at room temperature. A stock solution of the H63C expressed in Example 1(2) (200 μL, concentration in 0.05M Tds-HCl buffer of $1.6\times10^{-3}$M, pH 7.3) was added dropwise thereto and the solution was stirred gently under nitrogen at room temperature. 1.5 hours later, a protein monomer (I-1) was obtained in which the compound 1(8) and the H63C (V-1) were linked.

Hydrochloric acid was added to the solution of the protein monomer (I-1) so as to adjust a pH to 1.9, giving a protein monomer (II-1). An extraction operation was performed on the aqueous solution of the protein monomer (II-1) by adding 2-butanone (5 mL×4). The aqueous layers were separated and transferred to a dialysis membrane (Wako,

MWCO, 14,000 Da), and dialysis was performed at 4°C with a 0.05 M Tris-HCl buffer (pH 7.3) (500 mL×2 hours×3). An aqueous solution of the resulting protein polymer (III-1) was concentrated to about $10^{-3}$ M by ultrafiltration and kept in a cool, dark place.

It can be presumed that the protein polymer (III-1) has a random copolymer structure as shown in formula (III-11).

[Chemical Formula 29]

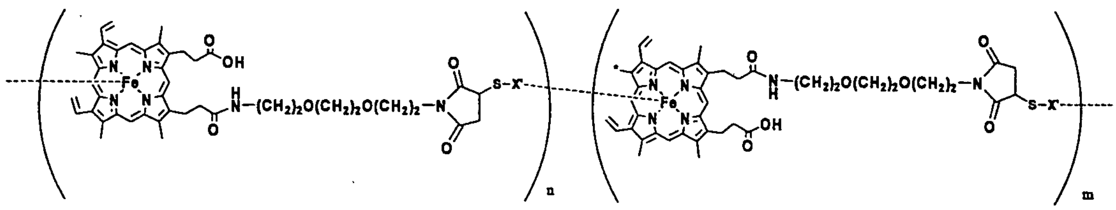

(III-11)

In the formula, X' represents X' that is present in a hemoprotein mutant, and the hemoprotein mutant is represented by the formula (V-1).

Formula (V-1):

[Chemical Formula 30] **HS-X'----Fe** (V-1)

indicates the protein mutant expressed in Example 1(2), i.e., the cytochrome $b_{562}$ mutant (H63C), having the same amino acid sequence as native cytochrome $b_{562}$ except that the histidine residue at the 63rd position is substituted with a cysteine residue. In the mutant, -SH represents a side-chain thiol group of the cysteine residue, and Fe is bonded to the four nitrogen atoms of a porphyrin group contained in the hemoprotein.

Example 2

(1) Production of compound represented by formula 1(2)

A compound represented by formula 1(2) was produced according to scheme 7 below. Specifically, production was carried out in the same manner as in the production of the compound represented by formula 1(8) of Example 1(1) except that N-Boc-1,2-diaminoethane (diamine (2)) was used in place of N-Boc-1,2-bis(2-aminoethoxy)ethane (diamine (8)).

[Chemical Formula 31]

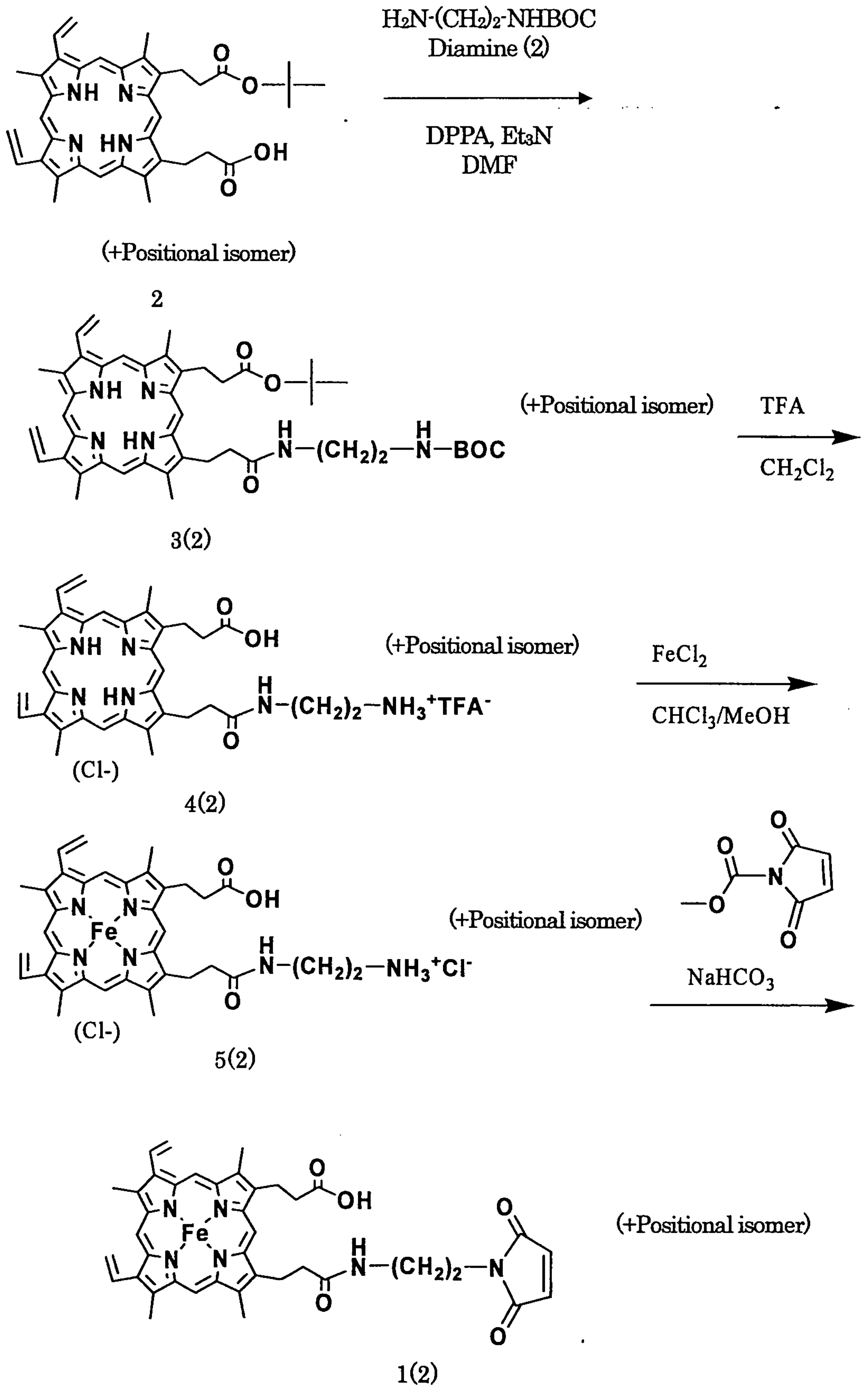


(+Positional isomer)

2

(+Positional isomer)

3(2)

(+Positional isomer)

4(2)

(+Positional isomer)

5(2)

(+Positional isomer)

1(2)

Scheme 7

Data of the compound 3(2), the compound 4(2), the compound 5(2), and the compound 1(2) thus obtained are presented below.

Compound 3(2): yield 33%.

$^1$H NMR (270 MHz, pyridine-$d_5$) δ:10.47 (s, 1H), 10.38 (s, 1H), 10.36 (s, 0.5H), 10.30 (s, 0.5H), 10.21 (s, 0.5H), 10.16 (s, 0.5H), 8.51-8.38 (m,2H), 6.45-6.16 (m, 4H), 4.60-4.45 (m, 4H), 3.65-3.41 (m, 16H), 3.43 (m, 2H), 3.33 (m, 2H), 1.33 (s, 9H), 1.26 (s, 9H) -3.39 (s, 2H).

ESI-TOF-MS (positive mode) m/z: found 761.70 (M + H)+, calculated for $C_{45}F_{57}N_6O_5$, 761.97.

UV-vis ($CHCl_3$) λmax/nm (absorption): 630 (0.032), 575 (0.041), 542 (0.070), 506 (0.084), 408(1.02).

Compound 4(2): yield 88%.

$^1$H NMR (270 MHz, pyridine-$d_5$) δ:10.64 (s, 1H), 10.44 (s, 1H), 10.36 (m, 1H), 10.25 (m, 1H), 8.52-8.42 (m, 2H), 6.47-6.17 (m, 4H), 4.63-4.50 (m, 4H), 3.67-3.52 (m, 16H), 3.42-3.28 (m, 4H)-3.29 (s, 2H).

ESI-TOF-MS (positive mode) m/z: found 605.31 (M-TEA)+, calculated for $C_{36}H_{41}N_6O_3$, 605.75.

UV-vis (MeOH) λmax/nm (absorption): 627 (0.034), 574 (0.048), 537 (0.087), 503 (0.10), 401(1.12).

Compound 5(2): yield 93%.

ESI-TOF-MS (positive mode) m/z: (M-Cl-HCl)+ calculated for $C_{36}H_{38}FeN_6O_3$, 658.57; found 658.21.

UV-vis (MeOH) λmax/nm (absorption): 598 (0.082), 478 (0.13), 398 (1.02).

Compound 1(2): yield 31%.

ESI-TOF-MS (positive mode) m/z: found 738.38 (M-$Cl^-$)+, calculated for $C_{40}H_{38}FeN_6O_5$, 738.23.

UV-vis ($CHCl_3$/MeOH =1/1, v/v) $\lambda_{max}$/nm (absorption): 609 (0.029), 498 (0.067), 399 (0.96).

(2) Production of protein monomer and protein polymer containing the monomer as a monomer unit (see scheme 8)

Using the compound represented by formula 1(2), a protein monomer and a protein polymer (III-2) that contains the monomer as a monomer unit were produced according to scheme 8 below. Specifically, production was carried out in the same manner as in the production depicted in scheme 6 of Example 1(3) except that the compound 1(2) was used in place of the compound 1(8).

[Chemical Formula 32]

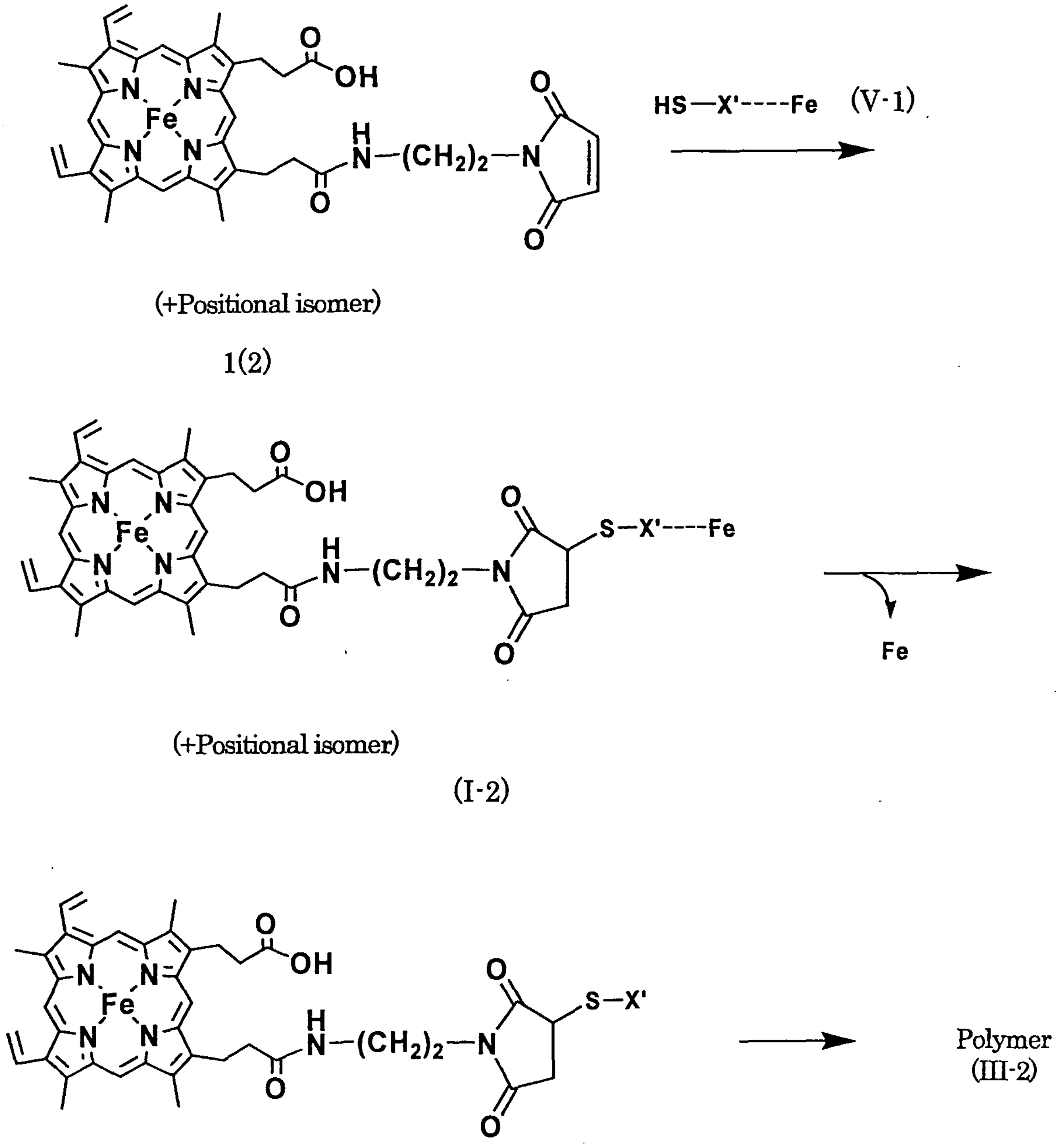


(+Positional isomer)

1(2)

(+Positional isomer)

(I-2)

(+Positional isomer)

(II-2)

Scheme 8

It can be presumed that the protein polymer (III-2) has a random copolymer structure as shown in formula (III-12).

[Chemical Formula 33]

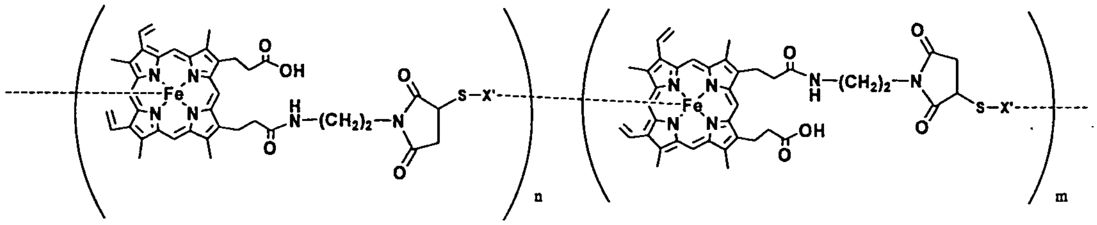

(III-12)

In the formula, X' represents X' that is present in a hemoprotein mutant, and the hemoprotein mutant is represented by the formula (V-1).
The formula:

[Chemical Formula 34] **HS-X'----Fe**

is as described above.

Example 3

(1) Preparation of myoglobin mutant (A125C) dimer

*E. coli* having a plasmid for a mutant protein was prepared according to a formulation described in a paper (S. Hirota, K. Azuma, M. Fukuda, S. Kuroiwa, N. Funasaki; Biochemistry 44, 10322 (2005)). The mutant protein was expressed in large amounts using an *E. coli* strain TB-1 according to a formulation described in a paper (B.A. Springer, S.G. Sliger; Proc. Natl. Acad. Sci. USA 84, 8961 (1987)) in the same manner as in the expression of a wild-type myoglobin. The expressed protein was purified with an anion-exchange column (DEAE Sepharose FF, 2.7 cm×10 cm, a cation-exchange column (CM-52, 2.7 cm×18 cm), and a gel filtration column (Sephadex G-50, 1.5 cm×100 cm), the fraction of a sperm whale-derived myoglobin mutantA125C dimer was recovered and used in the following experiment.

(2) Production of protein monomer and protein polymer containing the monomer as a monomer unit (see scheme 9)

[Chemical Formula 35]

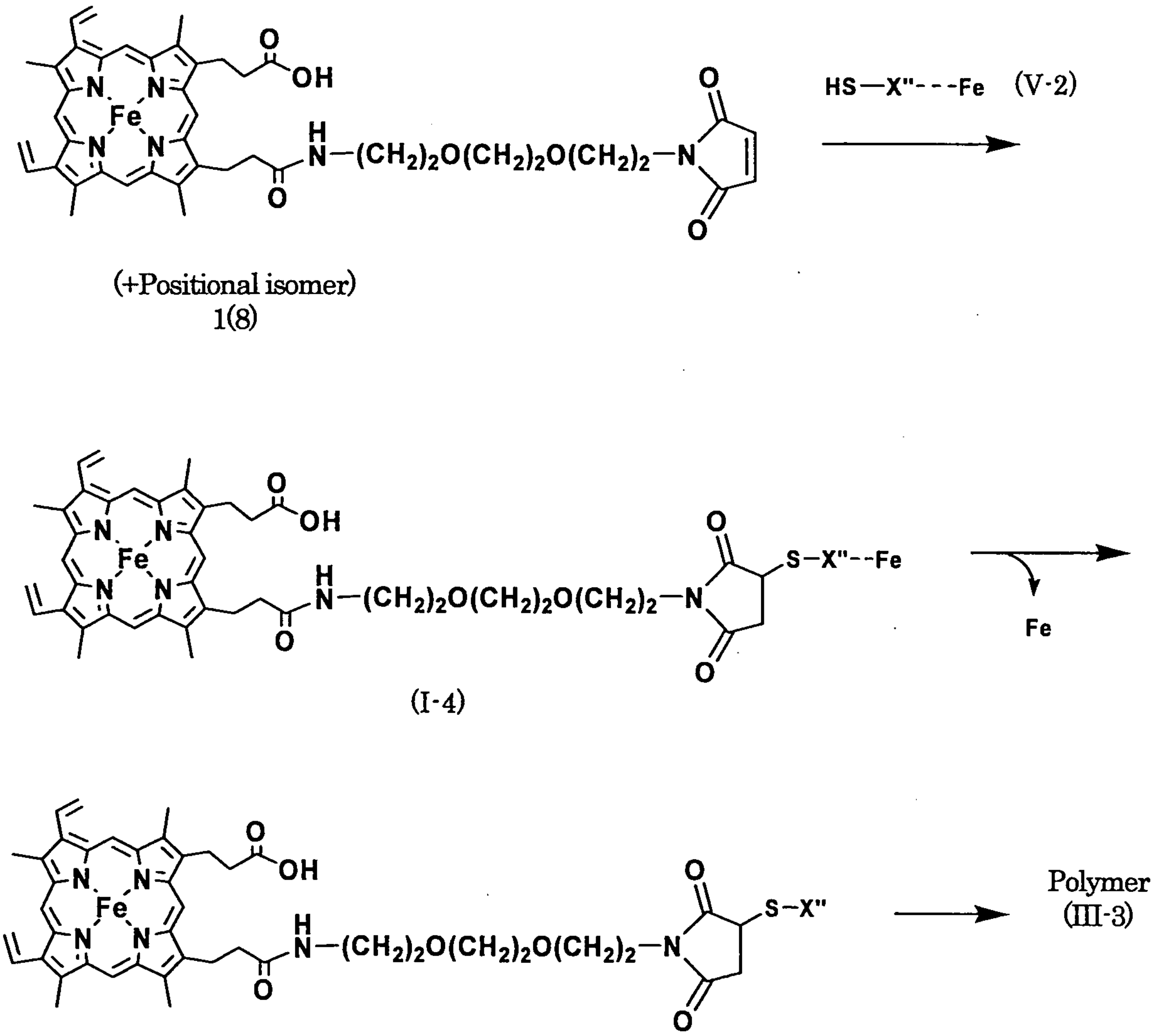


(+Positional isomer)
1(8)

(I-4)

(+Positional isomer)

(II-4)

Scheme 9

Formula (V-2):

[Chemical Formula 36] **HS-X"---Fe** (V-2)

indicates a monomer derived from the protein mutant expressed in Example 3(1), i.e., a sperm whale-derived myoglobin mutant (A125C), having the same amino acid sequence as a wild-type myoglobin (SEQ ID NO.2) except that the alanine residue at the126th position is replaced with a cysteine residue. In the mutant, -SH represents a side-chain thiol group of the cysteine residue, and Fe is bonded to the four nitrogen atoms of a porphyrin group contained in the hemoprotein.

The a stock solution of the A125C dimer prepared in Example 3(1) (200 μL, $1.10\times10^{-3}$ M (concentration in terms of monomer), a 0.1 M phosphate buffer, pH 7.0) was added DTT (dithiothreitol) in a large excess, dissolved, and then left to stand still for 30 minutes at 2°C. This solution was purified with a desalting/buffer exchange column (HiTrap Desalting (registered trademark), manufactured by GE Healthcare Biosciences) equilibrated with a 0.1 M aqueous L-histidine solution, thereby giving an A125C monomer (V-2). The A125C monomer (V-2) was replenished with a 0.1 M aqueous L-histidine solution so as to reach 1.5 mL, thus preparing anA125C monomer solution. A solution of the compound 1(8) ($1.1\times10^{-6}$ mol) was added to the A125C monomer solution and the mixture was stirred for 24 hours at room temperature, thereby giving a protein monomer (1-4) in which the compound 1(8) and the A125C monomer (V-2) were

bonded. The aforementioned solution of the compound 1(8) was prepared by dissolving the compound 1(8) ($1.1\times10^{-6}$ mol) in a mixed solvent (0.6 mL) of a 0.1 M aqueous L-histidine solution, DMSO, and dithionite (0.1 M aqueous L-histidine solution:DMSO = 6:1, dithionite was used in an amount equivalent to the A125C monomer). All the operations described above were performed in a glove box under a nitrogen atmosphere.

The reaction solution was transferred to a dialysis membrane (Wako, MWCO, 14,000 Da) and dialysis was performed at 4°C with a phosphate buffer (0.01 M, pH 6.0) (1 L×1 hour×2). 0.1 N hydrochloric acid was added to a solution of the protein monomer (1-4) obtained from the dialysis so as to adjust a pH to 2.25, giving a protein monomer (II-4). An extraction operation was performed on a solution of the protein monomer (II-4) by adding 2-butanone (5 mL×4). Aqueous layers were separated and transferred to a dialysis membrane (Wako, MWCO, 14,000 Da) and dialysis was performed at 4°C with a phosphate buffer (0.1 M, pH 7.0) (1 L×2 hours×3). An aqueous solution of the resulting protein polymer (III-3) was centrifuged to removed precipitates, and the protein polymer (III-3) supernatant was concentrated to about $10^{-3}$ M by ultrafiltration and kept in a cool, dark place.

It can be presumed that the protein polymer (III-3) has a random copolymer structure as shown in formula (III-13).

[0182] [Chemical Formula 37]

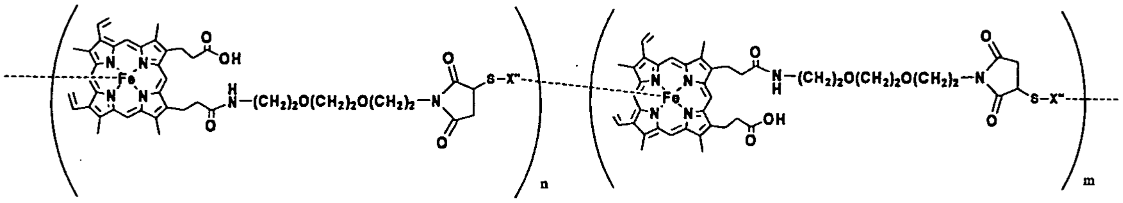

(III-13)

In the formula, X" represents X" that is present in a hemoprotein mutant, and the hemoprotein mutant is represented by the formula (V-2).

The formula:

[Chemical Formula 34] **HS-X"---Fe** (V-2)

is as described above.

Example 4

(3) Production of protein monomer and protein polymer containing the monomer as a monomer unit (see scheme 10)

Using the compound represented by formula 1(2), a protein monomer and a protein polymer (III-4) that contains the monomer as a monomer unit were produced according to scheme 10 below. Specifically, production was carried out in the same manner as the production depicted in scheme 9 of Example 3(2) except that the compound 1(2) was used in place of the compound 1(8).

[Chemical Formula 39]

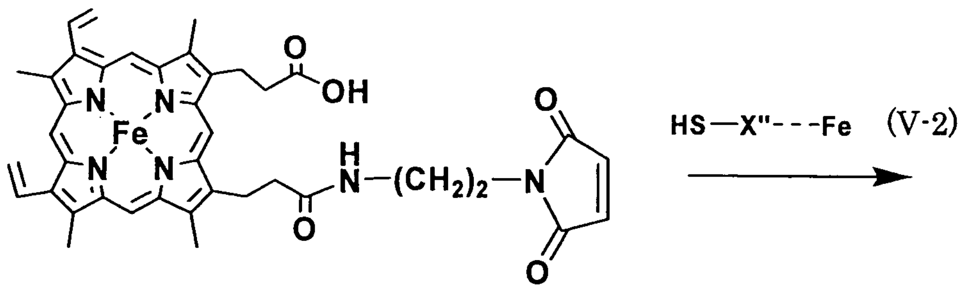


(+Positional isomer)
1(2)

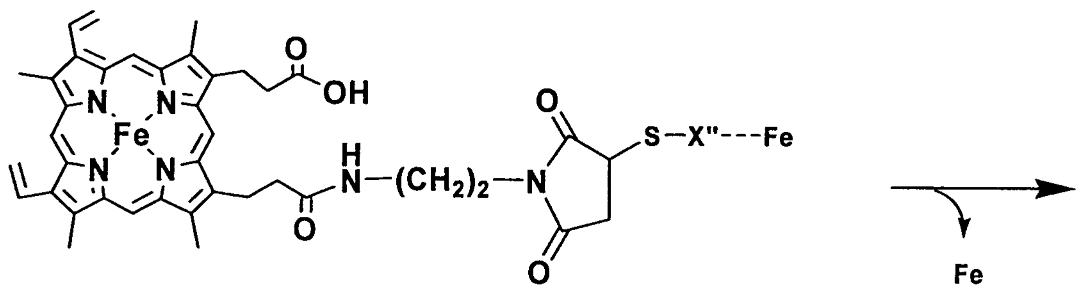


(+Positional isomer)

(I-5)

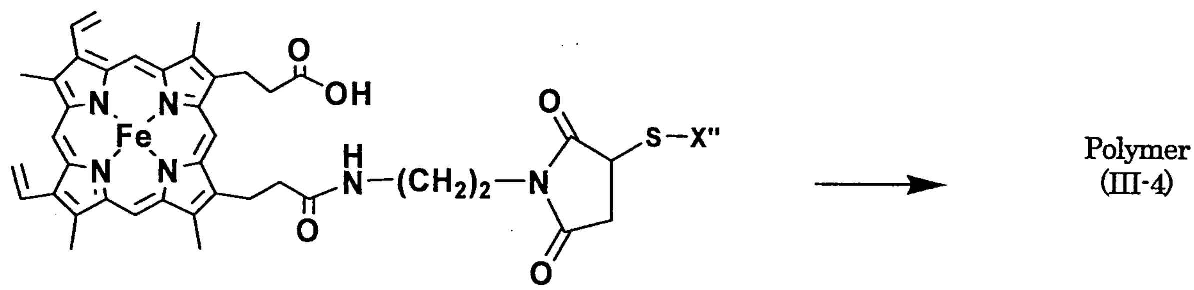


(+Positional isomer)

(II-5)

Scheme 10

It can be presumed that the protein polymer (III-4) has a random copolymer structure as shown in formula (III-14).

[Chemical Formula 40]

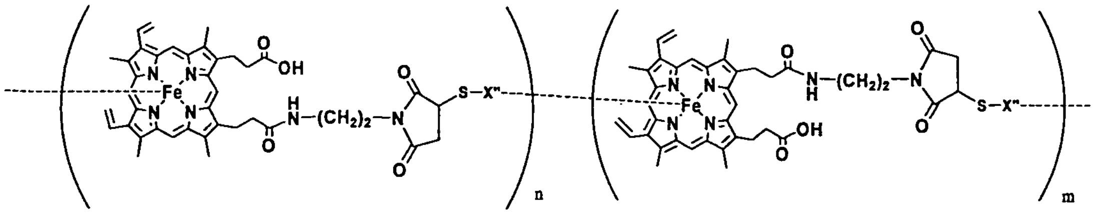

(III-14)

In the formula, X" represents X" that is present in a hemoprotein mutant, and the hemoprotein mutant is represented by the formula (V-2).
The formula:

[Chemical Formula 41] **HS-X"---Fe** (V-2)

is as described above.

Example 5

(i) Production of compound represented by formula 1(13)

A compound represented by formula 1(13) was produced according to scheme 11 below. Specifically, production was carried out in the same manner as in the production of the compound represented by formula 1(8) of Example 1(1) except that N-BOC-diethyleneglycol-bis(3-aminoprppyl)ether (diamine (13)) was used in place of N-Boc-1,2-bis(2-aminoethoxy)ethane (diamine (8)) (see scheme 5).

[Chemical Formula 42]

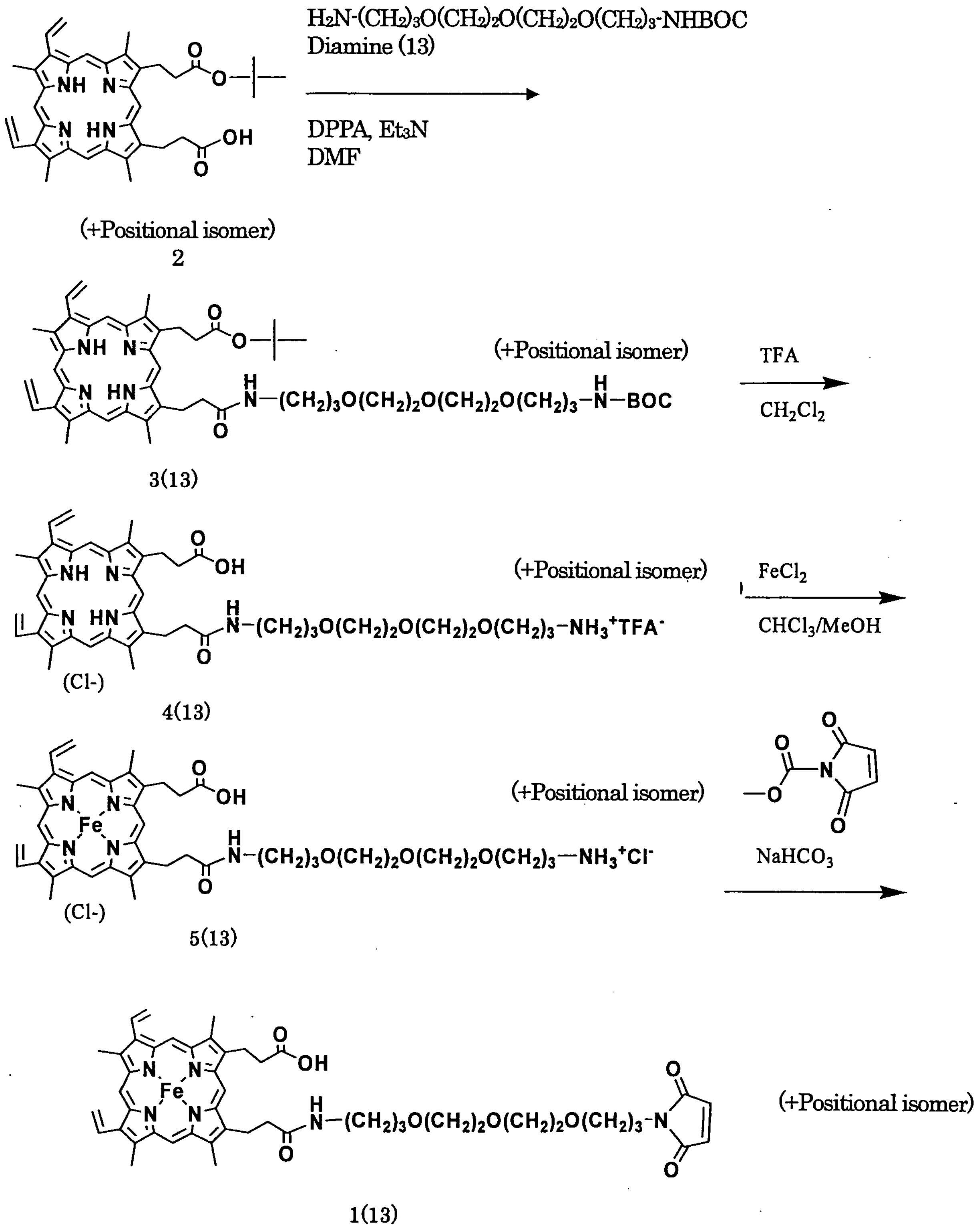


Scheme 11

Data of the compound 3(13), the compound 4(13), the compound 5(13), and the compound 1(13) thus obtained are presented below.

Compound 3(13): yield 49%.

$^1$H-NMR (270 MHz, pyridine-$d_5$) δ: 10.34 (s, 1H), 10.21 (s, 0.5H), 10.17 (s, 1H), 10.14 (s, 1H), 10.03 (s, 0.5H), 9.95 (s, 0.5H), 8.39-8.25 (m, 2H), 6.41-6.14 (m, 4H), 4.60-4.38 (m, 4H), 3.62-3.45 (m, 16H), 3.34-3.15(m, 12H), 3.06 (m, 2H,

2.76 (m, 2H), 2.61 (m, 2H), 2.44 (m, 2H), 1.74 (m, 2H), 1.49 (s, 9H), 1.43 (s, 9H), 1.39 (m, 2H), -3.80 (s, 2H). ESI-TOF-MS (positive mode) m/z: found 921.83 (M+H)+, calculated for $C_{53}H_{72}N_6O_8$, 922.18.
UV-vis ($CHCl_3$) λmax/nm (absorption): 630 (0.032), 576 (0.042), 540 (0.072), 506 (0.088), 408(1.05).

Compound 4(13): Yield 82%.
$^1H$ NMR (270 MHz, pyridine-$d_5$) δ: 10.41 (s, 1H), 10.19 (s, 0.5H), 10.14 (br, 1.5H), 10.00 (s, 0.5H), 9.94 (s, 0.5H), 8.42-8.27 (m, 2H), 6.43-6.12 (m, 4H), 4.60-4.41 (m, 4H), 3.61-3.47 (m, 16H), 3.33-3.22 (m, 8H), 3.13 (m, 2H), 2.93 (m, 2H), 2.80 (m, 2H), 2.71 (m, 2H), 1.90 (m, 2H), 1.44 (m, 2H) -3.88 (s, 2H).
ESI-TOF-MS (positive mode) m/z: found 765.55 (M-TEA)+, calculated for $C_{44}H_{57}N_6O_6$, 765.96.
UV-vis (MeOH) λmax/nm (absorption): 628 (0.035), 574 (0.044), 538 (0.073), 504 (0.090), 401(1.02).

Compound 5(13): The compound 5(13) was highly water soluble, and thus water washing of the precipitate at the end could not be performed sufficiently. Therefore, the compound was used in the subsequent reaction without purification. ESI-TOF-MS (positive mode) m/z: found 818.70 ($M-Cl^-$-HCl)+, calculated for $C_{44}H_{54}FeN_6O_6$, 818.72.
UV-vis (MeOH) λmax/nm (absorption): 601 (0.037), 494 (0.080), 396 (1.07).

Compound 1(13): Yield 43%.
ESI-TOF-MS (positive mode) m/z: found 898.38 ($M-Cl^-$)+, calculated for $C_{44}H_{46}FeN_eO_7$, 898.34.
UV-vis ($CHCl_3$/MeOH = 1/1, v/v) λmax/nm (absorption): 596 (0.064), 483 (0.087), 398 (0.78).

(2) Production of protein monomer and protein polymer containing the monomer as a monomer unit (see scheme 12)

Using the compound represented by formula 1(13), a protein monomer and a protein polymer (III-5) that contains the monomer as a monomer unit were produced according to scheme 12 below. Specifically, production was carried out in the same manner as the production depicted in scheme 9 of Example 3(2) except that compound 1(13) was used in place of compound 1(8).

[Chemical Formula 43]

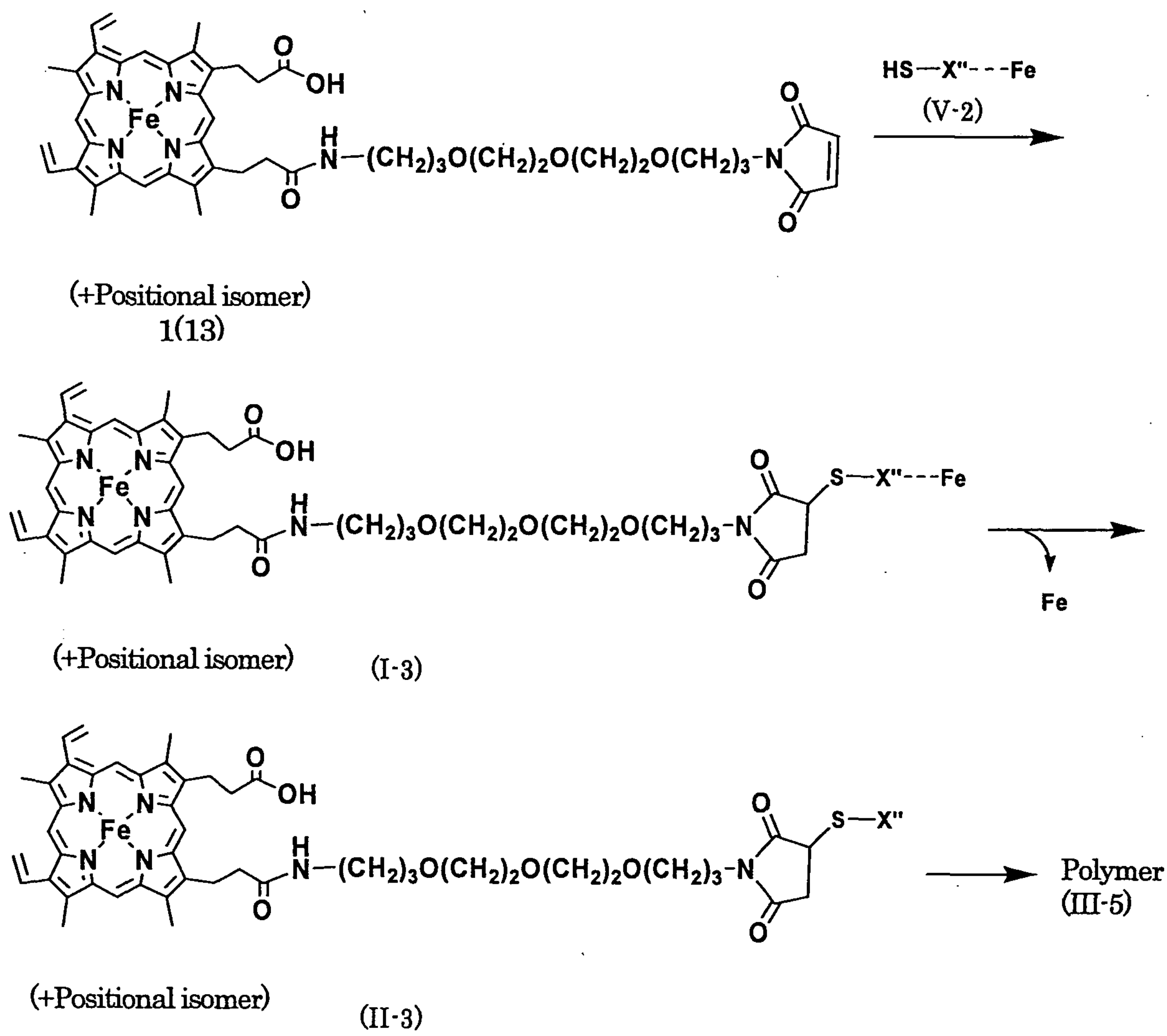


(+Positional isomer)
1(13)

(+Positional isomer) (I-3)

Polymer (III-5)

(+Positional isomer) (II-3)

Scheme 12

It can be presumed that the protein polymer (III-5) has a random copolymer structure as shown in formula (III-15).

[Chemical Formula 44]

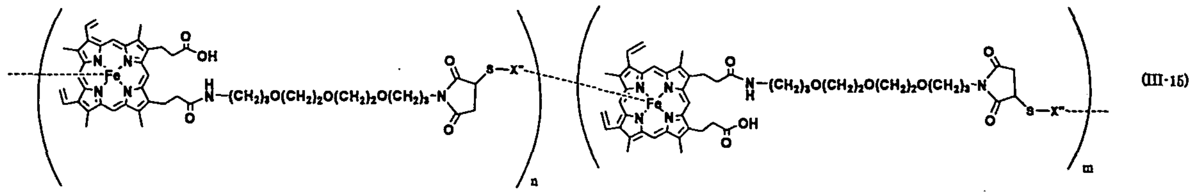

(III-15)

In the formula, X" represents X" that is present in a hemoprotein mutant, and the hemoprotein mutant is represented by the formula (V-2).

The formula:

[Chemical Formula 45] **HS-X"---Fe** (V-2)

is as described above.

[Various properties of polymers obtained in Examples 1 to 5]

(i) Measurement of UV-vis spectrum

Fig. 1a shows a UV-vis spectrum of a native myoglobin, Fig. 1b shows a UV-vis spectrum of the protein polymer (III-4) produced in Example 4, Fig. 1c shows a UV-vis spectrum of the protein polymer (III-3) produced in Example 3, and Fig. 1d shows a UV-vis spectrum of the protein polymer (III-5) produced in Example 5. It was confirmed from Figs. 1a, 1b, 1c, and 1d that the spectra of the protein polymer (III-4), the protein polymer (III-3), and the protein polymer (III-5) are very similar to that of a native myoglobin, and it thus was confirmed that the iron present in the protein polymer (III-4), the protein polymer (III-3), and the protein polymer (III-5) in which a porphyrin linker had been introduced into a mutant myoglobin was retained in the hem pocket.

In addition, Fig. 1e shows a UV-vis spectrum of an oxygen complex of a native myoglobin, and Fig. 1f shows a UV-vis spectrum of an oxygen complex of the protein polymer (III-5) produced in Example 5. It is known that myoglobin, which can store oxygen, atmospherically forms very stable oxygen complexes and exhibits a distinctive ultraviolet-visible absorption spectrum once the heme iron is reduced by a reducing agent to a divalent form. An oxygen complex was prepared by similarly treating the protein polymer (III-5) produced herein. Fig. 1f for this complex and Fig. 1e show a similar distinctive absorption at 417 nm, 543 nm, and 580 nm. It was therefore confirmed that the protein polymer of the present invention forms a very stable oxygen complex. Accordingly, it also was confirmed that the protein polymer of the present invention is in the form of a supramolecular assembly while maintaining the inherent function of the protein.

(ii) Measurement of affinity with oxygen

An oxygen complex of a native myoglobin and an oxygen complex of the oxygen-bound protein polymer (III-5) produced in Example 5 (a Fe(II) heme-oxygen complex of a myoglobin, oxy-Mb) were obtained by adding excessive amounts of dithionite to a native myoglobin and a solution of the protein polymer produced in Example 5 (a Fe(III) heme complex of a myoglobin, met-Mb) and then purifying them with Sephadex G25 columns. Kinetics measurements were performed at 25°C in a phosphate buffer (100 mM, pH 7.0). The auto-oxidation process was monitored at 37°C. Oxygen binding constant $K^{O2}$ can be obtained by dividing binding rate constant $K^{O2}_{on}$ by dissociation rate constant $K^{O2}_{off}$. The results obtained from the following measurements are presented in Table 1. Note that the protein polymer (III-5) was a polymer in which 11 protein monomers (n=11) were polymerized. Calculation was made using the entire protein polymer (III-5) as one molecule.

[Measurement of oxygen binding rate constant $K^{O2}_{on}$]

An oxygen complex of a native myoglobin and an oxygen complex of the oxygen-bound protein polymer (III-5) produced in Example 5 each in a phosphate buffer (100 mM, pH 7.0) were excited by laser flash photolysis (excitation wavelength of 532 nm, 5 ns pulse) in air ($[O_2] = 2.64 \times 10^{-4}$M). Thereafter, a change in absorbance at 434 nm, which is the wavelength of maximum absorption of both native myoglobin and the protein polymer produced in Example 5 (a Fe (II) heme complex of a myoglobin, deoxy-Mb), was investigated. The probe beam was passed through a monochromator. Fitting for a reaction curve was performed according to the nonlinear least squares method to determine the first-order reaction rate constant. The resulting rate constant then was divided by the concentration of $O_2$ to calculate an oxygen binding rate constant $K^{O2}_{on}$.

[Measurement of oxygen dissociation rate constant $K^{O2}_{off}$]

The oxygen dissociation rate was obtained according to the potassium ferricyanide method. A stopped-flow instrument was used in the measurement and the probe beam was passed through a monochromator. Fitting of a change in absorbance at 580 nm was carried out according to first-order kinetics in a $[K_3Fe(CN)_6]$>>Mb] condition. Using mathematical formula (1) below, $K^{O2}_{off}$ was determined from the rate constant observed in a condition where $K_3Fe(CN)_6$ was in a large excess.

[Mathematical Formula 1]

$$MbO_2 \underset{k^{O2}_{on}}{\overset{k^{O2}_{off}}{\rightleftharpoons}} O_2 + \text{deoxy-Mb} + K_3[Fe(CN)_6] \xrightarrow{k_{ox}} \text{met-Mb}$$

In the mathematical formula, $k_{ox}$ is a rate constant for oxidation from the deoxy form to the met form. If the steady-state approximation is applied, the reaction proceeds with the first-order reaction constant, and an apparent rate constant $k_{obs}$ can be expressed as mathematical formula (2):

[Mathematical Formula 2]

$$k_{obs} = \frac{k^{O2}_{off} k_{ox}[K_3[Fe(CN)_6]]}{k_{ox}[K_3[Fe(CN)_6]] + k^{O2}_{on}[O_2]} = \frac{k^{O2}_{off}}{1 + k^{O2}_{on}[O_2] / k_{ox}[K_3[Fe(CN)_6]]}$$

If the condition of $k_{ox}[K_3[Fe(CN)_6]] >> K^{O2}_{on}[O_2]$ is satisfied, an oxygen dissociation rate constant $K^{O2}_{off}$ can be calculated as below:

[Mathematical Formula 3]

$$k_{obs} = k^{O2}_{off}$$

[Measurement of auto-oxidation rate constant $k_{auto}$]

UV-vis spectra were measured at 37°C within a range of 500 to 650 nm every 30 minutes using as samples an oxygen complex of a native myoglobin and an oxygen complex of the protein polymer (III-5) produced in Example 5. A change in absorbance at 580 nm was plotted against time and fitted according to first-order kinetics to calculate an auto-oxidation rate constant $k_{auto}$. The results thus obtained are presented in Table 1.

[Table 1]

| | $k^{O2}_{on}$ (/μMs) | $k^{O2}_{off}$ (/S) | $K^{O2}$ (/M) | $k_{auto}$ (/hr) |
|---|---|---|---|---|
| Protein polymer (III-5) | $3.8\times10^2$ | 20 | $1.9\times10^7$ | 0.12 |
| Native myoglobin | 18 | 21 | $8.6\times10^5$ | 0.10 |

It was confirmed from the results presented in Table 1 that the protein polymer of the present invention has a greater affinity for oxygen than a native myoglobin.

(iii) Size exclusion chromatography measurement

The protein polymers obtained in Examples 3 to 5 were subjected to size exclusion chromatography. A 100 mM phosphate buffer (pH 7.0) was used as an eluent. Measurements were carried out at a temperature of 4°C at a flow rate of 0.5 mL/min. Fig. 2a shows the results. As shown in Fig. 2a, a component that eluted sooner had a larger molecular weight. The elution volume of a myoglobin mutant A125C monomer was 17.6 mL, and the elution volume of a myoglobin mutant A125C dimer was 16.2 mL (not shown). The elution volumes of the protein polymer (III-3), the protein polymer (III-4), and the protein polymer (III-5) were 10.5 mL, 11.3 mL, and 9.3 mL, respectively. It therefore was confirmed that the polymers had large molecular weights because the elution volumes of the protein polymers were smaller than those of the monomer and the dimer of the myoglobin mutant A125C. Also, it was confirmed that, regarding the protein polymer (III-3), the protein polymer (III-4), and the protein polymer (III-5), the longer the linker connecting the protein and the heme, the larger the molecular weight of a polymer. It was also confirmed that since the chromatogram of the protein

polymer (III-5) had a sharp rise at 8 mL, which was the detection limit, the protein polymer (III-5) contained components having larger molecular weights than the protein polymer (III-3) and the protein polymer (III-4). Meanwhile, since there is a proportional relationship between the common logarithm of molecular weight and the elution volume, it is possible to roughly calculate the molecular weights of the protein polymer (III-3), the protein polymer (III-4), and the protein polymer (III-5). From their molecular weights and the molecular weights of their monomers, the degrees of polymerization of the protein polymer (III-3), the protein polymer (III-4), and the protein polymer (III-5) were calculated to be 22, 18, and 33, respectively.

The protein polymer (III-1) produced in Example 1 and the protein polymer (III-2) produced in Example 2 were subjected to size exclusion chromatography A mixture prepared by adding 0.15 M NaCl to a 50 mM Tris-HCl buffer (pH 7.3) was used as an eluent. Measurements were carried out at a temperature of 4°C at a flow rate of 0.5 mL/min. Fig. 2b shows the results for the protein polymer (III-1) and the protein polymer (III-2). In Fig. 2b, a is a chromatogram of protein polymer (III-2), b is a chromatogram of protein polymer (III-3), c is a chromatogram of an H63C (V-1) dimer, and d is a chromatogram of the H63C (V-1) monomer. Presumably, the protein polymer (III-2) and the protein polymer (III-3) have huge molecular weights since a and b undergo sooner elution than c and d. Moreover, it was confirmed that the protein polymer (III-1) and the protein polymer (III-2) have broad molecular weight distributions since the peaks of a and b are broad.

The protein polymer obtained in Example 10 was subjected to size exclusion chromatography. An aqueous mixed solution of a Tris-HCl buffer (50 mM, pH 7.3) and NaCl (150 mM) was used as an eluent. Measurements were carried out at a temperature of 4°C at a flow rate of 0.5 mL/min. Fig. 2c shows the results. As shown in Fig. 2c, it was confirmed that, due to the interaction between Zn protoporphyrin and the heme pocket, the protein polymer (III-6) was mostly in the form of a dimer and a trimer.

(iv) Size exclusion chromatogram measurement under various concentration conditions

For the protein polymer (III-5) obtained in Example 5, a size exclusion chromatogram was measured in the same manner as in (ii) above using the polymer at a concentration of 10 μM, 100 μM, 500 μM, or 1000 μM. Fig. 3 shows the results. It was confirmed from Fig. 3 that the higher the concentration of the polymer (III-5), the longer the length of the protein polymer (III-5) and the smaller the amount of low molecular weight components in the protein polymer. This indicates that the molecular weight distribution of the protein polymer (III-5) is different depending on the concentration, and it thus can be understood that the molecular weight distribution is governed by thermodynamic equilibrium.

(v) Atomic force microscope (AFM) measurement

Samples used in the AFM measurement were prepared as follows. The surface of a high-orientation pyrolytic graphite (HOPG) substrate was cleaved and the cleaved surface was immersed for several seconds in an aqueous solution of a protein polymer obtained in Examples 3 to 5 (about $10^{-8}$M, in a 0.05 M Tris buffer, pH 7.3, or in a 0.1 M phosphate buffer, pH 7.0). The substrate was removed from the solution, sufficiently washed with water, sufficiently dried at room temperature in a calcium chloride-containing desiccator, and placed in an AFM measuring device. Measurements were carried out in a tapping mode at a scan rate of 2 Hz, and a single-crystal silicon probe having a radius of curvature of about 10 nm was used. Fig. 4a shows an AFM image of the protein polymer (III-4) obtained in Example 4, Fig. 4b shows an AFM image of the protein polymer (III-3) obtained in Example 3, Fig. 4c shows an AFM image of the protein polymer (III-5) obtained in Example 5, and Fig. 4d shows an AFM image of the protein polymer (III-2) obtained in Example 2.

Fig. 4a(a) shows an overall view of the protein polymer (III-4) and (b) shows a profile taken along the gray line in (a). Fig. 4b(c) shows an overall view of the protein polymer (III-3) and (d) shows a partially enlarged view and a cross-sectional profile thereof. Fig. 4c(e) shows an overall view of the protein polymer (III-5) and (f) shows a profile taken along the gray line in (e). An image showing liner polymeric structures of a uniform height was obtained for each of the three protein polymers. Moreover, a liner polymeric structure having a length corresponding to a few dozen monomers was also observed. Fig. 4d(a) shows an overall view of the protein polymer (III-2) and (b) shows an enlarged view of one of the structures observed in the overall view. It was verified from Fig. 4d(b) that this assembly had a length of about 200 nm and thus the structure was composed of about 80 monomers. The height of the assembly was in a range of 2.5 to 5 nm. This height corresponds to the height of one hemoprotein. Fig. 4d(c) shows an enlarged view of another structure observed in the overall view. This assembly was confirmed as having a doughnut shape. Fig. 4d(d) shows a profile taken along the gray line in (c). It was verified from (d) that all the assemblies had similar heights.

(vi) Mass spectrum measurement

Fig. 5 shows ESI-TOF-MS spectra and deconvoluted ESI-TOF-MS spectra of the aforementioned protein

monomers. Fig. 5(a) shows an ESI-TOF-MS spectrum of the protein monomer (II-5) obtained in Example 4 and Fig. 5 (b) shows a deconvoluted ESI-TOF-MS spectrum of the protein monomer (II-5). The molecular weight calculated for the protein monomer (II-5) was 18100.8. It was verified from Fig. 5(b) that the molecular weight of the protein monomer (II-5) was identical to the calculated value.

Fig. 5(c) shows an ESI-TOF-MS spectrum of the protein monomer (II-4) obtained in Example 3 and Fig. 5(d) shows a deconvoluted ESI-TOF-MS spectrum of the protein monomer (II-4). The molecular weight calculated for the protein monomer (II-4) was 18188.8. It was verified from Fig. 5(d) that the molecular weight of the protein monomer (II-4) was identical to the calculated value.

Fig. 5(e) shows an ESI-TOF-MS spectrum of the protein monomer (II-3) obtained in Example 5 and Fig. 5(f) shows a deconvoluted ESI-TOF-MS spectrum of the protein monomer (II-3). The molecular weight calculated for the protein monomer (II-3) was 18260.9. It was verified from Fig. 5(f) that the molecular weight of the protein monomer (II-3) was identical to the calculated value.

Example 6

Production of protein assembly or salt thereof

(1) Production of compound represented by formula (IV-1)

The compound represented by formula (IV-1) was produced according to scheme 13 below.

[Chemical Formula 46]

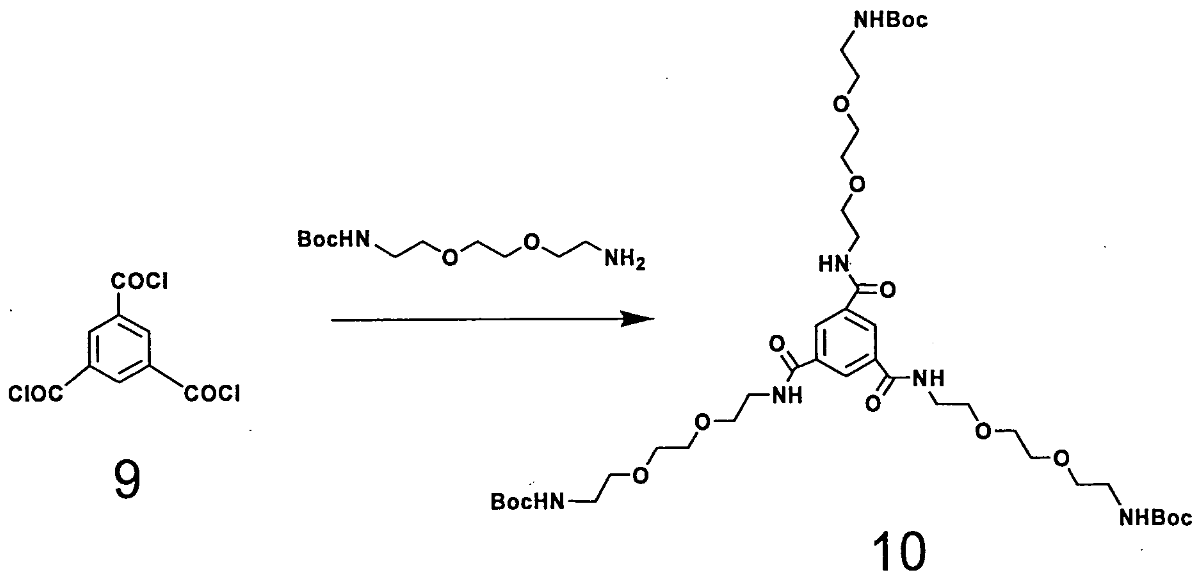

9

10

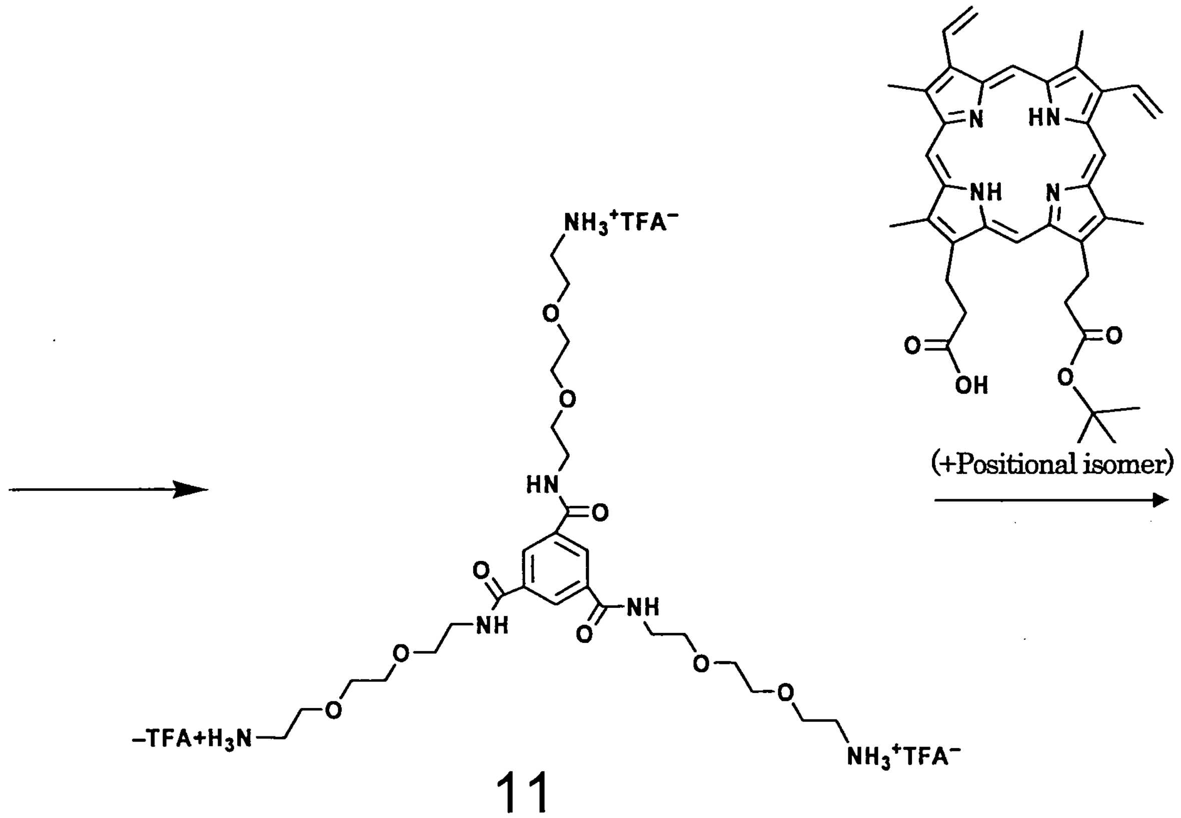


11

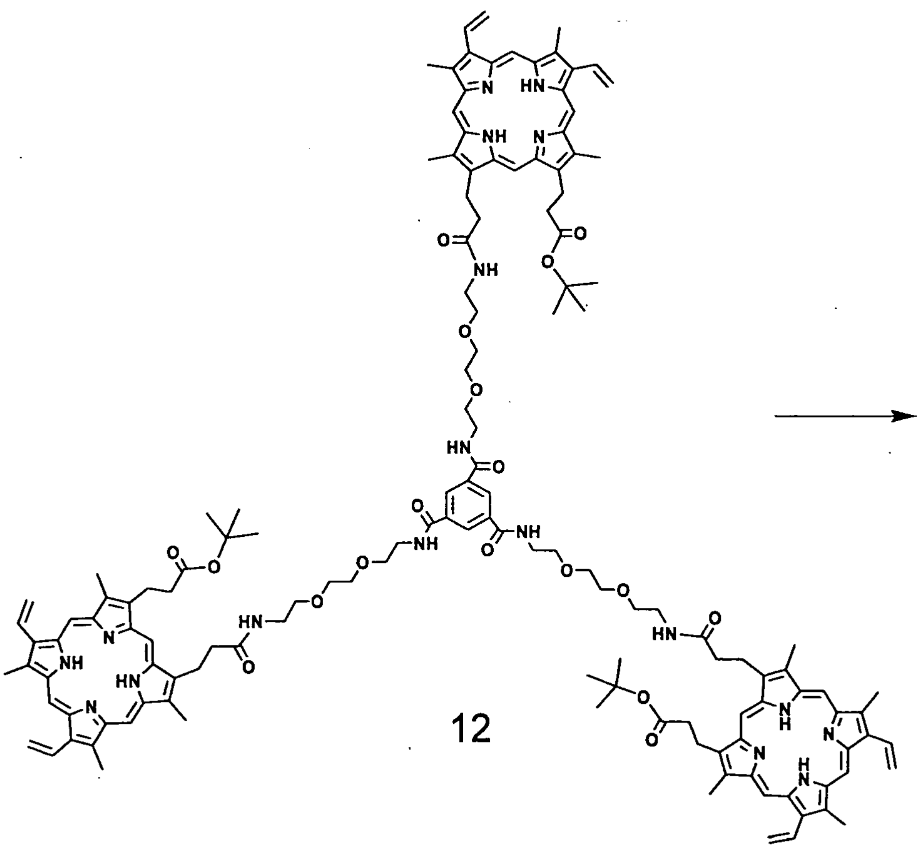

12

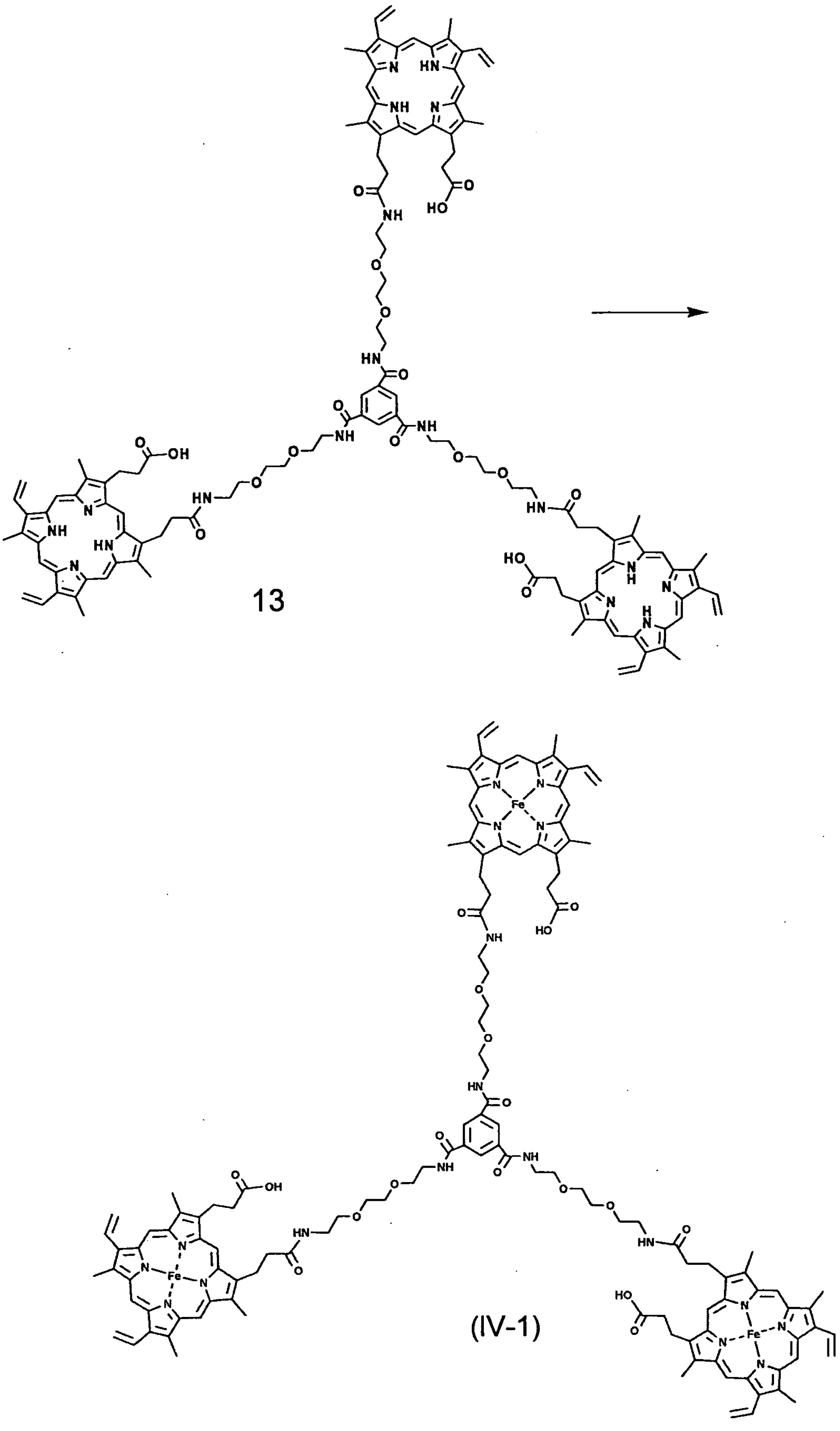

13

(IV-1)

Scheme 13

Note that a mixture of a protoporphyrin IX mono-t-butyl ester and a positional isomer thereof was used for the protoporphyrin IX mono-t-butyl ester shown in scheme 13, and the resulting compound represented by formula 12, the compound represented by formula 13, and the compound represented by formula (IV-1) in which a protoporphyrin IX mono-t-butyl ester as shown in Scheme 13 is bonded are shown in the structural formulas.

(i) Production of compound represented by formula 10

A methylene chloride solution (10 mL) of trimesoyl chloride (0.21 g, $8.1\times10^{-4}$ mol) represented by formula 9 was added to a methylene chloride solution (10 mL) of N-Boc-1,2-bis(aminoethoxy)ethane (0.80 g, $3.2\times10^{-3}$mol) and triethylamine (0.32 g, $3.2\times10^{-3}$ mol) dropwise and the resulting mixture was stirred while cooling in an ice bath. Five hours later, the mixture was washed with water. The organic layer separated from the mixture was dried over anhydrous sodium sulfate and the solvent then was distilled off under reduced pressure. The resulting residue was purified by silica gel column chromatography (eluant: $CHCl_3/CH_3OH$ = 20/1, v/v), giving a compound 10 as colorless oil (0.91 g, 31%).
$^1H$ NMR (400 MHz, $CDCl_3$) $\delta$: 8.44 (s, 3H), 5.30 (bs, 3H), 3.66-3.54 (m, 30H), 3.30-3.27 (m, 6H), 1.38 (s, 27H).
ESI-TOF-MS (positive mode) m/z: found 901.42 $(M+H)^+$, calculated for $C_{42}H_{73}N_6O_{15}$, 901.51.

(ii) Production of compound represented by formula 11

Trifluoroacetic acid (5 mL) was added to a methylene chloride solution (10 mL) of the compound 10 (0.91 g, $10\times10^{-3}$ mol) and the resulting mixture was stirred while cooling in an ice bath. Seven hours later, the solvent was distilled off from the mixture under reduced pressure and the residue was dissolved in a minimum amount of methanol Diethyl ether (100 mL) was added to the methanol solution, the white solids thus generated were recovered and dried, and a compound 11 thus was obtained (0.63 g, 99%).
$^1H$ NMR (400 MHz, $D_2O$) $\delta$: 8.21 (s, 3H), 3.70-3.63 (m, 24H), 3.56 (t, 6H, J = 5.2 Hz), 3.08 (t, 6H, J = 5.2 Hz).
ESI-TOF-MS (positive mode) m/z: found 601.36 $(M-3TFA^--2H^+)^+$, calculate for $C_{40}H_{49}N_6O_5$, 601.36.

(iii) Production of compound 12 (representative structural formula shown in formula 12)

Aprotoporphyrin IX mono-t-butyl ester (100 mg, $1.6\times10^{-4}$ mol) and the compound 11 (35 mg, $4.0\times10^{-5}$ mol) were dissolved in DMF (15 mL) and placed under a nitrogen atmosphere. Tb this solution were added diphenylphosphoryl azide (178 mg, $6.4\times10^{-4}$ mol) and triethylamine (65 mg, $6.4\times10^{-4}$ mol) and the resulting mixture was stirred at room temperature for 4 hours. Thereafter, diphenylphosphoryl azide (178 mg, $6.4\times10^{-4}$ mol) and triethylamine (65 mg, $6.4\times10^{-4}$ mol) were added and the mixture was stirred for 2 more hours. The solvent was distilled off from the mixture under reduced pressure, and the resulting residue was purified by silica gel column chromatography (eluant: $CHCl_3/CH_3OH$ =15/1, v/v). In addition, the resulting product was purified by size exclusion chromatography. The resulting product was dissolved in a minimum amount of chloroform, the purple precipitate formed by adding hexane to the chloroform solution was recovered, and a compound 12 thus was obtained (62 mg, 66%).
$^1H$ NMR (400 MHz, pyridine-$d_5$) $\delta$: 10.25-9.72 (m, 12H), 9.13 (bs, 3H), 8.90 (s, 3H), 8.52 (bs, 3H), 8.38-8.11 (m, 6H), 6.34-6.05 (m, 12H), 4.51-4.39 (m, 12H), 3.52-3.29 (m, 60H), 3.05-2.95 (m, 12H), 2.51-2.49 (m, 6H), 2.45-2.32 (m, 6H), 1.39 (s, 27H)-4.00 (bs, 6H) (due to the presence of a plurality of positional isomers, peaks were split into complex patterns)
ESI-TOF-MS (positive mode) m/z: found 1213.18 $(M+H+Na)^{2+}$, calculated for $C_{141}H_{169}N_{18}NaO_{18}$, 2426.95.
UV-vis (DMF) $\lambda$max/nm (absorbance): 630 (0.024), 576 (0.032), 540 (0.055), 506 (0.070), 405 (0.873).

(iv) Production of compound 13 (representative structural formula shown in formula 13)

Trifluoroacetic acid (4 mL) was added to the compound 12 (62 mg, $2.6\times10^{-5}$ mol) dissolved in formic acid (1 mL) and the resulting mixture was stirred at room temperature for 6 hours. Thereafter, the solvent was distilled off from the mixture under reduced pressure and the resulting residue was dissolved in a minimum amount of methanol. The purple solids generated by adding diethyl ether to the methanol solution were recovered, and thus a compound 13 was obtained (52 mg, 89%).
$^1H$ NMR (400 MHz, pyridine-$d_5$) $\delta$: 10.40-9.72 (m, 12H), 9.11 (bs, 3H), 8.89 (s, 3H), 8.58 (bs, 3H), 8.38-8.11 (m, 6H), 6.34-6.06 (m, 12H), 4.52-4.39 (m, 12H), 3.52-3.20 (m, 60H), 3.07-3.01 (m, 12H), 2.57-2.56 (m, 6H), 2.55-2.40 (m, 6H) -4.00 (bs, 6H) (Due to the presence of a plurality of positional isomers, peaks were split into complicated patterns).
ESI-TOF-MS (positive mode) m/z: found 1118.33 $(M+2H)^{2+}$, calculated for $C_{129}H_{146}N_{18}O_{18}$, 2236.65.
UV-vis (DMF) $\lambda$max/nm (absorbance): 631 (0.034), 577 (0.047), 541 (0.071), 507 (0.091), 404(0.944).

(v) Production of compound (IV-1) (representative structural formula shown in formula 12)

The compound 13, iron chloride n-hydrate (200 mg), and sodium hydrogencarbonate (20 mg) were dissolved in a mixed solution of chloroform and methanol (chloroform:methanol =10:1, 20mL) under a nitrogen atmosphere, and the resulting mixture was heated to reflux. Four hours later, the solvent was distilled off from the mixture under reduced pressure, and the residue was dissolved in a mixed solution of chloroform and methanol (chloroform:methanol = 2:1) and the solution was washed with a 0.05M aqueous hydrochloric acid solution. After the resulting organic layer was dried over anhydrous sodium sulfate, the solvent was distilled off under reduced pressure. The resulting residue was dissolved in a minimum amount of methanol, and the purple solids generated by adding diethyl ether to the methanol solution was recovered. The resulting solids were washed with water and sufficiently dried, and thus a compound (IV-1) was obtained (47 mg, 82%).

ESI-TOF-MS (positive mode) m/z: found 1208.42 $(M\text{-}3Cl\text{-}2H+Na)^{2+}$, calculated for $C_{129}H_{136}Fe_3N_{18}NaO_{18}$, 2417.10.
FAB-MS (positive mode, m-nitrobenzyl alcohol matrix) m/z: found, 2394.19 $(M\text{-}3Cl\text{-}2H)^+$, calculated for $C_{129}H_{136}Fe_3N_{18}O_{18}$, 2394.11.
UV-vis (DMF) λmax/nm (absorbance): 595 (0.093), 571 (0.11), 397 (0.99).

(2) Production of assembly from triad and protein monomer

An assembly was produced by mixing the protein monomer (II-2) and the compound (IV-1) (triad) in a molar ratio of 40:1. Specifically, an assembly was prepared by adding a solution ($10\times10^{-4}$ mol/l, 1 μL, $1\times10^{-4}$μmol) of the compound (IV-1) (triad) dissolved in a mixed solution (DMSO:water =1:1) to a solution ($4.0\times10^{-4}$ mol/l, 10 μL, $4.0\times10^{-3}$ μmol) of the protein monomer (II-2) dissolved in a 0.05M Tris-HCl buffer (pH 7.3) and leaving the mixture to stand still at 4°C overnight.

Example 7

An assembly was produced by mixing the protein monomer (II-2) and the compound (IV-1) (triad) in a molar ratio of 1:1. Specifically, an assembly was prepared by adding a solution ($4.0\times10^{-3}$ mol/l, 1 μL, $4\times10^{-3}$μmol) of the compound (IV-1) (triad) dissolved in a mixed solution (DMSO:water =1:1) to a solution ($4.0\times10^{-4}$ mol/l, 10 μL, $4.0\times10^{-3}$ μmol) of the protein monomer (II-2) dissolved in a 0.05 M Tris-HCl buffer (pH 7.3) and leaving the mixture to stand still at 4°C overnight.

Example 8

An assembly was produced by mixing the protein monomer (II-2) and the compound (IV-1) (triad) in a molar ratio of 10:1. Specifically, an assembly was prepared by adding a solution ($4.0\times10^{-4}$ mol/l, 1 μL, $4\times10^{-4}$μmol) of the compound (IV-1) (triad) dissolved in a mixed solution (DMSO:water =1:1) to a solution ($4.0\times10^{-4}$ mol/l, 10 μL, $4.0\times10^{-3}$μmol) of the protein monomer (II-2) dissolved in a 0.05 M Tris-HCl buffer (pH 7.3) and leaving the mixture to stand still at 4°C overnight.

Example 9

An assembly was produced by mixing the protein monomer (II-2) and the compound (IV-1) (triad) in a molar ratio of 100:1. Specifically, an assembly was prepared by adding a solution ($4.0\times10^{-5}$ mol/l, 1 μL, $4.0\times10^{-5}$ μmol) of the compound (IV-1) (triad) dissolved in a mixed solution (DMSO:water =1:1) to a solution ($4.0\times10^{-4}$ mol/l, 10 μL, $4.0\times10^{-3}$ μmol) of the protein monomer (II-2) dissolved in a 0.05 M Tris-HCl buffer (pH 7.3) and leaving the mixture to stand still at 4°C overnight.

[Atomic force microscope (AFM) measurement]

Samples used in the AFM measurement were prepared as follows. The surface of a high-orientation pyrolytic graphite (HOPG) substrate was cleaved, and the cleaved surface was immersed for several seconds in an aqueous solution of the assembly obtained in Example 6 (about $10^{-8}$ M, in a 0.05 M Tris buffer, pH 7.3). The substrate was removed from the solution, sufficiently washed with water, sufficiently dried at room temperature in a calcium chloride-containing desiccator, and placed in an AFM measuring device. Measurements were carried out in a tapping mode at a scan rate of 2 Hz, and a single-crystal silicon probe having a radius of curvature of about 10 nm was used. Fig. 6 shows AFM images of the assembly obtained in Example 6.

Fig. 6(a) shows an AFM image of the assembly obtained by mixing the protein monomer (II-2) and the compound (IV-1) (triad) in a molar ratio of 40:1. Fig. 6(b) is an enlarged view of Fig. 6(a). Fig. 6(c) shows a cross-sectional profile observed along the gray line in Fig. 6(b). According to the cross-sectional profile, this assembly has a thickness of about 4 nm. It was verified from this height information that the assembly was a nanosheet having a height corresponding to one protein (2.5 to 5.0 nm).

Fig. 7 shows an AFM image of the assembly obtained in Example 7, Fig. 8 shows an AFM image of the assembly obtained in Example 8, and Fig. 9 shows AFM images of the assembly obtained in Example 9. Fig. 10 shows anAFM image of the compound (IV-1) (triad).

It was verified from Fig. 10 that when only the compound (IV-1) is used, only dots are observed in the image. Presumably, those dots are an agglomerate of the compound (IV-1). It was verified from Fig. 7 that the use of the assembly obtained in Example 7 results in rods. Since the height of the rods is less than 1 nm, the rods are presumably an agglomerate of a modified protein. It was verified from Fig. 8 that the use of the assembly obtained in Example 8 results in the formation of a locally denser network than the assembly obtained in Example 6. It was verified from Fig. 9 that the use of the assembly obtained in Example 9 results in the formation of a generally sparser network than the assembly obtained in Example 6. Fig. 9(b) shows a cross-sectional profile observed along the gray line in Fig. 9(a). According to the cross-sectional profile, this assembly has a thickness of about 4 nm. It was verified from this height information that the assembly was a nanosheet having a height corresponding to one protein (2.5 to 5.0 nm). In Fig. 9 (c), a part of Fig. 9(a) is enlarged and depicted three-dimensionally. Fig. 9(c) shows that the protein chains of the assembly are branched.

Example 10

(1) Production of compound represented by formula 11(2)

A compound represented by formula 11(2) was produced according to scheme 14 below. Specifically, a compound represented by formula 4(2) was produced in the same manner as in the production of the compound represented by formula 4(2) of Example 2. In addition, a compound represented by formula 15(2) was produced in the same manner as in the production of the compound represented by formula 1(2) of Example 2.

[Chemical Formula 47]

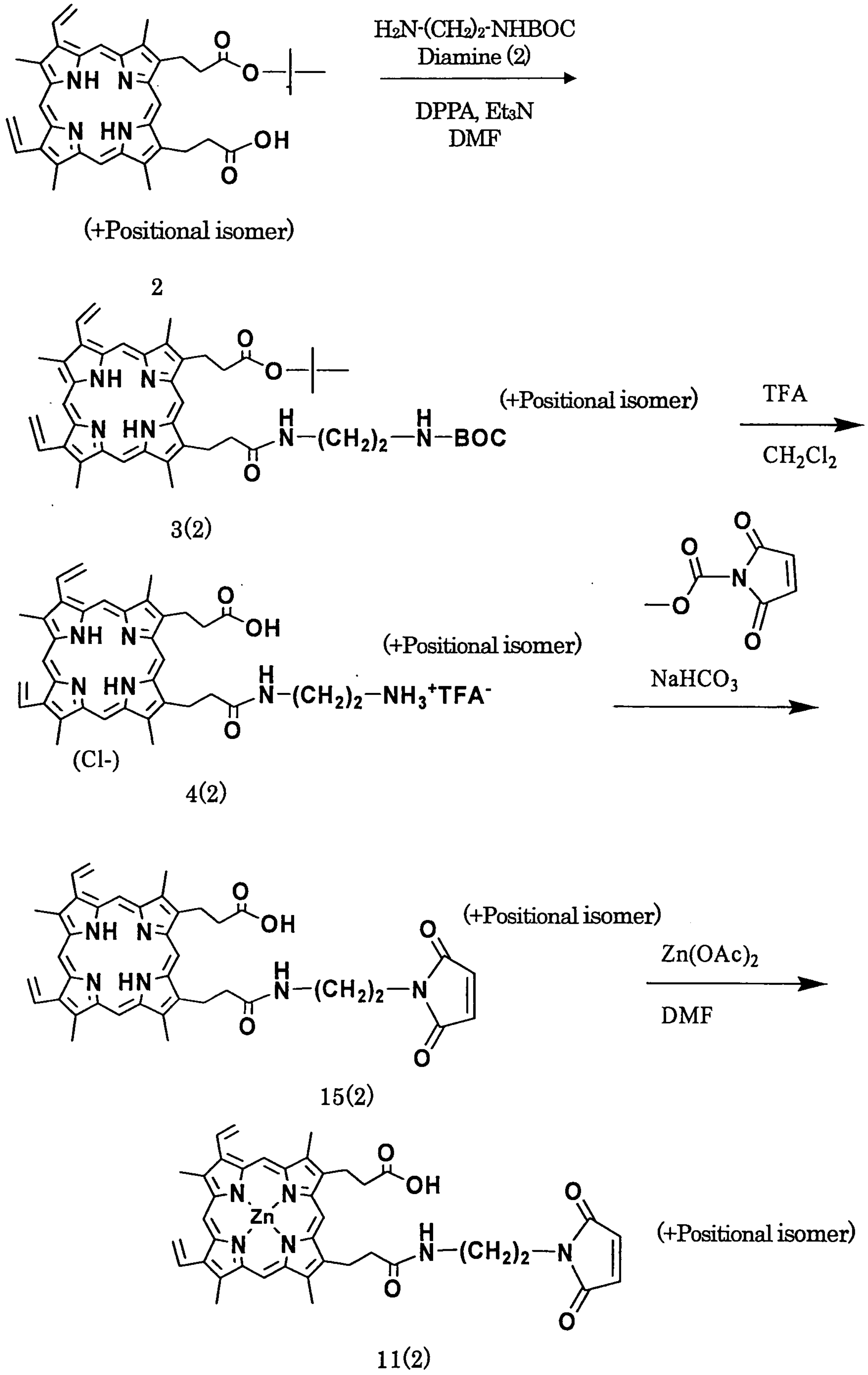


2

3(2)

4(2)

15(2)

11(2)

Scheme 14

Compound 3(2): yield 40%.
$^1$H-NMR (400 MHz, DMSO-$d_6$) δ: 10.44-10.10 (4H, m), 8.45 (2H, d), 7.91 (1H, s), 6.47 (2H, d), 6.20 (2H, d), 4.51-4.38 (4H, m), 3.66 (6H, s), 3.57 (6H, s), 3.42-3.10 (2H, m), 2.90-2.81 (2H, m), 1.28 (9H, s), 1.24 (9H, s), -3.27 (2H, s).
ESI-TOF-MS (positive mode) m/z: found 761.38 $[M+H]^+$, calculated for $C_{45}H_{56}N_6O_5$, 761.44.

Compound 4(2): yield 95%.
$^1$H-NMR (400 MHz, DMSO-$d_6$) δ:10.44-10.23 (4H, m), 8.57 (2H, d), 8.06 (1H, s), 7.87-7.58 (3H, br), 6.46 (2H, d), 6.24 (2H, d), 4.33 (4H, s), 3.76 (6H, s), 3.65 (6H, s), 3.26-3.07 (2H, m), 2.80-2.61 (2H, m), -3.87 (2H, s).
ESI-TOF-MS (positive mode) m/z: found 605.32 $[M+H]^+$, calculated for $C_{36}H_{41}N6O3$, 605.75.

Compound 15(2): Under a nitrogen atmosphere, the compound 4(2) (60.0 mg, 0.0990 mmol) was dissolved in a mixed solvent (saturated sodium hydrogencarbonate:acetone =1:2 (volume ratio), 5 mL) in a 2-neck flask (30 mL). A solution (2 mL) of N-(methoxycarbonyl)-maleimide (produced in reference to HELVETICA CHIMICA ACTA, voL 58, pp. 531-541 (1975)) (27.0 mg, 0.170 mmol) dissolved in a mixed solvent as mentioned above was added thereto and then stirred at 0°C for 2 hours. Thereafter, the mixture was diluted with water (30 mL) and stirred for 1 more hour. The pH of the mixture was adjusted with 1 M hydrochloric acid to be 4 and then the mixture was extracted with chloroform. The obtained organic phase was washed successively with an aqueous citric acid solution having a pH of 4 and saturated brine and then dried over sodium sulfate. The solvent was distilled off from the organic phase under reduced pressure, and the resulting residue was purified with a silica gel column (chloroform/methanol = 5/1, v/v), thereby giving the title compound (20 mg, 31%).

$^1$H-NMR (400 MHz, DMSO-$d_6$) δ: 10.32-10.12 (4H, m), 8.46 (2H, d), 8.14-8.09 (1H, br), 6.87 (2H, d), 6.40 (2H, d), 6.21 (2H, d), 4.37-4.29 (2H, m), 4.29-4.22 (2H, m), 3.77 (6H, s), 3.59 (6H, s), 3.28-3.16(2H, m), 2.99-2.88 (2H, m), -4.05 (2H, s).
ESI-TOF-MS (positive mode) m/z: found 685.24 $[M+H]^+$, calculated for $C_{40}H_{40}N_6O_5$, 685.31.

Formula 11(2):

A DMF (6 mL) solution of the compound 15(2) (21.0 mg, 0.0307 mmol) was introduced into a recovery flask (30 mL) and the mixture was heated to 40°C. Then, zinc acetate (24.2 mg, 0.132 mmol) was added and the mixture was stirred for 5 hours. After distilling off the solvent from the mixture under reduced pressure, the resulting residue was dissolved in chloroform, and the chloroform solution was washed with saturated brine and dried over sodium sulfate. Thereafter, the solvent was distilled off from the chloroform solution under reduced pressure, and the resulting residue was dried in a vacuum, thus giving the title compound (20.0 mg, 90%).

$^1$H-NMR (400 MHz, DMSO-$d_6$) δ: 10.30-10.09 (4H, m), 8.76-8.54 (2H, m), 7.43-7.41 (1H, br), 6.98 (2H, d), 4.40-4.33 (2H, m), 4.32-4.25 (2H, m), 3.76-3.68 (6H, m), 3.62-3.53 (6H, m), 3.30-3.18 (2H, m), 3.09-2.98 (2H, m).
ESI-TOF-MS (positive mode) m/z: found 747.12 $[M+H]^+$, calculated for $C_{40}H_{38}N_6O_5Zn$, 747.22.
UV-Vis (DMF) λmax/nm (absorption): 419 (0.787), 547 (0.0569), 584 (0.0581).

(2) Production of protein monomer and protein polymer containing the monomer as a monomer unit (see scheme 15)

[Chemical Formula 48]

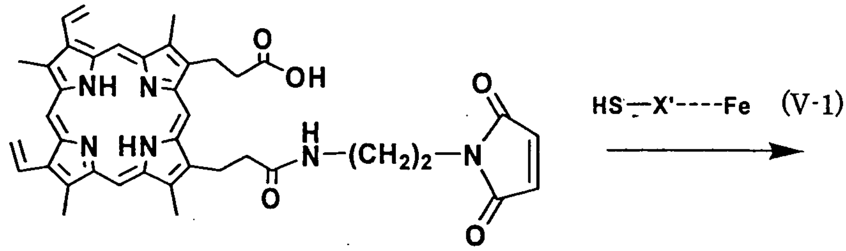


(+Positional isomer)
15(2)

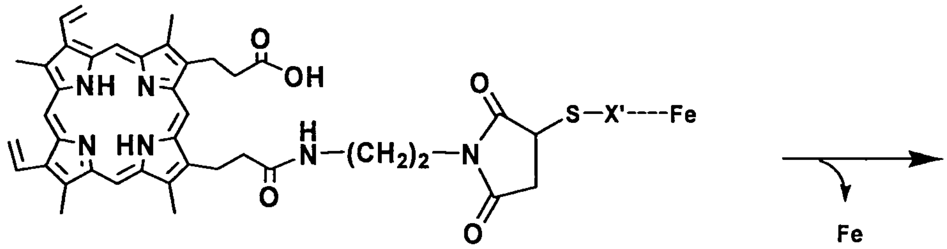


(+Positional isomer)
16(2)

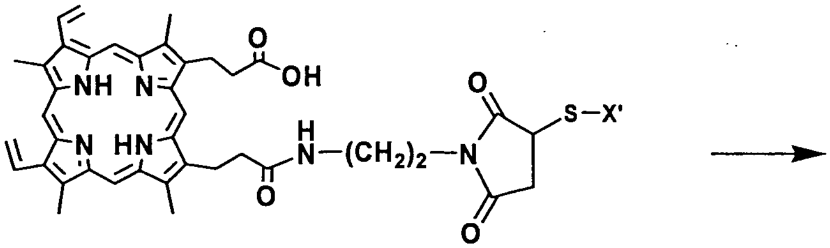

(+Positional isomer)
17(2)

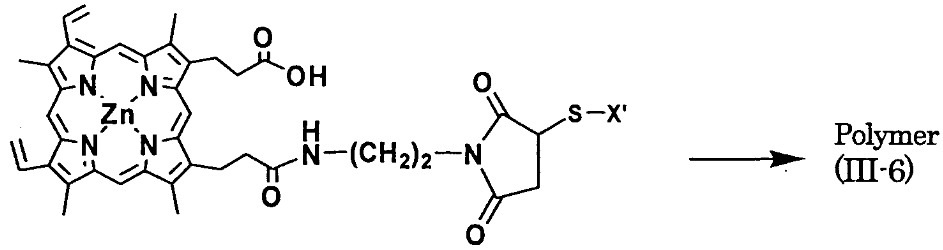


(+Positional isomer)

(II-6)

Scheme 15

Formula (V-1) above:

[Chemical Formula 49] **HS-X'----Fe**

is as described above.

A stock solution of the H63C expressed in Example 1(2) (200 μL, concentration of $3.11\times10^{-3}$ M in a 10 mM Tris-HCl buffer, pH 7.3) and a 10 mM dithiothreitol solution (10 mM Tris-HCl buffer (pH 7.3), 2.8 mL) were mixed to reduce the oxidized form of cytochrome $b_{562}$ (H63C) contained. This reaction solution was subjected to ultrafiltration several times, and a large excess of dithiothreitol was removed. Thereafter, desalting was performed with a desalting column (HiTrap Desalting, 5 mL, manufactured by GE Healthcare), thereby giving a reduced cytochrome $b_{562}$ (H63C) solution. The reduced cytochrome $b_{562}$ (H63C) solution (1.6 mL) was diluted by adding a 10 mM Tris-HCl (pH 7.3,1.8 mL) buffer, a DMSO solution ($4.71\times10^{-6}$ mol, 0.6 mL) of the compound 15(2) was then added gradually, and the resulting mixture was stirred under shading at room temperature for 4 hours. Excessive compound 15(2) present in the reaction solution was removed by extraction with 2-butanone, thereby giving a compound 16(2) in which the compound 15(2) and the H63C (V-1) were bonded.

UV (10 mM pH 7.3, Tris-HCl buffer) λmax/nm (absorption): 373 (0.725), 417 (0.803), 523 (0.150), 567 (0.112), 631 (0.0470), 670 (0.0424).

After adding histidine (13 mg) to the solution ($4.45\times10^{-6}$ mol, 4.1 mL) of the compound 16(2), 0.1M hydrochloric acid was added to adjust the pH of the solution to be 2. The liberated heme was extracted with butanone and the extract was subjected to 90-minute dialysis with a Tris-HCl buffer (10mM, pH 7.3) three times, thereby giving a compound 17 (2) (Apo form).

Zinc acetate (38.1 mg, $1.04\times10^{-5}$ mol, 100 eq.) was added to a solution ($1.04\times10^{-3}$ M, 2 mL, 10 mM Tris-HCl buffer, pH 7.3) of the compound 17(2) (Apo form) and the resulting mixture was stirred at 40°C for 4 hours. After cooling to room temperature, the reaction solution was subjected to 90-minute dialysis with a 10mM Tris-HCl buffer (pH 7.3) three times, thereby removing excessive zinc acetate and giving a crude product. The product was subjected to a desalting treatment (HiTrap Desalting, 5 mL, GE Healthcare), thereby removing the remaining salts such as zinc salt. Next, the product was purified using an anion-exchange column (HiTrap Q FF, 5 mL, GE Healthcare), thereby giving a protein monomer (II-6) ($1.78\times10^{-7}$ mol, $8.91\times10^{-5}$ M, yield of 8.5%). UV-Vis spectrum measurements on the product revealed an absorption at 417, 523, and 567 nm, which is distinctive to zinc protoporphyrin, showing that zinc protoporphyrin was bonded therein.

UV-Vis (10 mM, pH 7.3, Tris-HCl buffer) λmax/nm (absorption): 417 (0.0637), 523 (0.00880), 567 (0.00850).

After concentrating the solution of the protein monomer (II-6), the precipitates formed in the solution were removed by centrifugation (at 8000 rpm for 10 minutes at 4°C) and filtration, thereby giving a protein polymer (III-6) as a supernatant.

It can be presumed that the protein polymer (III-6) has a random copolymer structure as shown in formula (III-16).

[Chemical Formula 50]

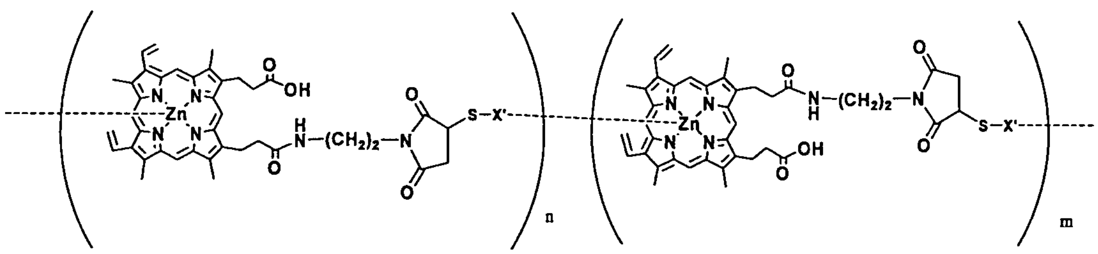

(III-16)

In the formula, X' represents X' that is present in a hemoprotein mutant, and the hemoprotein mutant is represented by the formula (V-1).

Example 11

(1) Production of hemin-modified gold electrode (A)

A gold electrode (a glass electrode on which gold was vapor-deposited) was produced as follows. A glass electrode (Matsunami Glass Ind. Ltd. s3399X5 BK-71 edge polished 13×36 t0.7 both sides polished) was immersed in

a 5 M aqueous potassium hydroxide solution for 12 hours and then subjected to ultrasonic cleaning for 20 minutes. This glass electrode was washed with ultrapure water and then ethanol, and dried. A gold wire for use in vapor deposition was immersed in a mixed solution of concentrated sulfuric acid and hydrogen peroxide (concentrated sulfuric acid/hydrogen peroxide = 3/1) for 3 minutes, washed with ultrapure water and then ethanol, and dried. The washed gold wire, chromium powder, and the glass electrode were placed in a deposition apparatus, a voltage was applied to the chromium powder at 260°C in a vacuum to vapor-deposit chromium on the surface of the glass electrode, and then a voltage was applied to the gold wire to vapor-deposit thin gold film over the chromium film. The deposition apparatus was returned to atmospheric-pressure and room-temperature conditions, and the gold electrode (a glass electrode on which gold was vapor-deposited) was removed.

Next, according to the method of A. Das et., al. (A. Das et al., Biophysical Chemistry, 2006, 123, 102-112.), the gold electrode was modified with heme (see scheme 16).

[Chemical Formula 51]

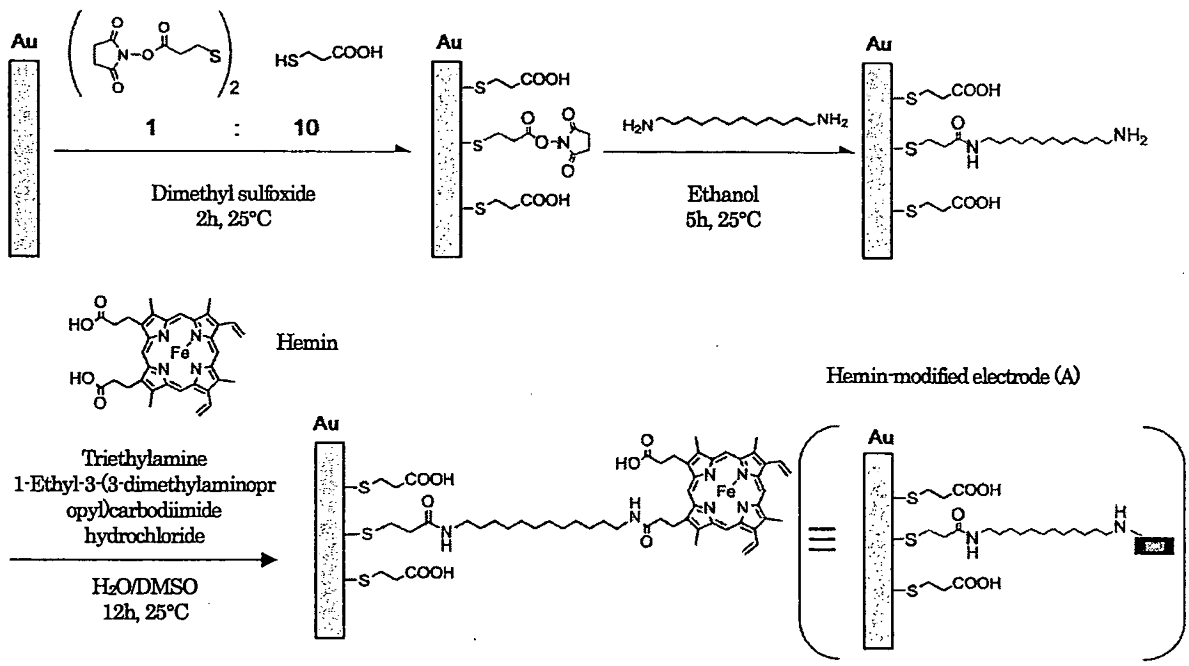


Scheme 16

Specifically, first, the gold electrode was washed while applying an electric potential of -1.2V Next, the gold electrode was immersed at room temperature for 1.5 hours in a dimethylsulfoxide solution (500 μL) in which the ratio of concentration of 3-mercaptopropionic acid/3,3'-dithiobis(succinimidyl propionate) was adjusted to be 100 mM/1.00 mM. Thereafter, the surface of the gold electrode was washed with dimethyl sulfoxide and then ultrapure water. Next, the gold electrode was immersed in a 1,12-diaminododecane solution (20 mM, the solvent was a mixed solution of ethanol (95%) and water (5%), 500 μL), at room temperature for 4 hours. Thereafter, the surface of the gold electrode was washed with dimethyl sulfoxide and then ultrapure water. The gold electrode was immersed in a mixed solution of 1-ethyl-3-(3-dimethylaminopropyl)carbodiimide hydrochloride (300 mM aqueous solution, 25 μL) and an aqueous triethylamine solution (300 mM, 25 μL). Thereafter, hemin dissolved in dimethyl sulfoxide (10 mM solution, 50 μL) was added, and the gold electrode was kept immersed at 4°C overnight to induce modification by a coupling reaction on the surface of the gold electrode. Next, the gold electrode was washed with ultrapure water, thereby giving a hemin-modified gold electrode.

(2) Production of protein monomer-modified gold electrode (B)

The hemin-modified gold electrode (A) obtained in (1) was immersed in the apo form of pocytochrome $b_{562}$ (wild type, 50 pL, 10 mM, pH 7.0 phosphate buffer) at 4°C for 18 hours. The electrode was washed with a MOPS buffer (100 mM, pH 7.0), thereby giving a protein monomer-modified gold electrode (B) (see scheme 17).

[Chemical Formula 52]

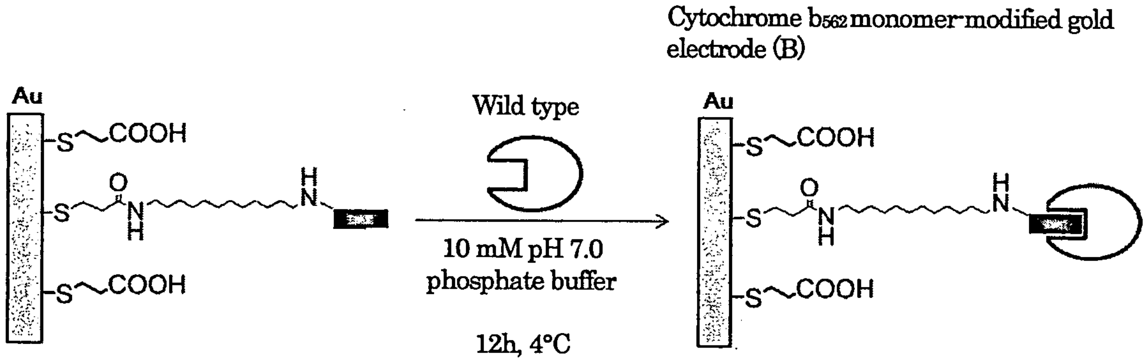


Scheme 17

(3) Production of protein polymer-modified gold electrode (C)

The hemin-modified gold electrode (A) obtained in (1) was immersed in the apo form of the protein polymer (II-2) (produced in Example 2, 50 μL, 10 mM, pH 7.0 phosphate buffer) at 4°C for 18 hours. The electrode was washed with a MOPS buffer (100 mM, pH 7.0), thereby giving a protein polymer-modified gold electrode (C) (see scheme 18).

[Chemical Formula 53]

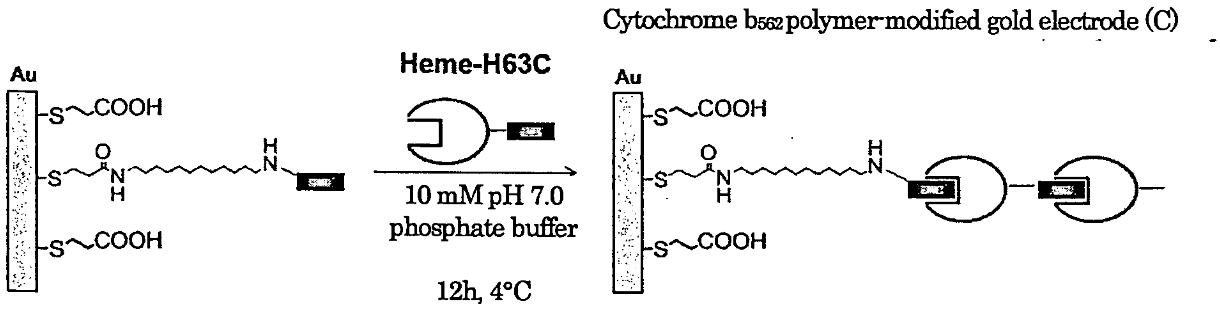


Scheme 18

(4) Production of hemin-modified gold electrode (D)

A hemin-modified electrode (D) was obtained in the same manner as in Example 11(1) except that 1,6-diaminohexane was used in place of 1,12-diaminododecane.

(5) Production of protein polymer-modified gold electrode (E)

A protein polymer-modified electrode (E) was obtained in the same manner as in Example 11(3) except that the hemin-modified gold electrode (D) obtained in (4) was used in place of the hemin-modified gold electrode (A) obtained in (1).

(i) Electrochemical measurement

Electrochemical measurements were carried out with anALS/CH Instruments Electrochemical Analyzer (Model 610B).

The hemin-modified gold electrode (A), the protein monomer-modified gold electrode (B), and the protein polymer-modified gold electrode (C) were attached to cells and a MOPS buffer (100mM, pH 7.0) was added to the cells until the electrodes were immersed in the MOPS buffer. The buffer introduced into the cells was deaerated by bubbling argon for 30 minutes, and the hemin-modified gold electrode (A), the protein monomer-modified gold electrode (B), the protein polymer-modified gold electrode (C), the hemin-modified gold electrode (D), and the protein polymer-modified gold electrode (E) were subjected to cyclic voltammetry and an impedance measurement. Sweep rate: 0.30 [V/s], counter electrode: platinum, reference electrode: silver/silver chloride.

(a) Cyclic voltammetry

The results of cyclic voltammetry (sweep rate: 0.30 (V/s), counter electrode: platinum, reference electrode: silver/silver chloride) showed that the E1/2 values of the hemin-modified gold electrode (A), the protein monomer-modified gold electrode (B), and the protein polymer-modified gold electrode (C) were each about -0.30 [V] (vs silver/silver chloride), indicating a heme-derived redox response. Figs. 11(a), 11(b), and 11(c) shows cyclic voltammograms of the hemin-modified gold electrode (A), the protein monomer-modified gold electrode (B), and the protein polymer-modified gold electrode (C), respectively. The redox response on the protein polymer-modified gold electrode (C) showed a decreased amount of faradic current and an increased value of nonfaradic current, and it was verified therefrom that the substrate was modified with a hemoprotein.

(b) Correlation between sweep rate and peak current value

In regard to the hemin-modified gold electrode (A), the protein monomer-modified gold electrode (B), the protein polymer-modified gold electrode (C), the hemin-modified gold electrode (D), and the protein polymer-modified gold electrode (E), plotting the reduction peak current value against the sweep rate revealed a linear relationship in all cases. Figs. 12(a), 12(b), 12(c), 12(d), and 12(e) show cyclic voltammograms of the hemin-modified gold electrode (A), the protein monomer-modified gold electrode (B), the protein polymer-modified gold electrode (C), the hemin-modified gold electrode (D), and the protein polymer-modified gold electrode (E) obtained with various sweep rates, respectively. Moreover, Figs. 13(a), 13(b), 13(c), 13(d), and 13(e) are graphs showing the peak currents obtained with the hemin-modified gold electrode (A), the protein monomer-modified gold electrode (B), the protein polymer-modified gold electrode (C), the hemin-modified gold electrode (D), and the protein polymer-modified gold electrode (E), respectively. According to Figs. 12 and 13, there is a proportional relationship between the sweep rate and the current value, indicating that there is an electrochemical response of the surface-adsorbed species. It therefore was verified that the hemin-modified gold electrode (A), the protein monomer-modified gold electrode (B), the protein polymer-modified gold electrode (C), the hemin-modified gold electrode (D), and the protein polymer-modified gold electrode (E) are modified by a heme molecule, cytochrome $b_{562}$, a protein polymer, a heme molecule, and a protein polymer, respectively.

(c) Calculation of degree of surface modification

In the case of an adsorption system, the quantity of electricity can be calculated from the current peak area indicated on a cyclic voltammogram, therefore: quantity of electricity Q = nFx (where n is the number of electrons, F is the Faraday constant, and x is the molar number of a material modified on the electrode surface). Using this mathematical formula, the molecular weight of and the extent of surface coverage by the surface-adsorbed species were calculated. The quantity of electricity consumed during the reaction on the hemin-modified gold electrode (A) was $1.66\times10^{-6}$ [mol $\cdot$ $m^{-2}$], and the extent of surface coverage was calculated to be 3% assuming that the size of one hemin molecule as a flat surface is $10\times10$ [Å]. This correlated well to the immersion of the electrode in 3-mercaptopropionic acid/3,3'-dithiobis (succinimidyl propyonate) adjusted to have a concentration ratio of 100 mM/1 mM during the initial stage of electrode production, indicating successful, nearly quantitative surface modification on the hemin modification gold electrode (A).

(d) Differential pulse voltammetry (DPV)

DPV is a method that applies a fixed pulse voltage at a regular interval to a direct voltage that is increased at a constant rate and is a measurement method that yields a high resolution peak. DPV was performed on the hemin-modified gold electrode (A), the protein monomer-modified gold electrode (B), the protein polymer-modified gold electrode (C), the hemin-modified gold electrode (D), and the protein polymer-modified gold electrode (E). Figs. 14(a), 14(b), 14 (c), 14(d), and 14(e) show the results obtained from the hemin-modified gold electrode (A), the protein monomer modified gold electrode (B), the protein polymer-modified gold electrode (C), the hemin-modified gold electrode (D), and the protein polymer-modified gold electrode (E), respectively. It is clear from Fig. 14 that the hemin-modified gold electrode (A), the protein monomer-modified gold electrode (B), and the protein polymer-modified gold electrode (C) each have

an oxidation peak potential and a reduction peak potential both near E = -0.31 [V], and the hemin-modified gold electrode (D) and the protein polymer-modified gold electrode (E) have -0.30 and -0.34 [V], respectively, indicating that the oxidation and reduction potentials are nearly identical. It was verified therefrom that the gold electrodes were modified with a heme molecule, cytochrome $b_{562}$, and a protein polymer, respectively

(e) Impedance measurement

Alternating current impedance measurement that takes advantage of the fact that the voltage across a resistance part and the voltage across a capacitor part are out of phase when an AC voltage is applied allows the resistance of an electrode to be measured, if the system is viewed as an electric circuit. If the horizontal axis is for impedance expressed as a real number and the vertical axis is for impedance expressed as an imaginary number, changing the frequency of an AC voltage reveals a semicircle that is distinctive to a parallel circuit of a resistor and a capacitor. The diameter of the semicircle is called charge transfer resistance, and charge transfer resistance is increased as a substance builds up on the surface and the thickness is increased. The resistances of the hemin-modified gold electrode (A), the protein monomer-modified gold electrode (B), and the protein polymer-modified gold electrode (C) were $4.0\times10^3\ \Omega$, $1.0\times10^4\ \Omega$, and $2.2\times10^4\ \Omega$, respectively (see Fig. 15). The relative magnitude of resistance was electrode (A) < electrode (B) < electrode (C) and the resistance greatly was enhanced on the electrode (C) in particular, thereby establishing that the gold electrode was successfully modified with a protein polymer.

[Conditions of impedance measurement]

Impedance was measured in an aqueous solution of 100 mM potassium chloride and 5 mM $K_3Fe(CN)_6/K_4Fe(CN)_6$.
Sweep rate: 0.30 [V/s]
Counter electrode: platinum
Reference electrode: silver/silver chloride
AC voltage range: -0.2±0.01 [V] (vs silver/silver chloride)
Frequency: 100000 to 0.1 [Hz]

Industrial Applicability

The protein polymer of the present invention is also usable as an enzyme assembly.

Sequence Listing Free Text

SEQ ID NO.1: Cytochrome $b_{562}$
SEQ ID NO. 2: Sperm whale-derived wild-type myoglobin
SEQ ID NO. 3: Horseradish peroxidase
SEQ ID NO. 4: H63C cytochrome $b_{562}$
SEQ ID NO. 5: Mutation site-introduced primer SEQ ID NO. 6: M13 primer M4
SEQ ID NO. 7: M13 primer RV
SEQ ID NO. 8: MUT4 primer
SEQ ID NO. 9: Hemoglobin α-subunit (human)
SEQ ID NO. 10: Hemoglobin β-subunit (human)

**Claims**

1. A protein monomer represented by formula (I) or a salt thereof:

[Chemical Formula 1]

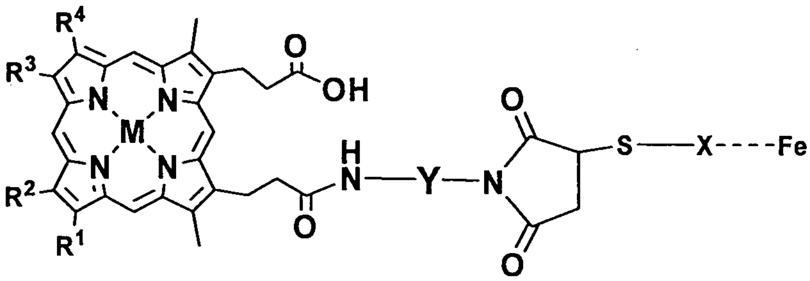

(I)

wherein,
$R^1$, $R^2$, $R^3$, and $R^4$ each independently represent a hydrogen atom, a lower alkyl group, a halogen-substituted lower alkyl group, or a lower alkenyl group;
Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
X represents X that is present in a hemoprotein mutant, and the hemoprotein mutant is represented by formula (V):

[Chemical Formula 2] **HS-X----Fe** (V)

the mutant is a protein that has the same amino acid sequence as a native hemoprotein except that one amino acid residue is replaced with a cysteine residue, and in the mutant -SH represents a side-chain thiol group of the cysteine residue and Fe is bonded to four nitrogen atoms of a porphyrin group contained in the hemoprotein.

2. A protein monomer represented by formula (II) or a salt thereof

[Chemical Formula 3]

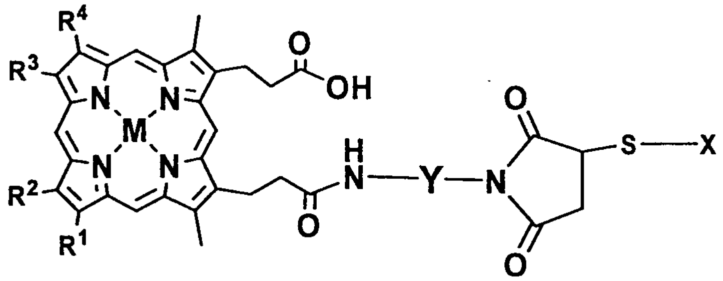

(II)

wherein,
$R^1$, $R^2$, $R^3$, and $R^4$ each independently represent a hydrogen atom, a lower alkyl group, a halogen-substituted lower alkyl group, a halogen-substituted lower alkyl group, or a lower alkenyl group; Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
X represents X that is present in a hemoprotein mutant, and the hemoprotein mutant is represented by formula (V):

[Chemical Formula 4] **HS-X----Fe** (V)

the mutant is a protein that has the same amino acid sequence as a native hemoprotein except that one amino acid residue is replaced with a cysteine residue, and in the mutant -SH represents a side-chain thiol group of the cysteine residue and Fe is bonded to four nitrogen atoms of a porphyrin group contained in the hemoprotein.

**3.** A protein polymer or a salt thereof comprising as a monomer unit the protein monomer represented by formula (II) or the salt thereof according to claim 2.

**4.** The protein polymer or the salt thereof according to claim 4, wherein the protein polymer is a random protein polymer represented by formula (III):

[Chemical Formula 5]

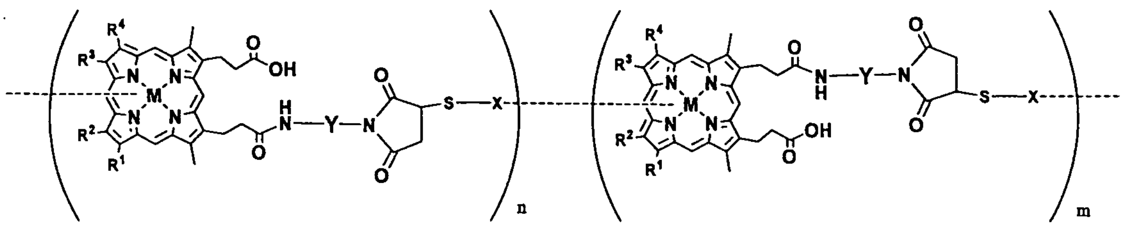

(III)

wherein,
$R^1$, $R^2$, $R^3$, and $R^4$ each independently represent a hydrogen atom, a lower alkyl group, a halogen-substituted lower alkyl group, or a lower alkenyl group;
Y is a group represented by a formula $-(CH_2)_{n1}-$, $-(CH_2)_{n1}-O-(CH_2)_{n2}-$, $-(CH_2)_{n1}-O-(CH_2)_{n2}-O-(CH_2)_{n3}-$, or $-(CH_2)_{n1}-O-(CH_2)_{n2}-O-(CH_2)_{n3}-O-(CH_2)_{n4}-$, wherein n1, n2, n3, and n4 each independently represent an integer of 1 to 20;
M is selected from the group consisting of Fe, Zn, and Co; and
X represents X that is present in a hemoprotein mutant, and the hemoprotein mutant is represented by formula (V):

[Chemical Formula 6] **HS-X----Fe** (V)

the mutant is a protein that has the same amino acid sequence as a native hemoprotein except that one amino acid

residue is replaced with a cysteine residue, and in the mutant -SH represents a side-chain thiol group of the cysteine residue and Fe is bonded to four nitrogen atoms of a porphyrin group contained in the hemoprotein.

**5.** A method for producing the protein polymer or the salt thereof according to claim 3, comprising treating the protein monomer or the salt thereof according to claim 2 under a neutral condition to provide the protein polymer or the salt thereof according to claim 3.

**6.** A protein assembly or a salt thereof comprising a triad represented by formula (IV) or a salt thereof and the protein monomer represented by formula (II) or the salt thereof according to claim 2:

[Chemical Formula 7]

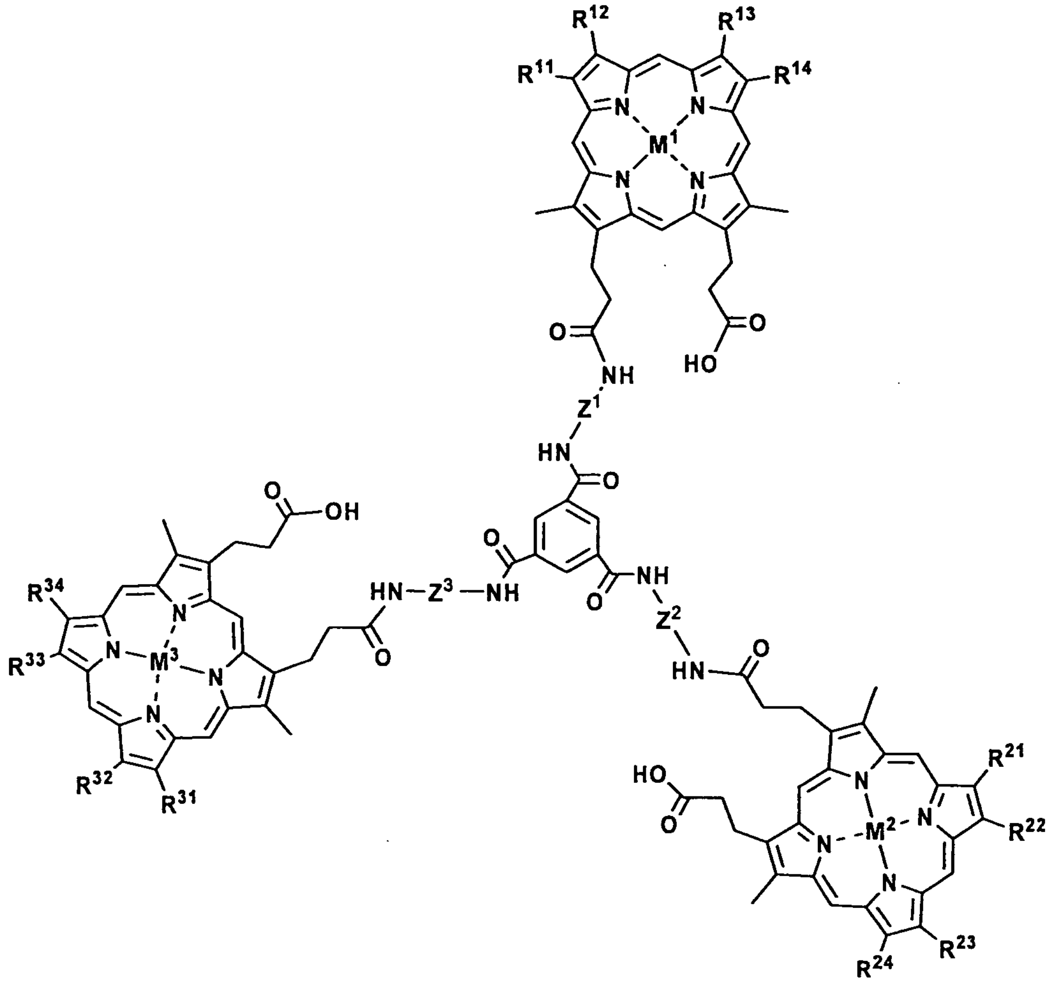

(IV)

wherein,
$R^{11}$, $R^{12}$, $R^{13}$, $R^{14}$, $R^{21}$, $R^{22}$, $R^{23}$, $R^{24}$, $R^{31}$,$R^{32}$, $R^{33}$, and $R^{34}$ each independently represent a hydrogen atom, a lower alkyl group, a halogen-substituted lower alkyl group, or a lower alkenyl group;
$M^1$, $M^2$, and $M^3$ are each independently selected from the group consisting of Fe, Zn, and Co; and
$Z^1$, $Z^2$, and $Z^3$ each independently represent a group represented by a formula $-(CH_2)_{m1}-$, $-(CH_2)_{m1}-O-(CH_2)_{m2}-$, $-(CH_2)_{m1}-O-(CH_2)_{m2}-O-(CH_2)_{m3}-$, or $-(CH_2)_{m1}-O-(CH_2)_{m2}-O-(CH_2)_{m3}-O-(CH_2)_{m4}-$, wherein m1, m2, m3, and m4 each independently represent an integer of 1 to 20.

**7.** A method for producing the protein assembly or the salt thereof according to claim 6, comprising treating a triad represented by formula (IV) or a salt thereof with the protein monomer represented by (II) or the salt thereof according to claim 2 under a neutral condition to provide the protein assembly or the salt thereof according to claim 6:

[Chemical Formula 8]

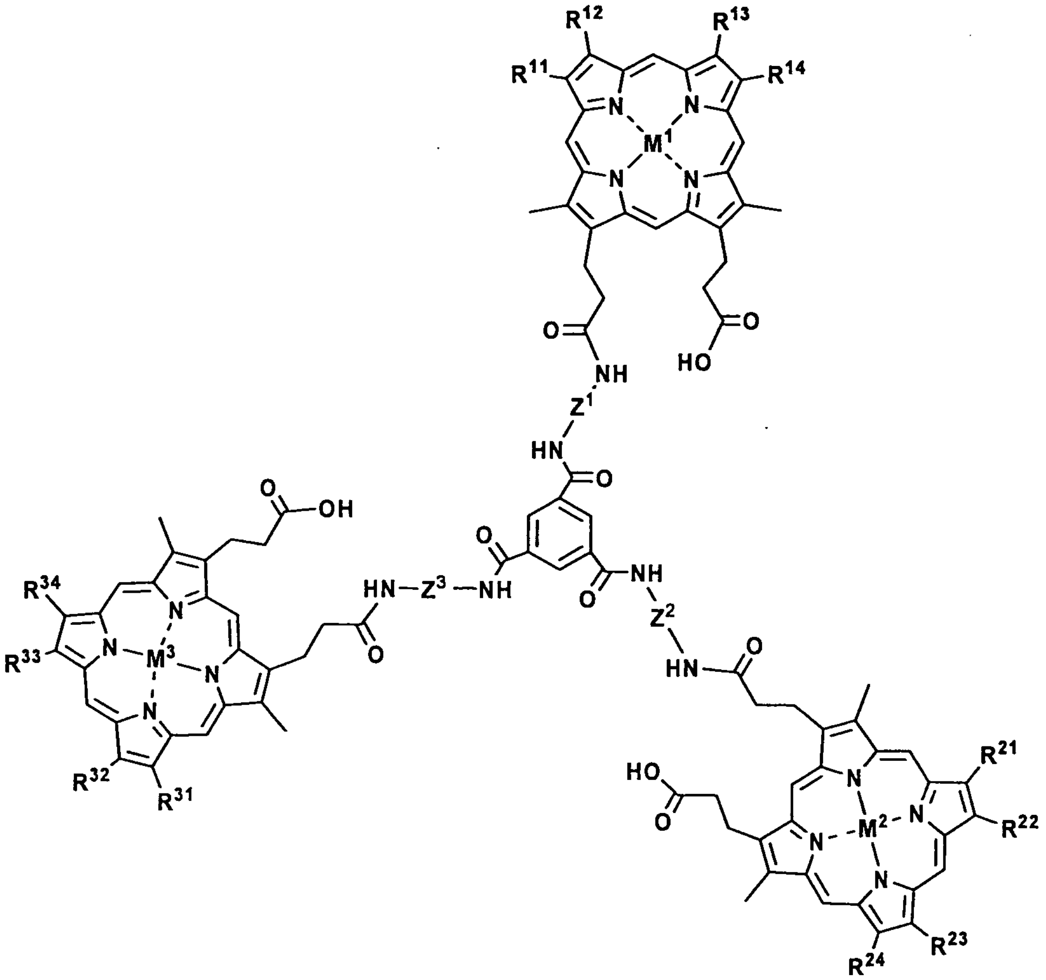

(IV)

wherein,
$R^{11}$, $R^{12}$, $R^{13}$, $R^{14}$, $R^{21}$, $R^{22}$, $R^{23}$, $R^{24}$, $R^{31}$,$R^{32}$, $R^{33}$, and $R^{34}$ each independently represent a hydrogen atom, a lower alkyl group, a halogen-substituted lower alkyl group, or a lower alkenyl group;
$M^1$, $M^2$, and $M^3$ are each independently selected from the group consisting of Fe, Zn, and Co; and
$Z^1$, $Z^2$, and $Z^3$ each independently represent a group represented by a formula $-(CH_2)_{m1}-$, $-(CH_2)_{m1}-O-(CH_2)_{m2}-$, $-(CH_2)_{m1}-O-(CH_2)_{m2}-O-(CH_2)_{m3}-$, or $-(CH_2)_{m1}-O-(CH_2)_{m2}-O-(CH_2)_{m3}-O-(CH_2)_{m4}-$, wherein m1, m2, m3, and m4 each independently represent an integer of 1 to 20.

**8.** A nanosheet comprising the protein assembly or the salt thereof according to claim 6.

**9.** A device comprising at least one selected from the group consisting of a protein monomer, a protein monomer salt, a protein polymer, a protein polymer salt, a protein assembly, and a protein assembly salt,
the protein monomer or the protein monomer salt being the protein monomer or the salt thereof according to claim 1 or the protein monomer or the salt thereof according to claim 2,
the protein polymer or the protein polymer salt being the protein polymer or the salt thereof according to claim 3 or the protein polymer or the salt thereof according to claim 4, and
the protein assembly or the protein assembly salt being the protein assembly or the protein assembly salt according to claim 6.

**10.** A substrate modified with at least one selected from the group consisting of a protein monomer, a protein monomer salt, a protein polymer, and a protein polymer salt,
the protein monomer or the protein monomer salt being the protein monomer or the salt thereof according to claim 2 wherein M is Fe, and

the protein polymer or the protein polymer salt being the protein polymer or the salt thereof according to claim 3 wherein M is Fe or the protein polymer or the salt thereof according to claim 4 wherein M is Fe.

FIG. 1a

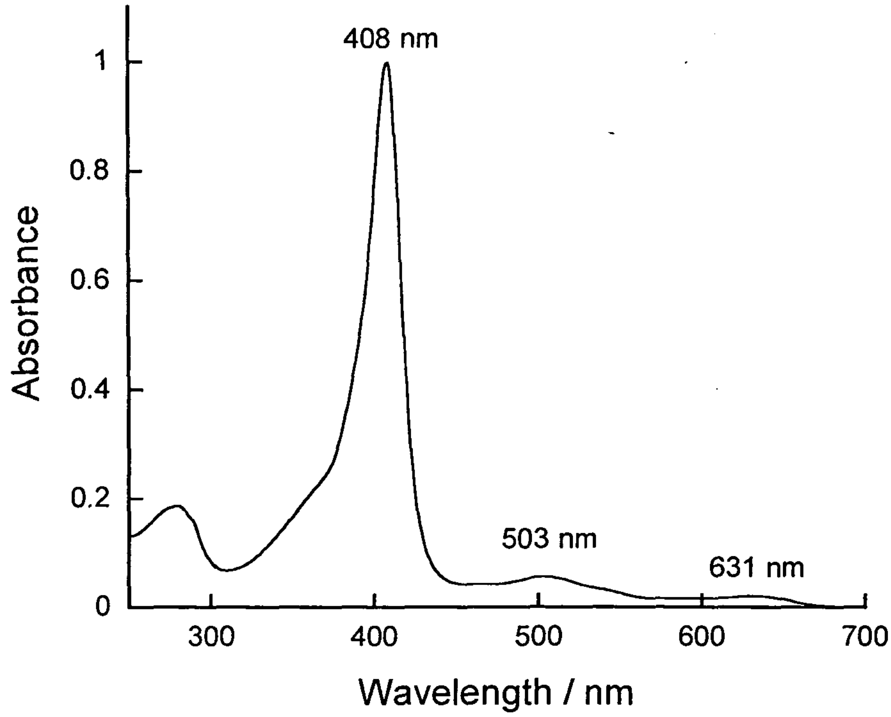
408 nm
503 nm
631 nm
Absorbance
0
0.2
0.4
0.6
0.8
1
300
400
500
600
700
Wavelength / nm

FIG. 1b

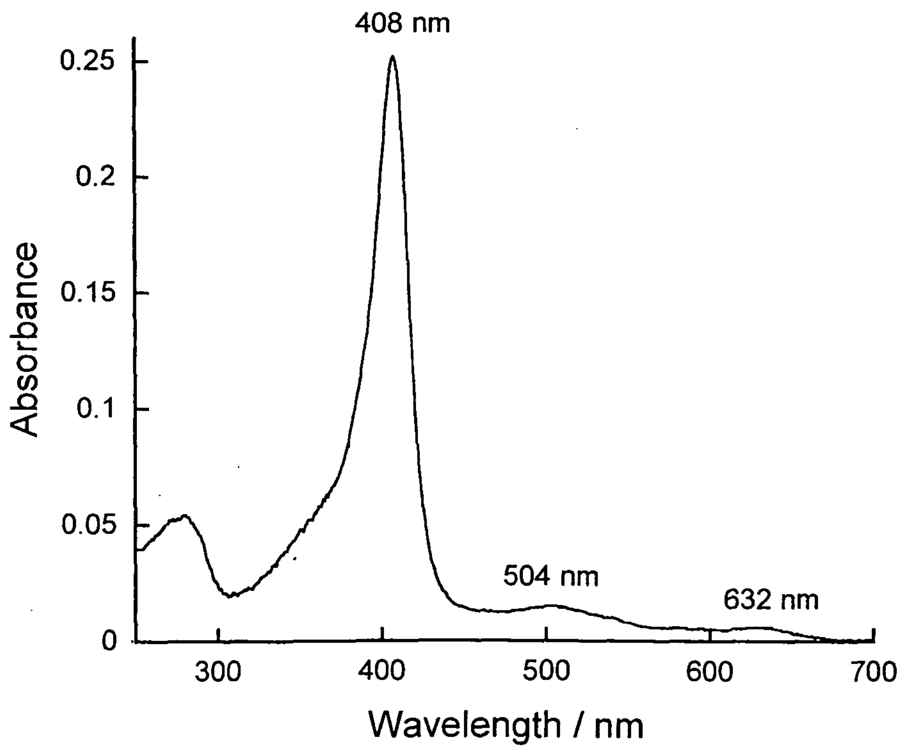
408 nm
504 nm
632 nm
Absorbance
0
0.05
0.1
0.15
0.2
0.25
300
400
500
600
700
Wavelength / nm

FIG. 1c

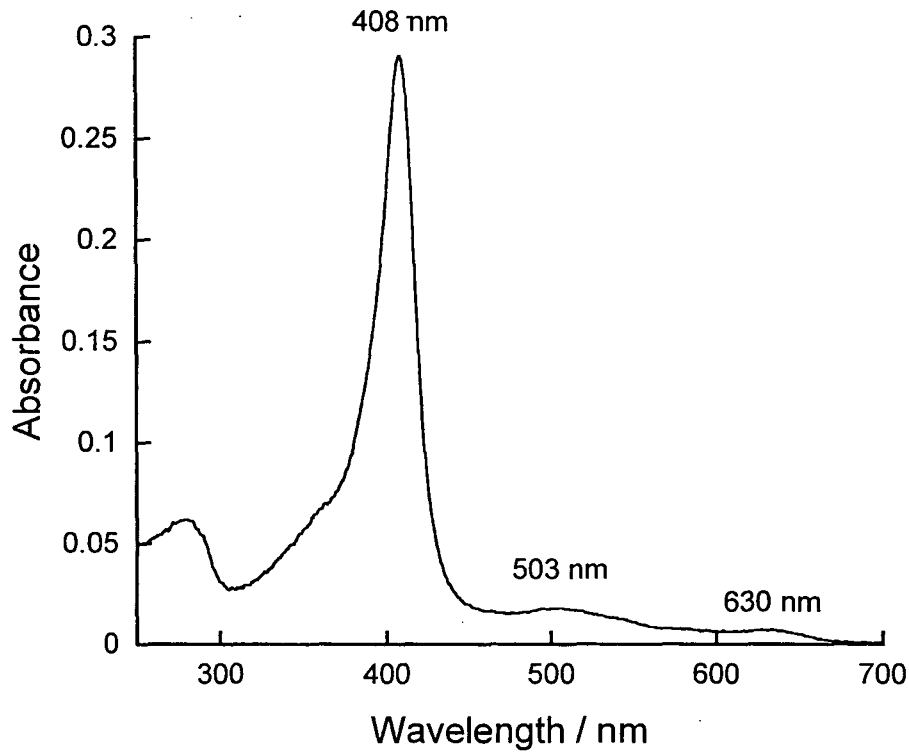
408 nm
503 nm
630 nm
Absorbance
0.3
0.25
0.2
0.15
0.1
0.05
0
300
400
500
600
700
Wavelength / nm

FIG. 1d

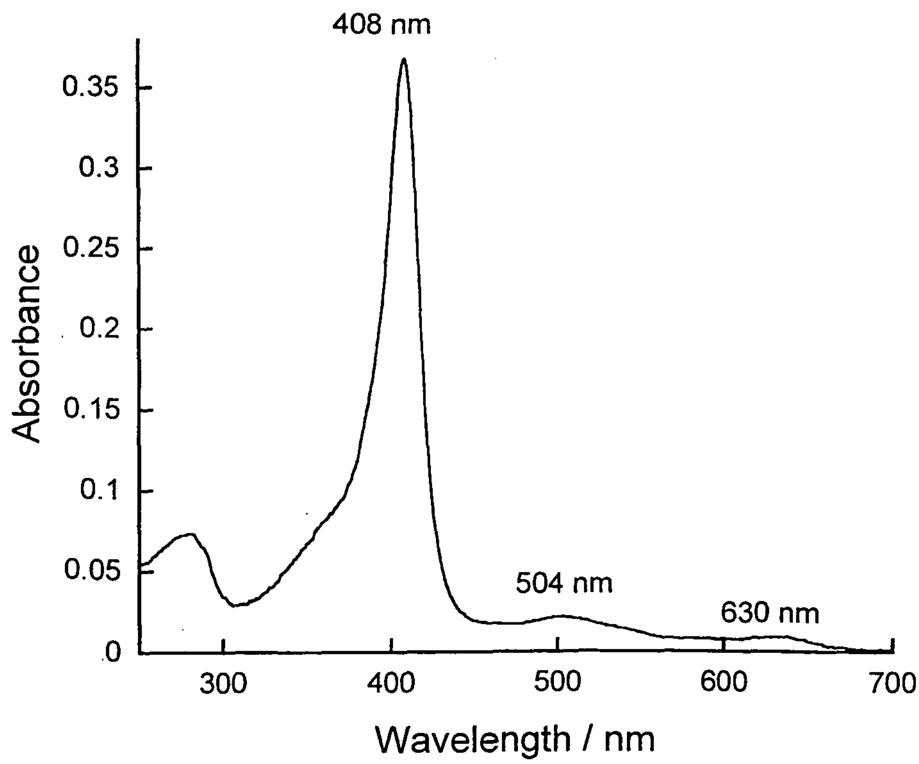
408 nm
504 nm
630 nm
Absorbance
0.35
0.3
0.25
0.2
0.15
0.1
0.05
0
300
400
500
600
700
Wavelength / nm

FIG. 1e

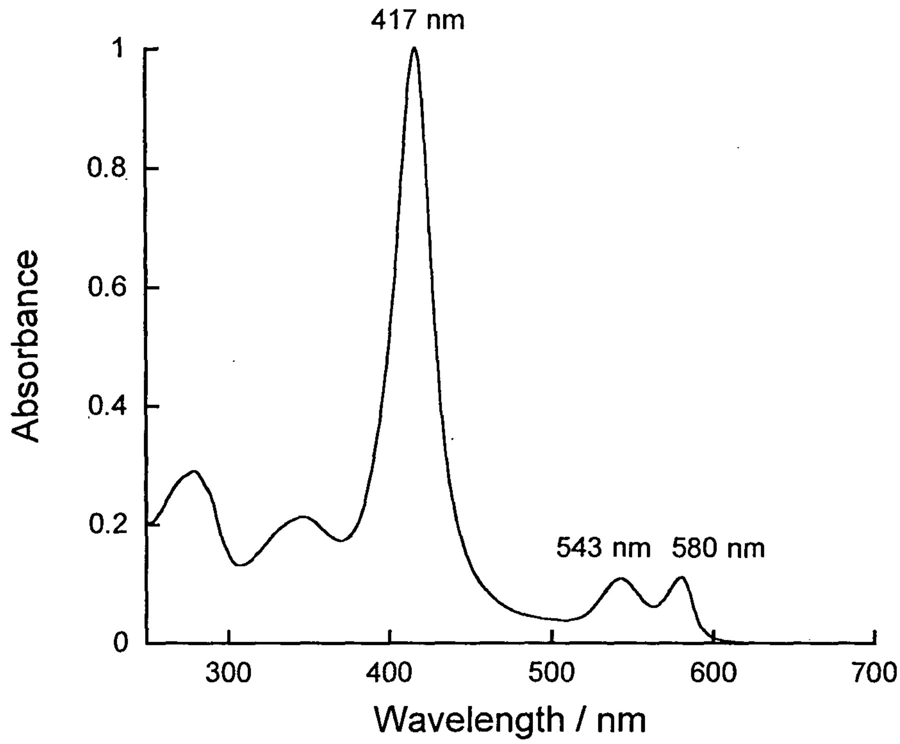
417 nm
543 nm
580 nm
Absorbance
Wavelength / nm
0
0.2
0.4
0.6
0.8
1
300
400
500
600
700

FIG. 1f

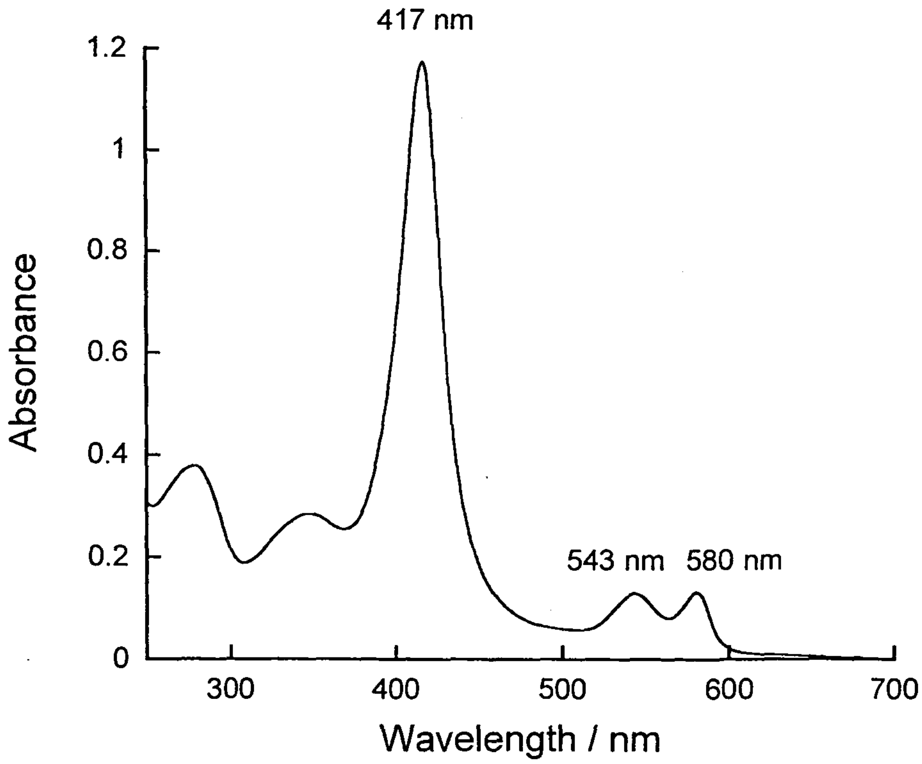
417 nm
543 nm
580 nm
Absorbance
Wavelength / nm
0
0.2
0.4
0.6
0.8
1
1.2
300
400
500
600
700

FIG. 2a

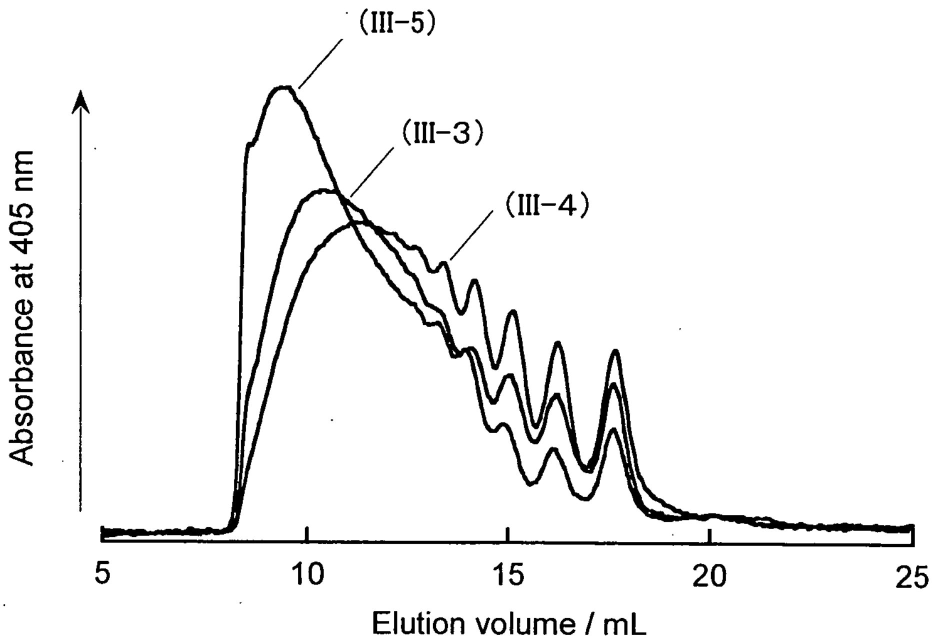
(III-5)
(III-3)
(III-4)
Absorbance at 405 nm
5
10
15
20
25
Elution volume / mL

FIG. 2b

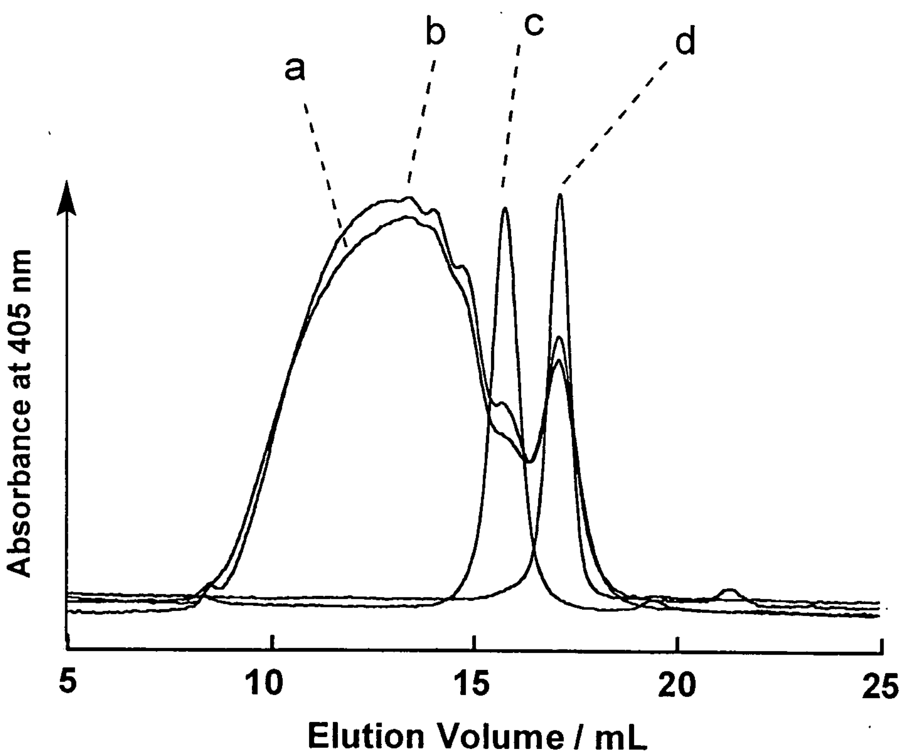
a
b
c
d
Absorbance at 405 nm
5
10
15
20
25
Elution Volume / mL

FIG. 2c

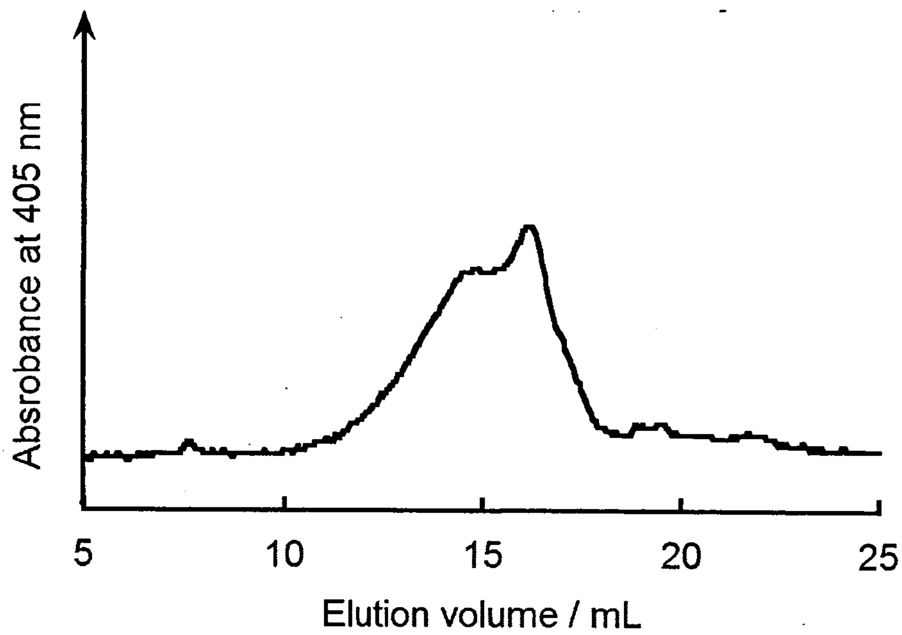
Absrobance at 405 nm
5
10
15
20
25
Elution volume / mL

FIG. 3

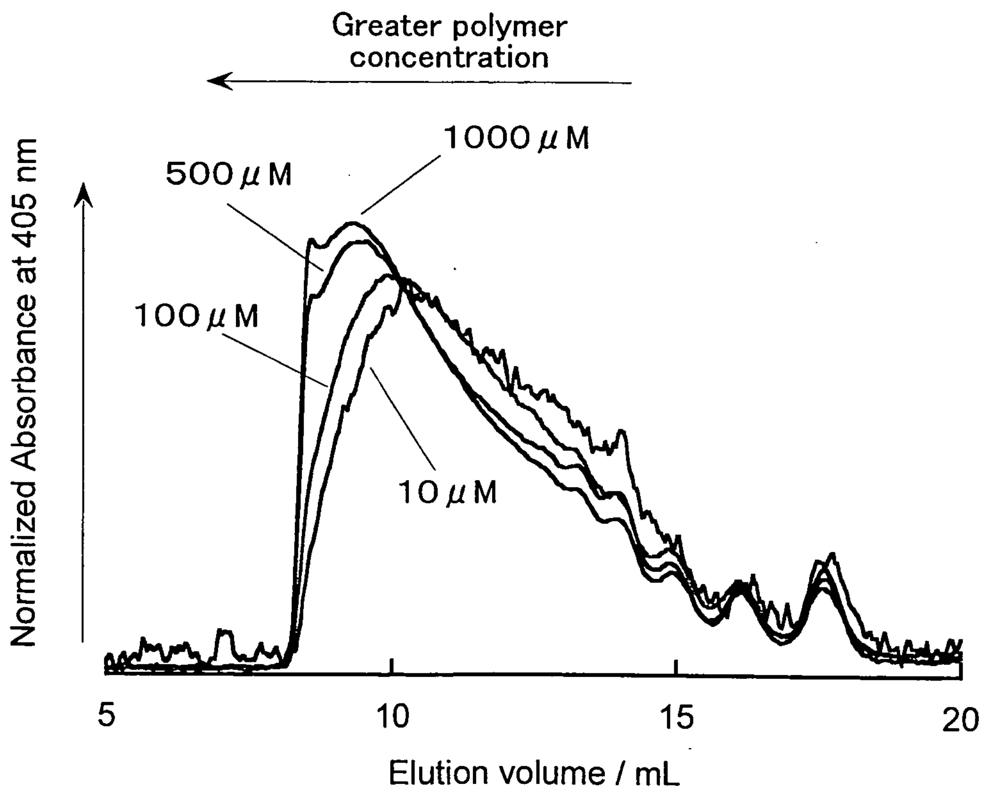
Greater polymer concentration
1000 μM
500 μM
100 μM
10 μM
Normalized Absorbance at 405 nm
5
10
15
20
Elution volume / mL

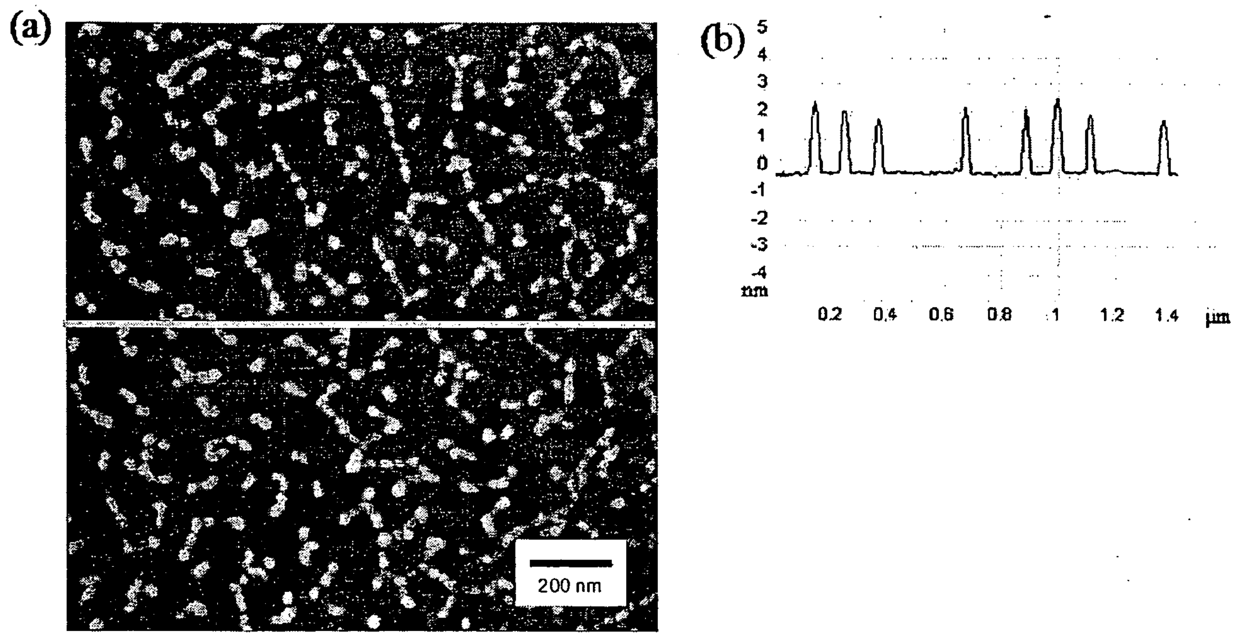
(a)
200 nm
(b)
5
4
3
2
1
0
-1
-2
-3
-4
nm
0.2
0.4
0.6
0.8
1
1.2
1.4
µm

FIG. 4a

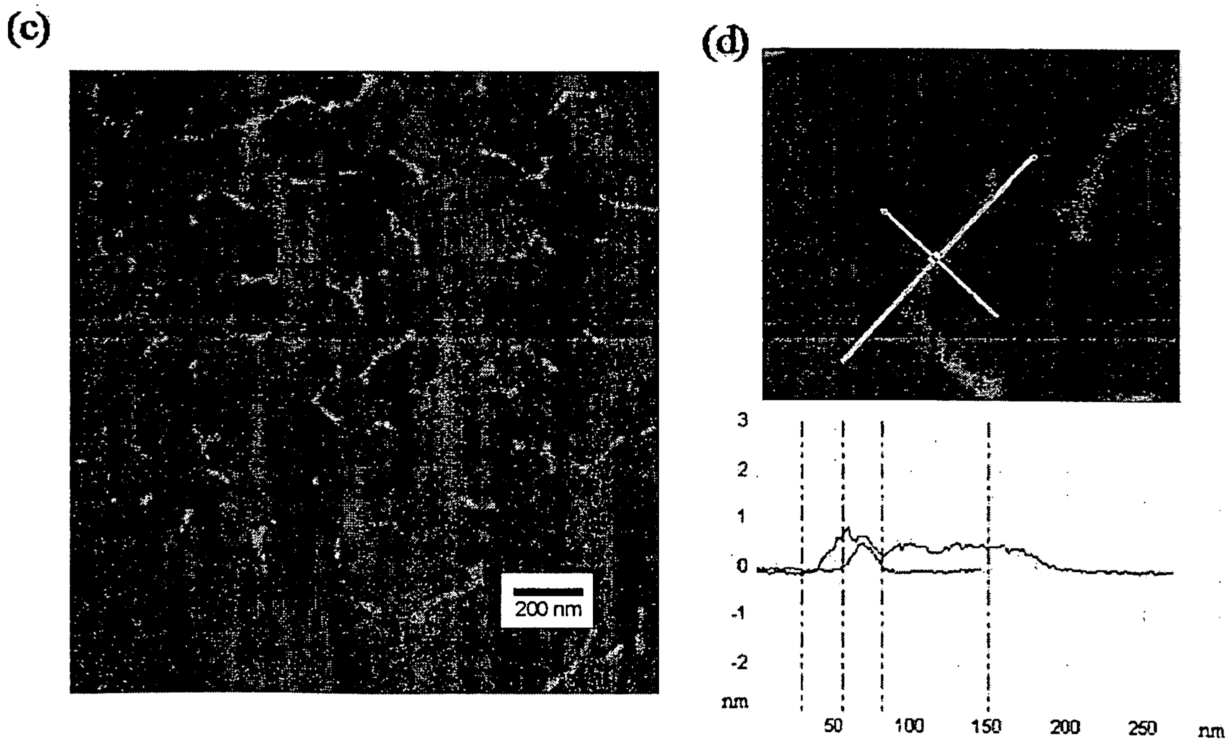
(c)
200 nm
(d)
3
2
1
0
-1
-2
nm
50
100
150
200
250
nm

FIG. 4b

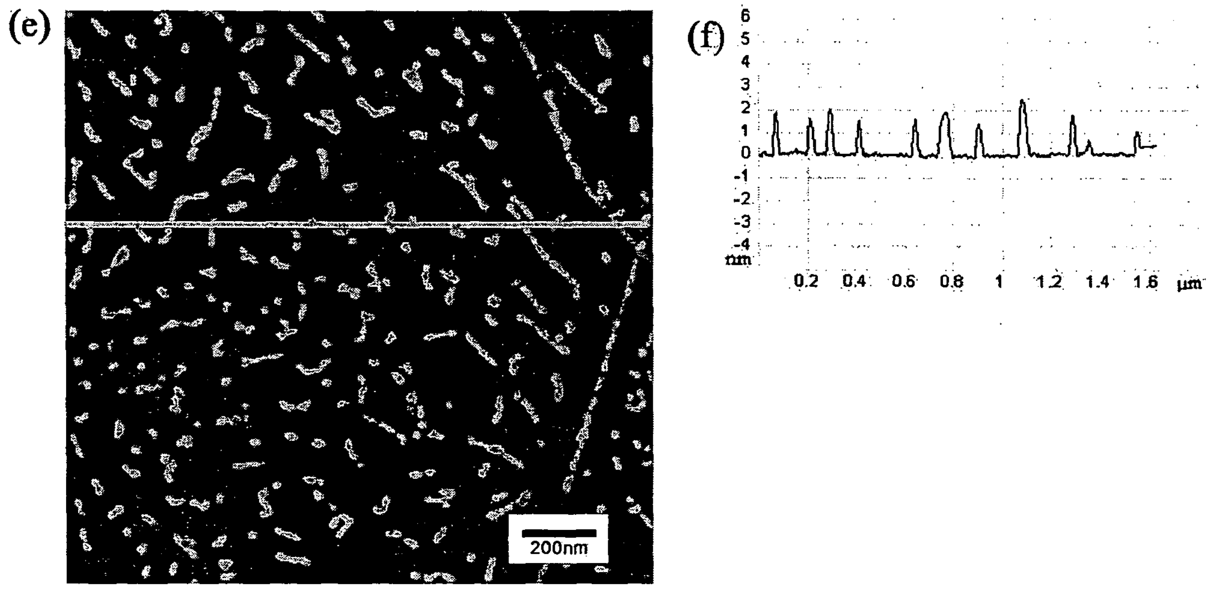


FIG. 4c

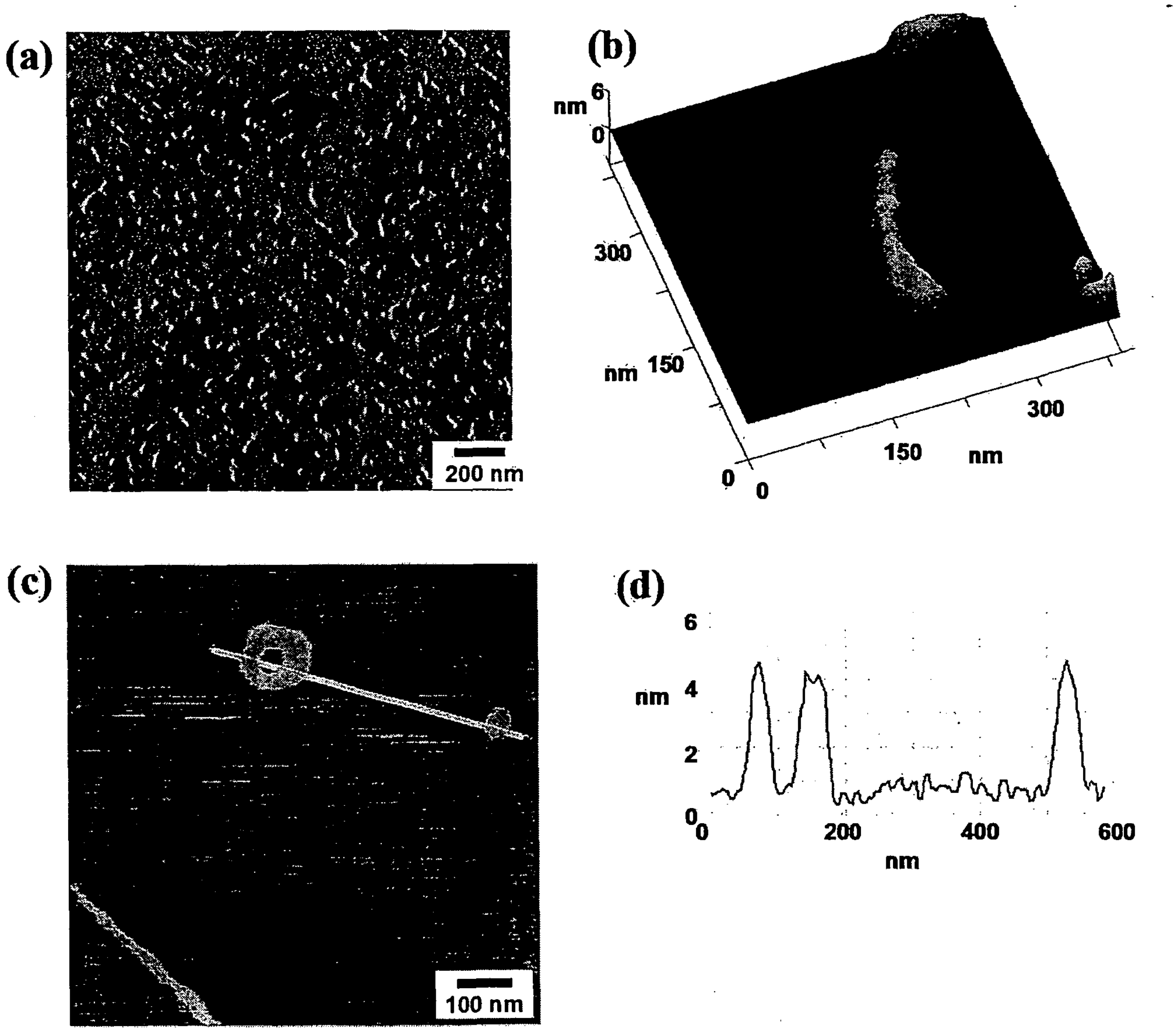
(a)
200 nm
(b)
nm
6
0
300
nm 150
0
0
150
300
nm
(c)
100 nm
(d)
6
nm 4
2
0
0
200
400
600
nm

FIG. 4d

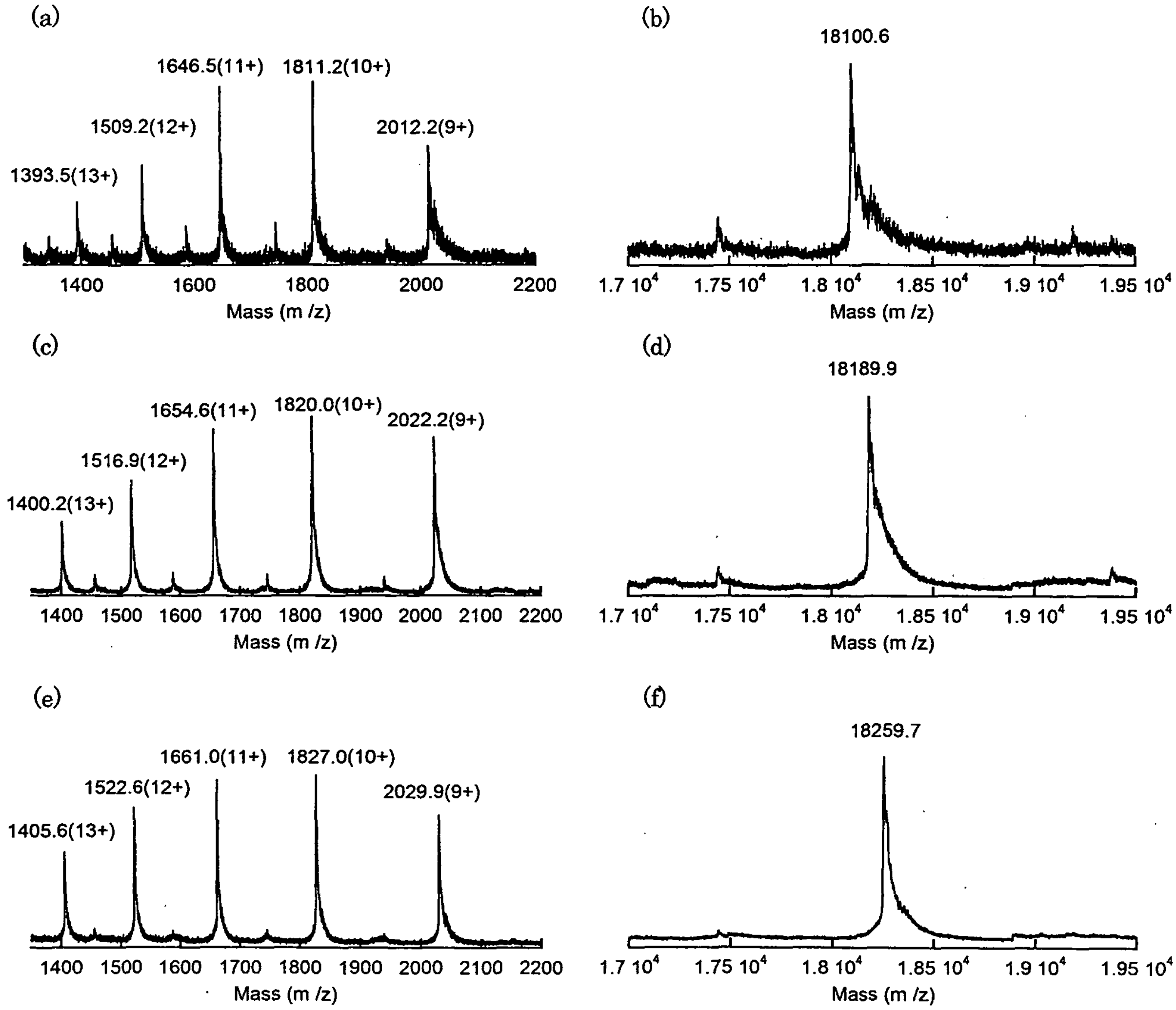

(a)
1393.5(13+)
1509.2(12+)
1646.5(11+)
1811.2(10+)
2012.2(9+)
1400
1600
1800
2000
2200
Mass (m /z)
(b)
18100.6
1.7 10⁴
1.75 10⁴
1.8 10⁴
1.85 10⁴
1.9 10⁴
1.95 10⁴
Mass (m /z)
(c)
1400.2(13+)
1516.9(12+)
1654.6(11+)
1820.0(10+)
2022.2(9+)
1400 1500 1600 1700 1800 1900 2000 2100 2200
Mass (m /z)
(d)
18189.9
1.7 10⁴
1.75 10⁴
1.8 10⁴
1.85 10⁴
1.9 10⁴
1.95 10⁴
Mass (m /z)
(e)
1405.6(13+)
1522.6(12+)
1661.0(11+)
1827.0(10+)
2029.9(9+)
1400 1500 1600 1700 1800 1900 2000 2100 2200
Mass (m /z)
(f)
18259.7
1.7 10⁴
1.75 10⁴
1.8 10⁴
1.85 10⁴
1.9 10⁴
1.95 10⁴
Mass (m /z)


FIG. 5

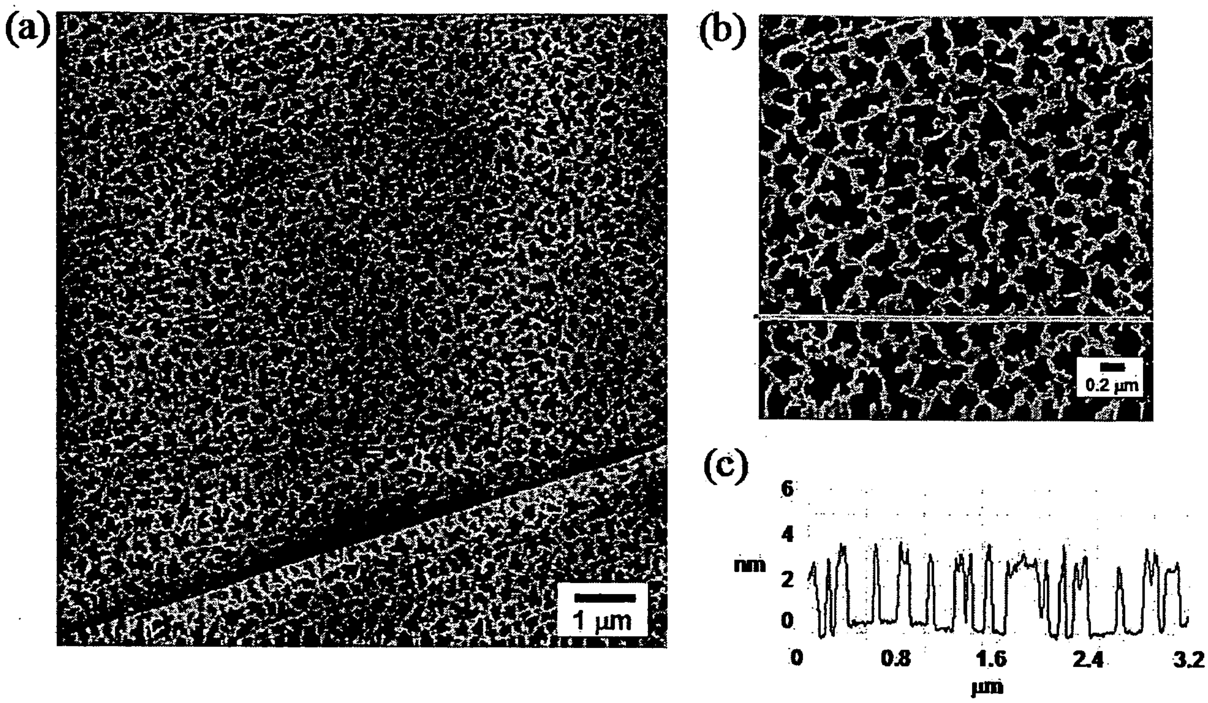


FIG. 6

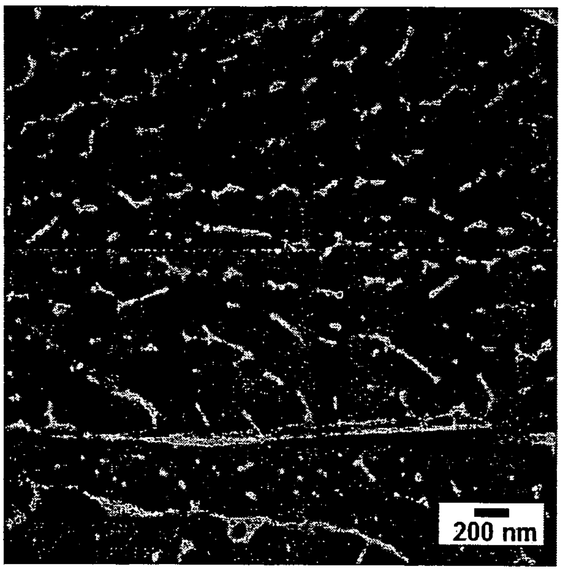


FIG. 7

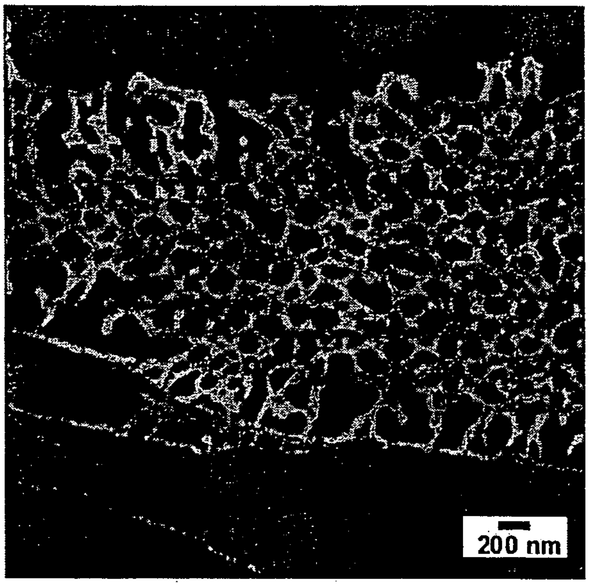


FIG. 8

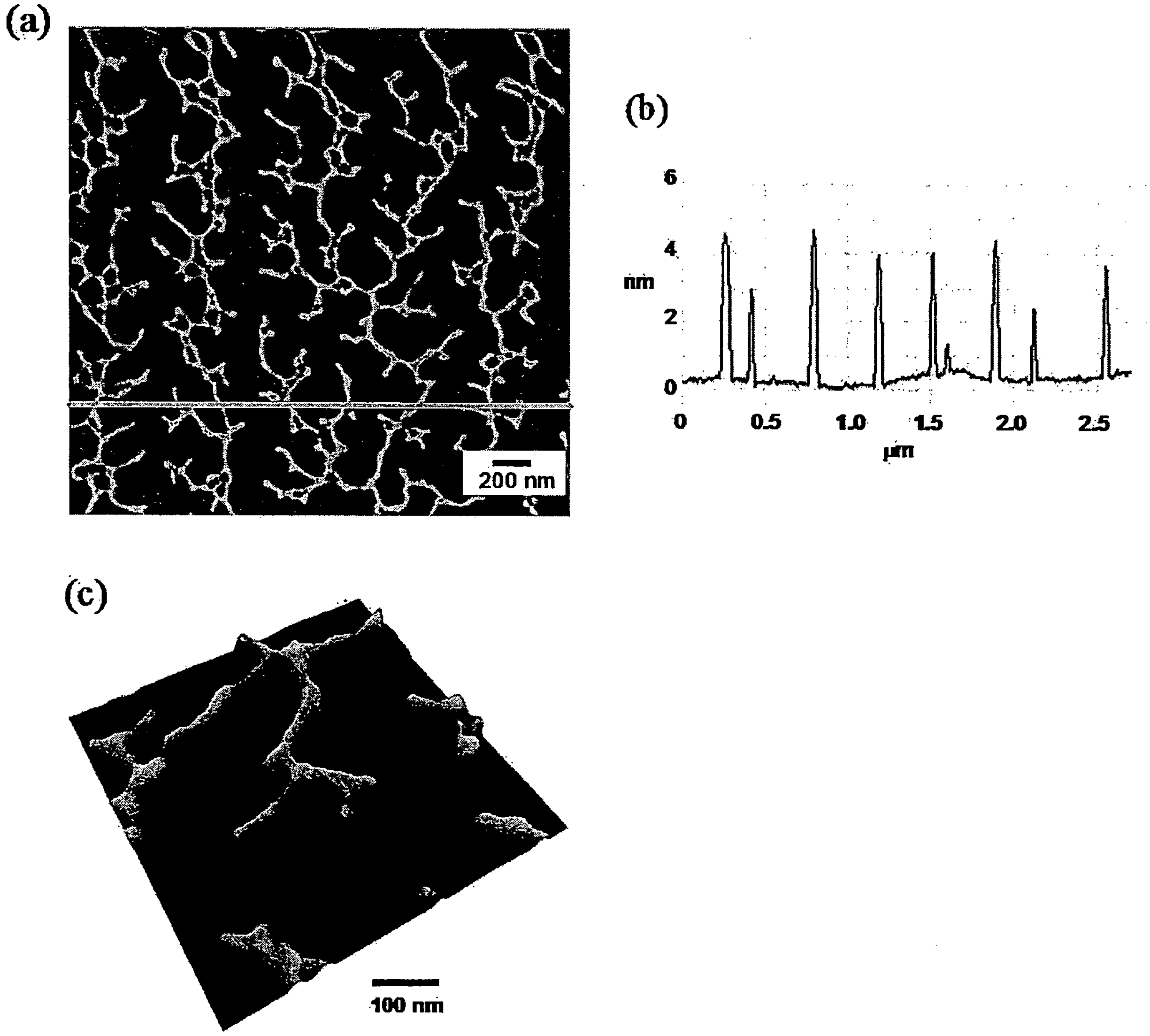


FIG. 9

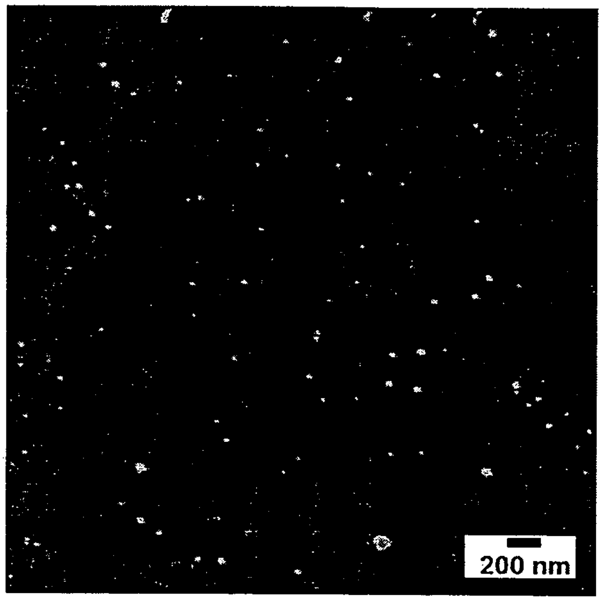


FIG. 10

FIG. 11 (a)

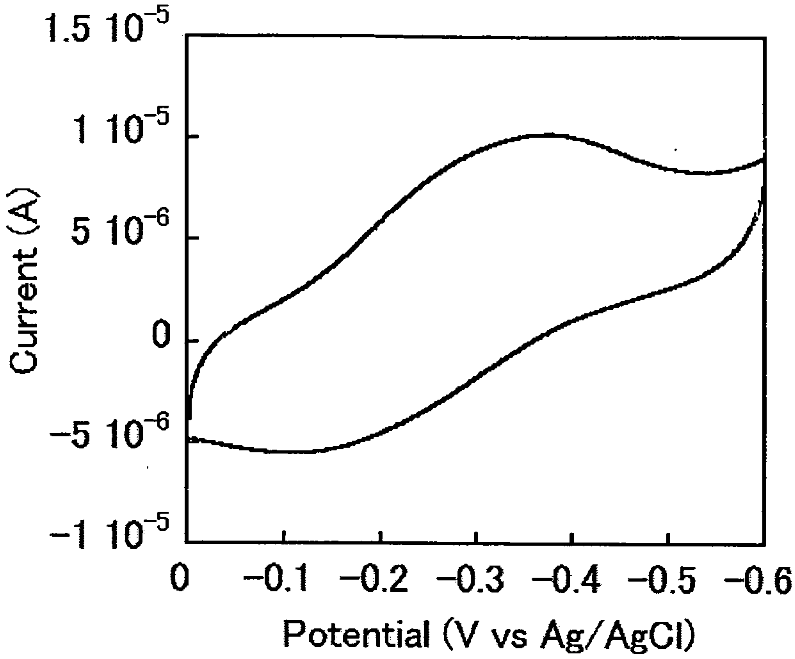


FIG. 11 (b)

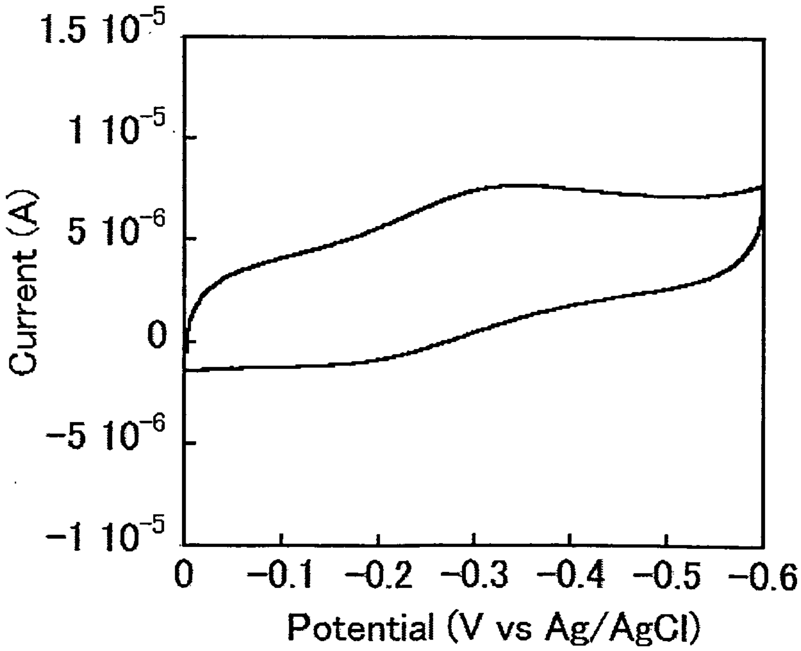

FIG. 11(c)

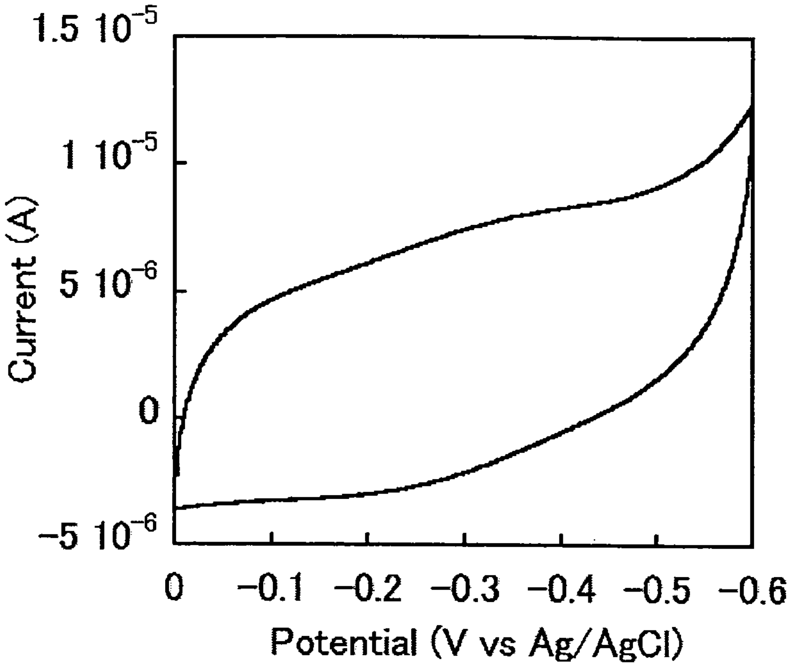


FIG. 12(a)

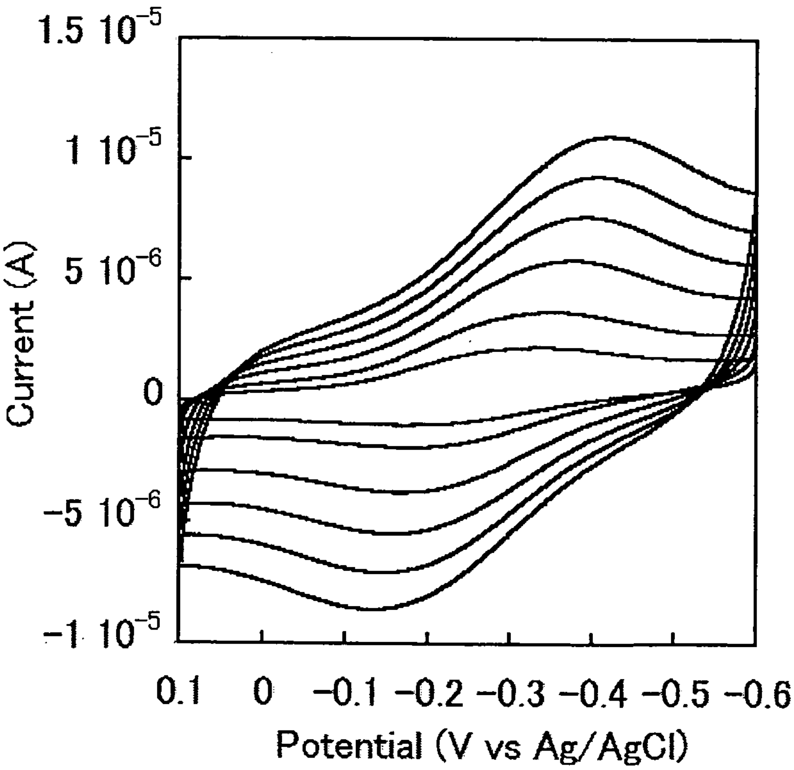

FIG. 12(b)

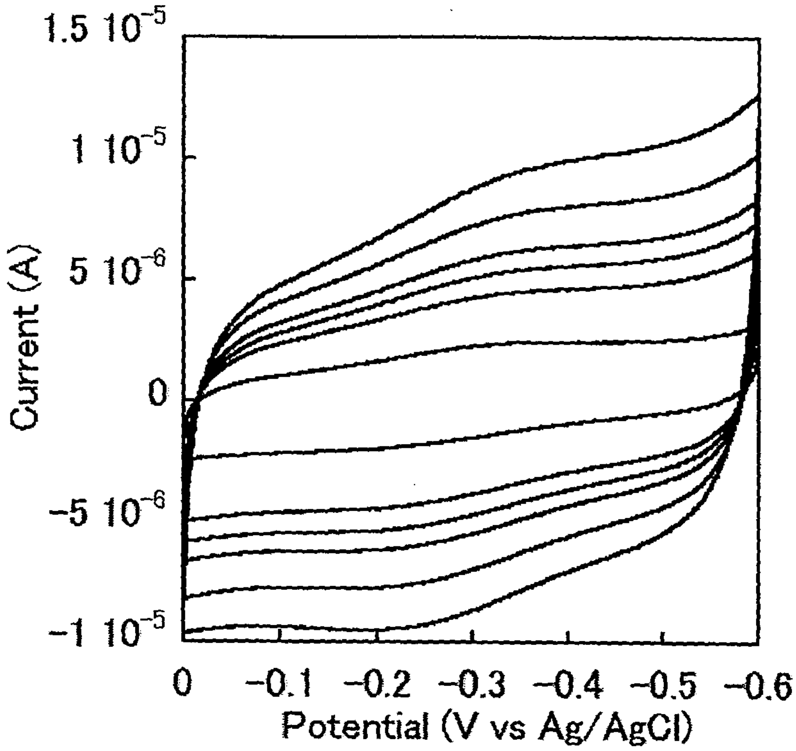


FIG. 12(c)

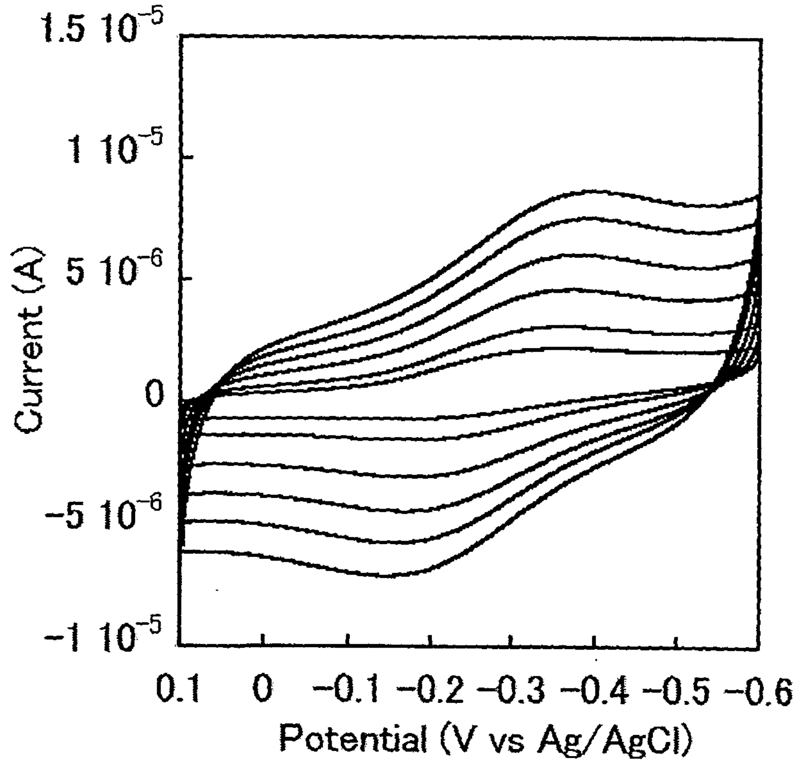

FIG. 12(d)

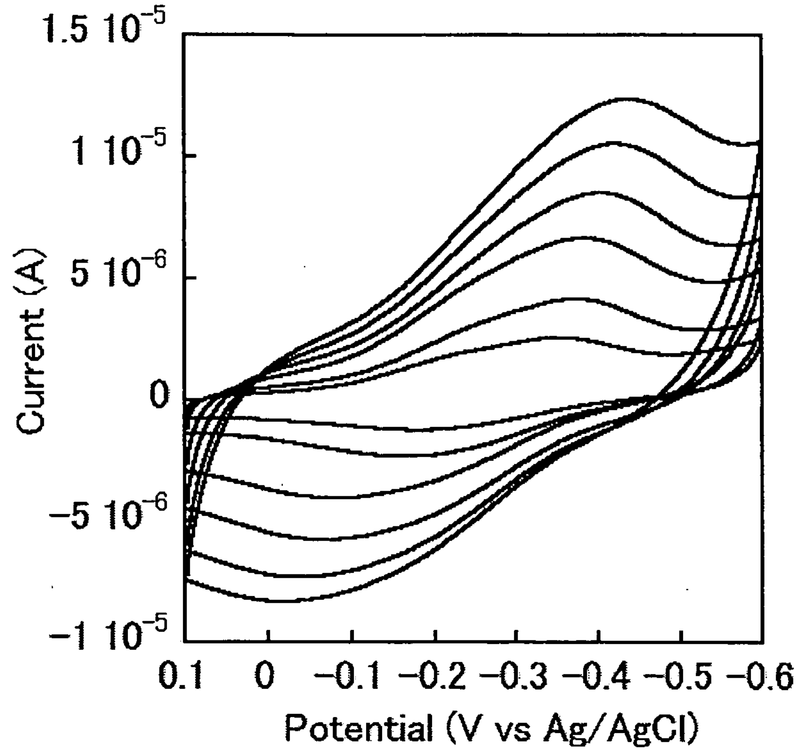


FIG. 12(e)

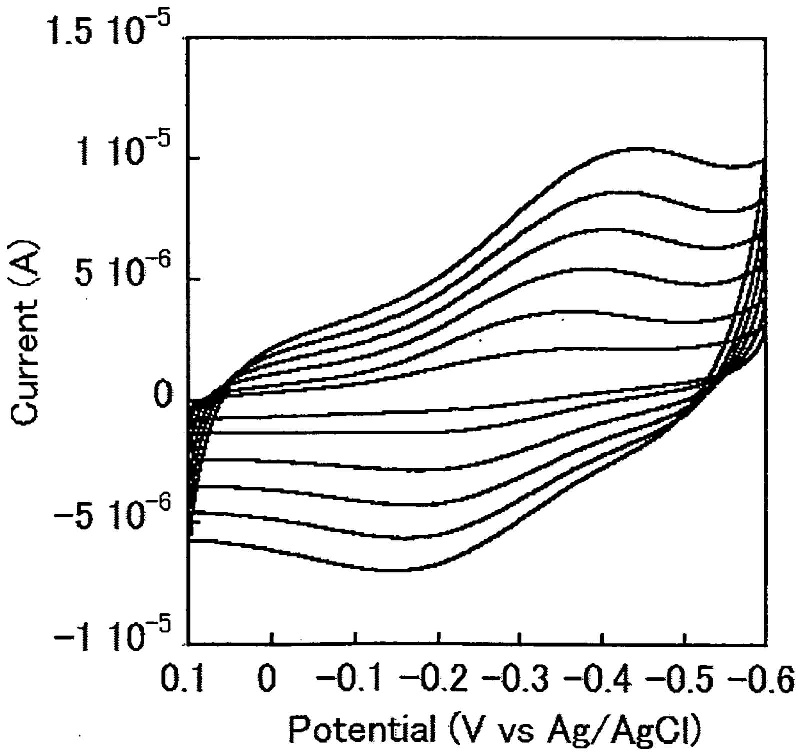

FIG. 13(a)

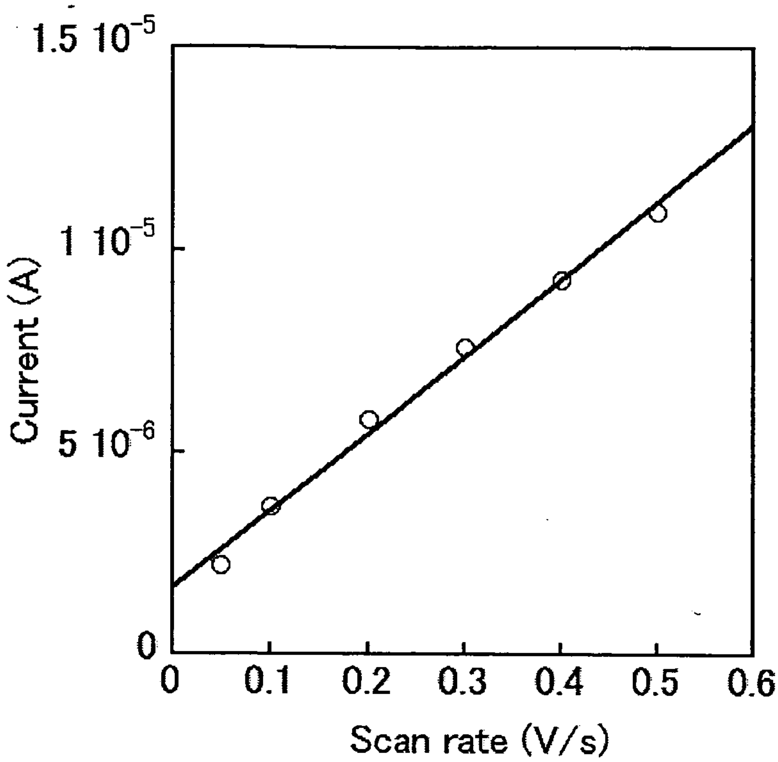


FIG. 13(b)

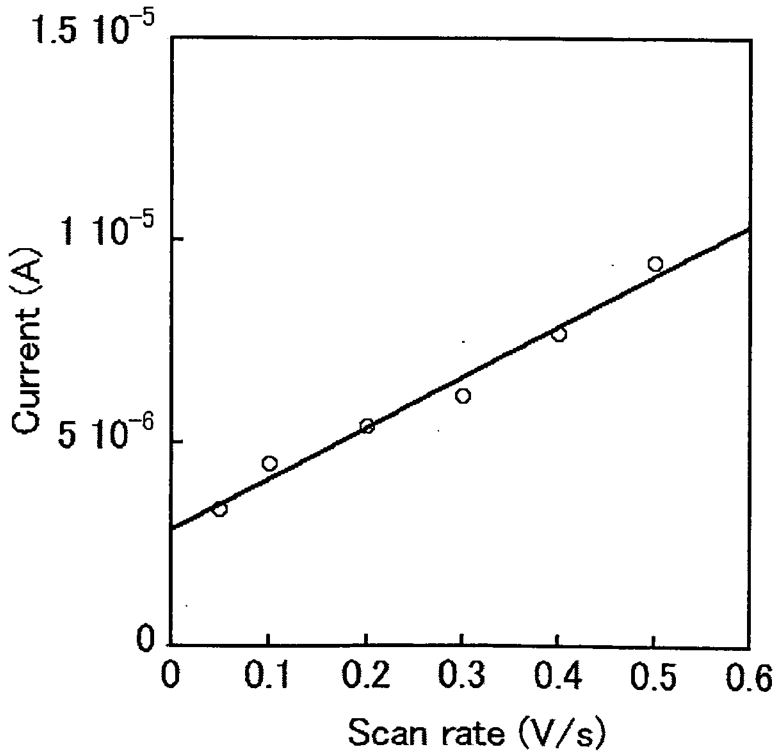

FIG. 13(c)

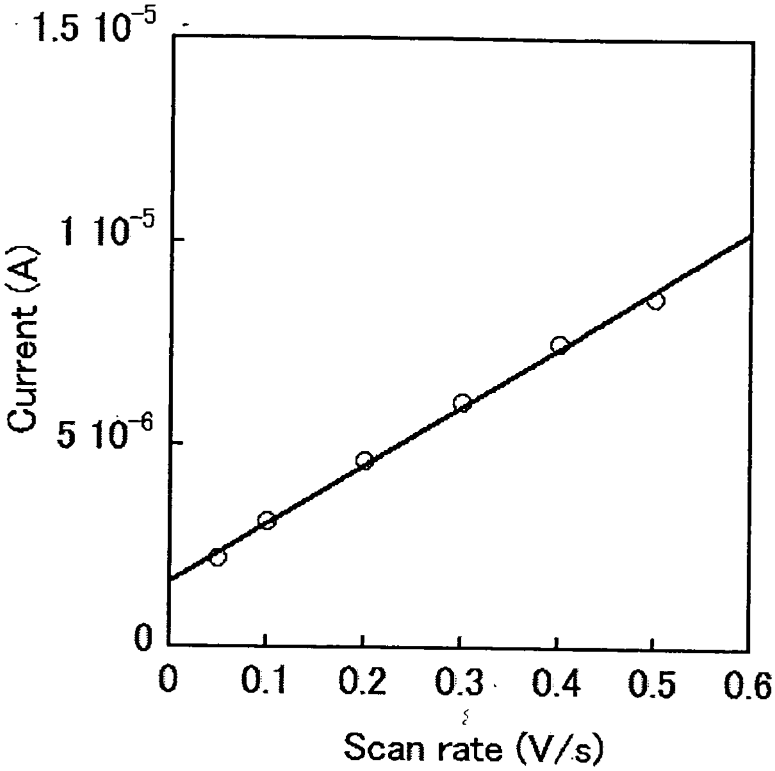


FIG. 13(d)

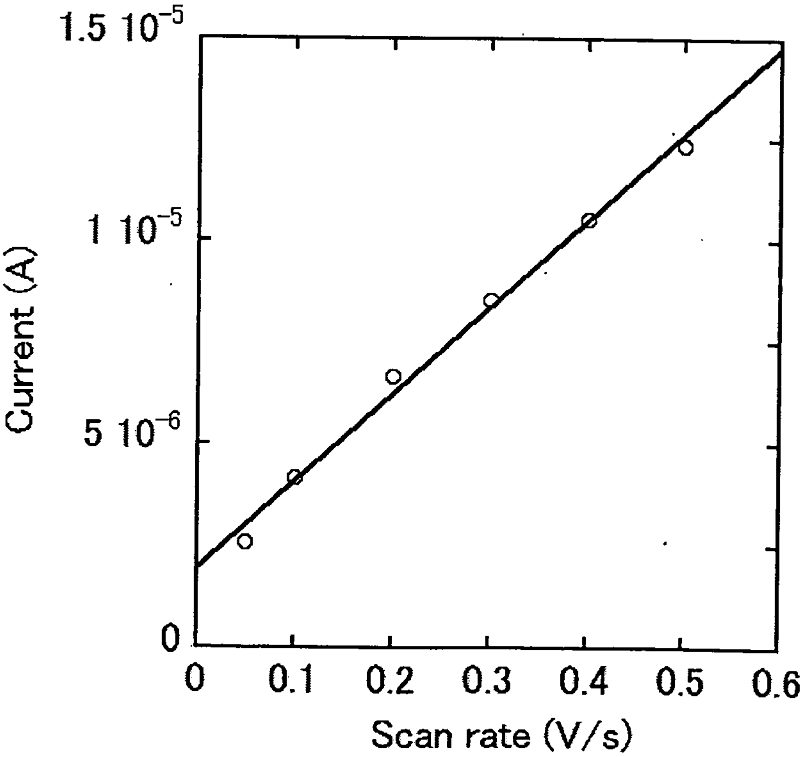

FIG. 13(e)

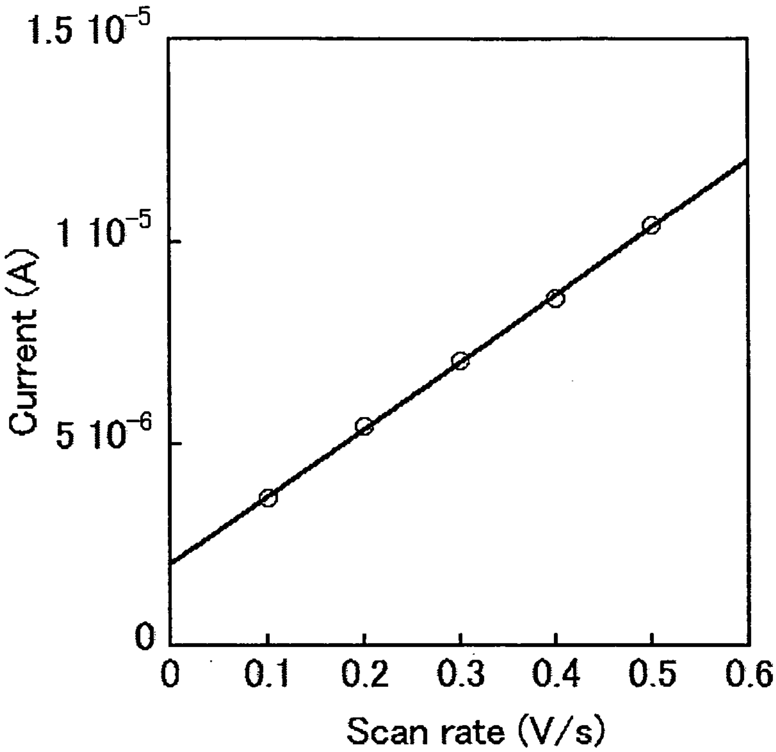


FIG. 14(a)

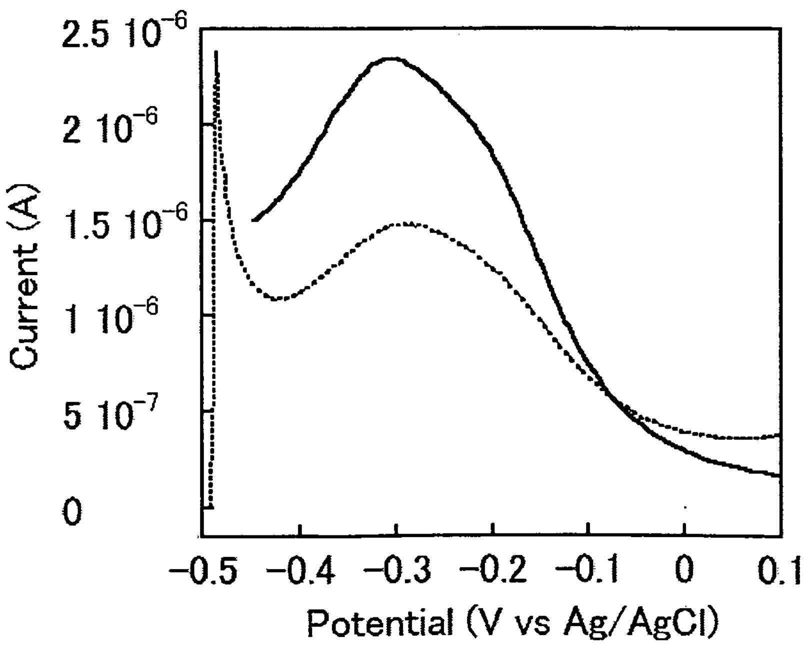

FIG. 14(b)

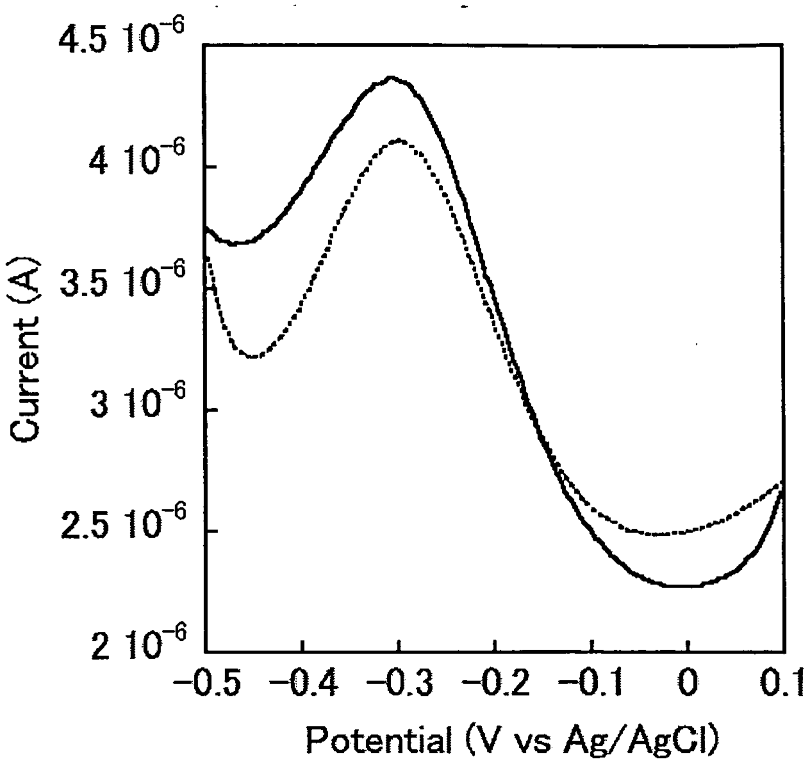


FIG. 14(c)

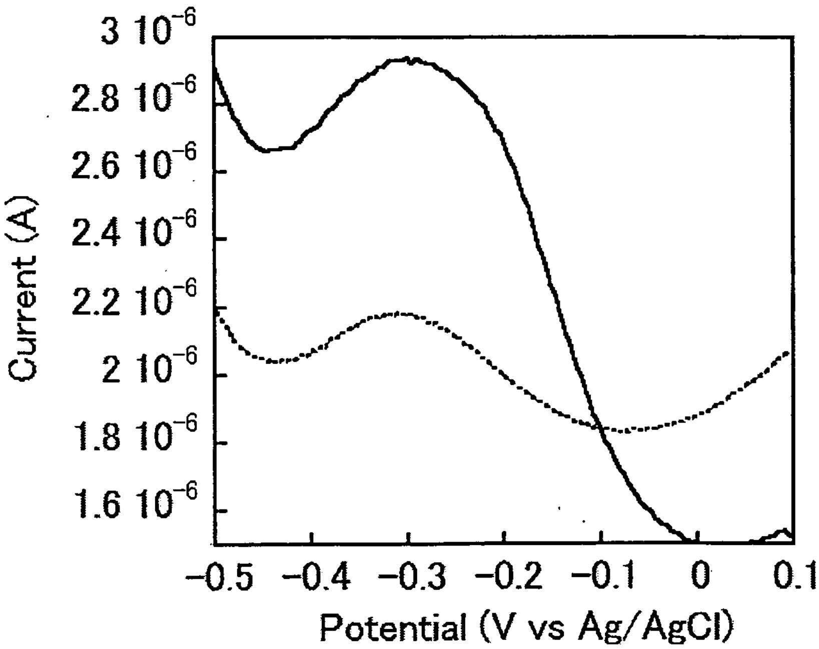

FIG. 14(d)

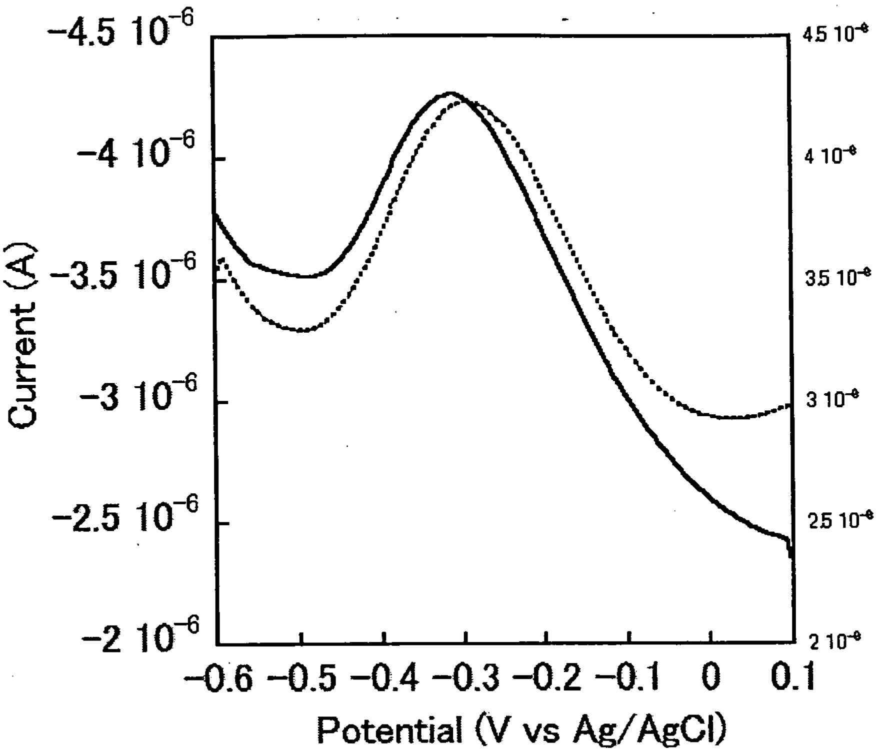


FIG. 14(e)

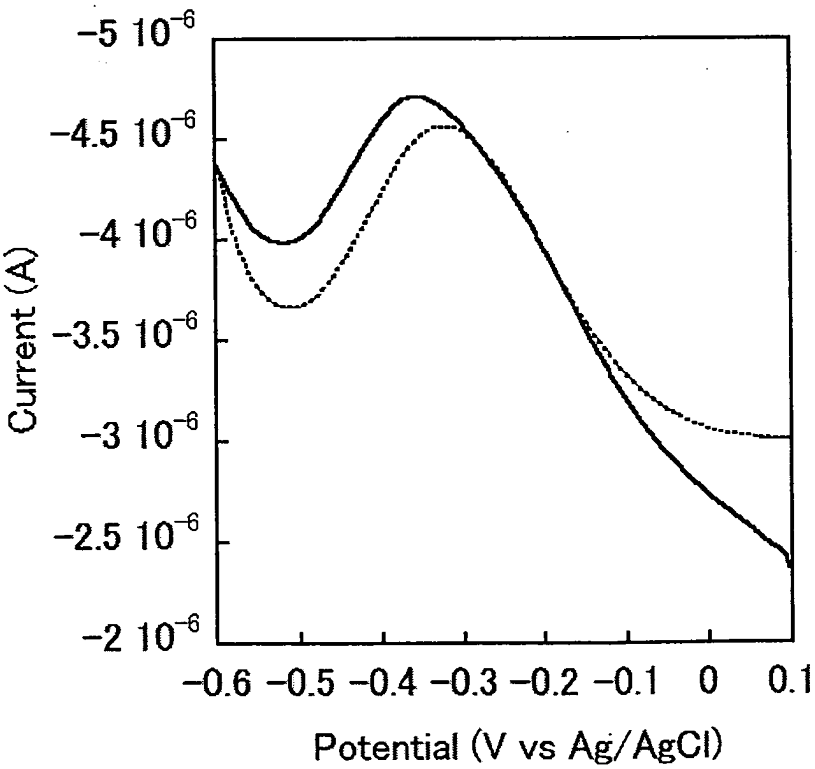

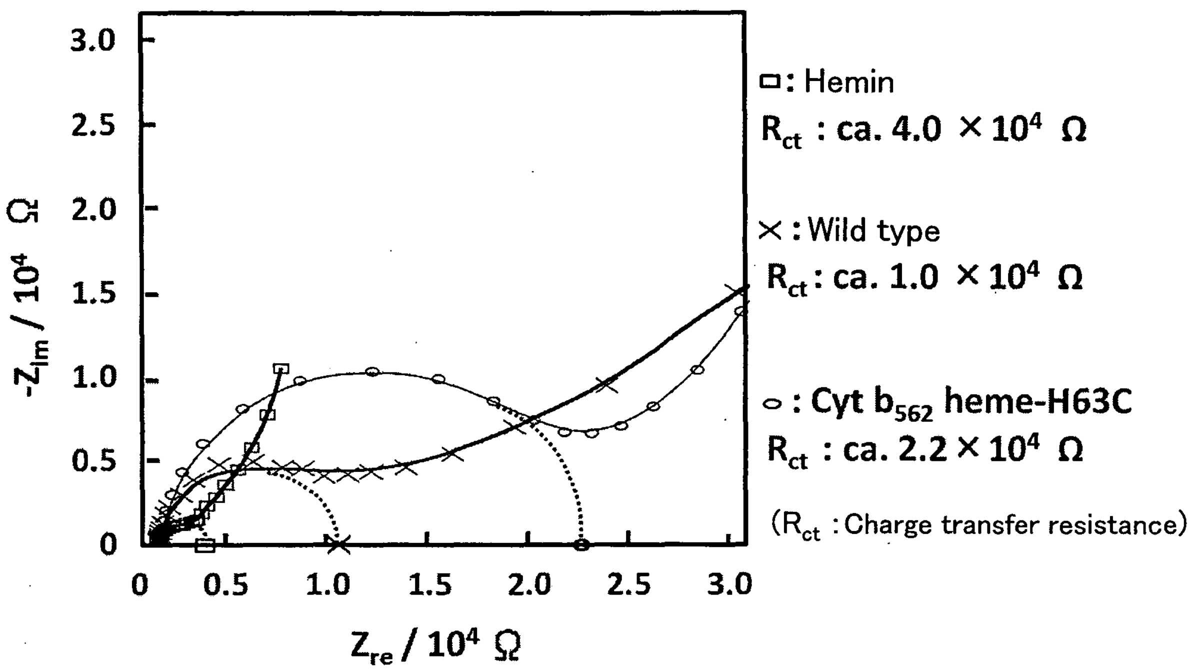
3.0
2.5
2.0
1.5
1.0
0.5
0
$-Z_{Im}$ / $10^4$ Ω
0
0.5
1.0
1.5
2.0
2.5
3.0
$Z_{re}$ / $10^4$ Ω
□: Hemin
$R_{ct}$ : ca. 4.0 × $10^4$ Ω
×: Wild type
$R_{ct}$: ca. 1.0 × $10^4$ Ω
○: Cyt $b_{562}$ heme-H63C
$R_{ct}$ : ca. 2.2 × $10^4$ Ω
($R_{ct}$ : Charge transfer resistance)

FIG. 15

## INTERNATIONAL SEARCH REPORT

International application No.
PCT/JP2009/054569

A. CLASSIFICATION OF SUBJECT MATTER
*C07K14/795*(2006.01)i

According to International Patent Classification (IPC) or to both national classification and IPC

B. FIELDS SEARCHED

Minimum documentation searched (classification system followed by classification symbols)
C07K14/795

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
GenBank/EMBL/DDBJ/GeneSeq, SwissProt/PIR/GeneSeq, PubMed, Science Direct, Wiley InterScience, ACSPUBLICATIONS, JSTPlus(JDreamII), BIOSIS/WPI(DIALOG)

C. DOCUMENTS CONSIDERED TO BE RELEVANT

| Category* | Citation of document, with indication, where appropriate, of the relevant passages | Relevant to claim No. |
|---|---|---|
| Y<br>A | KITAGISHI H., Supramolecular Hemoprotein Linear Assembly by Successive Interprotein Heme-Heme Pocket Interactions., J. Am. Chem. Soc., 2007, vol.129, p.10326-10327 | 1-5,9,10<br>6-8 |
| Y<br>A | SHAFER D.E., Reaction of Tris(2-carboxyethyl) phosphine (TCEP) with maleimide and alpha-haloacyl groups: anomalous elution of TCEP by gel filtration., Anal. Biochem., 2000, vol.282, p.161-164 | 1-5,9,10<br>6-8 |
| Y<br>A | ROGERS L.K., Detection of reversible protein thiol modifications in tissues., Anal. Biochem., 2006, vol.358, p.171-184 | 1-5,9,10<br>6-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 May, 2009 (25.05.09) | 02 June, 2009 (02.06.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

INTERNATIONAL SEARCH REPORT

International application No.
PCT/JP2009/054569

C (Continuation). DOCUMENTS CONSIDERED TO BE RELEVANT

| Category* | Citation of document, with indication, where appropriate, of the relevant passages | Relevant to claim No. |
|---|---|---|
| P,X | KITAGISHI H., Self-assembly of one- and two-dimensional hemoprotein systems by polymerization through heme-heme pocket interactions., Angew. Chem. Int. Ed. Engl., 2008.12.03, vol.48, p.1271-1274 | 1-10 |
| P,Y | KITAGISHI H, Thermodynamically controlled supramolecular polymerization of cytochrome b 562., Biopolymers, 2008.11.10, vol.91, p.194-200 | 1-5,9,10 |
| P,Y | JP 2008-222565 A (Osaka University), 25 September, 2008 (25.09.08), Particularly, Claims; examples (Family: none) | 1-5,9,10 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **J. M. Lehn.** Supramolecular Chemistry: Concepts and Perspectives. VCH Publication, 1995
- **A. Das et al.** *J. Biophysical Chemistry,* 2006, vol. 123, 102-112
- **T. Matsuo ; T. Hayashi ; Y Hisaeda.** *J. Am. Chem. Soc.,* 2002, vol. 124, 11234
- **M. Trester-Zedlitz ; K. Kamada ; S.K Burley ; D. Fenyo ; B.T. Chait ; T.W Muir.** *J. Am. Chem. Soc.,* 2003, vol. 125, 2416
- **R. Schneider ; F. Schmitt ; C. Frochot ; Y Fort ; N. Lourette ; F. Guillemin ; J.F. Mueller ; M. Barberi-Heyob.** *Bioorg. Med., Chem.,* 2005, vol. 13, 2799
- **O. Keller ; J. Rudinger.** *Helv Chim. Acta.,* 1975, vol. 58, 531
- **Y Kawamata ; S. Machida ; T. Ogawa ; K. Horie ; T. Nagamune.** *J. Lumin.,* 2002, vol. 98, 141
- **S. Hirota ; K. Azuma ; M. Fukuda ; S. Kuroiwa ; N. Funasaki.** *Biochemistry,* 2005, vol. 44, 10322
- **B.A. Springer ; S.G. Sliger.** *Proc. Natl. Acad. Sci. USA,* 1987, vol. 84, 8961
- *HELVETICA CHIMICA ACTA,* 1975, vol. 58, 531-541
- **A. Das et al.** *Biophysical Chemistry,* 2006, vol. 123, 102-112

SEQUENCE LISTING

<110> Osaka University

<120> Hemprotein polymer

<130> H3381

<160> 10

<170> PatentIn version 3.1

<210> 1
<211> 106
<212> PRT
<213> Escherichia coli

<400> 1

Ala Asp Leu Glu Asp Asn Met Glu Thr Leu Asn Asp Asn Leu Lys Val
1 5 10 15

Ile Glu Lys Ala Asp Asn Ala Ala Gln Val Lys Asp Ala Leu Thr Lys
20 25 30

Met Arg Ala Ala Ala Leu Asp Ala Gln Lys Ala Thr Pro Pro Lys Leu
35 40 45

Glu Asp Lys Ser Pro Asp Ser Pro Glu Met Lys Asp Phe Arg His Gly
50 55 60

Phe Asp Ile Leu Val Gly Gln Ile Asp Asp Ala Leu Lys Leu Ala Asn
65 70 75 80

Glu Gly Lys Val Lys Glu Ala Gln Ala Ala Ala Glu Gln Leu Lys Thr
85 90 95

Thr Arg Asn Ala Tyr His Gln Lys Tyr Arg
100 105

<210> 2

<211> 154
<212> PRT
<213> Escherichia coli

<400> 2

```
Met Val Leu Ser Glu Gly Glu Trp Gln Leu Val Leu His Val Trp Ala
1               5                   10                  15

Lys Val Glu Ala Asp Val Ala Gly His Gly Gln Asp Ile Leu Ile Arg
            20                  25                  30

Leu Phe Lys Ser His Pro Glu Thr Leu Glu Lys Phe Asp Arg Phe Lys
        35                  40                  45

His Leu Lys Thr Glu Ala Glu Met Lys Ala Ser Glu Asp Leu Lys Lys
    50                  55                  60

His Gly Val Thr Val Leu Thr Ala Leu Gly Ala Ile Leu Lys Lys Lys
65                  70                  75                  80

Gly His His Glu Ala Glu Leu Lys Pro Leu Ala Gln Ser His Ala Thr
                85                  90                  95

Lys His Lys Ile Pro Ile Lys Tyr Leu Glu Phe Ile Ser Glu Ala Ile
            100                 105                 110

Ile His Val Leu His Ser Arg His Pro Gly Asn Phe Gly Ala Asp Ala
        115                 120                 125

Gln Gly Ala Met Asn Lys Ala Leu Glu Leu Phe Arg Lys Asp Ile Ala
    130                 135                 140

Ala Lys Tyr Lys Glu Leu Gly Tyr Gln Gly
145                 150
```

<210> 3
<211> 306

<212> PRT
<213> plant

<400> 3

Gln Leu Thr Pro Thr Phe Tyr Asp Asn Ser Cys Pro Asn Val Ser Asn
1 5 10 15

Ile Val Arg Asp Thr Ile Val Asn Glu Leu Arg Ser Asp Pro Arg Ile
20 25 30

Ala Ala Ser Ile Leu Arg Leu His Phe His Asp Cys Phe Val Asn Gly
35 40 45

Cys Asp Ala Ser Ile Leu Leu Asp Asn Thr Thr Ser Phe Arg Thr Glu
50 55 60

Lys Asp Ala Phe Gly Asn Ala Asn Ser Ala Arg Gly Phe Pro Val Ile
65 70 75 80

Asp Arg Met Lys Ala Ala Val Glu Ser Ala Cys Pro Arg Thr Val Ser
85 90 95

Cys Ala Asp Leu Leu Thr Ile Ala Ala Gln Gln Ser Val Thr Leu Ala
100 105 110

Gly Gly Pro Ser Trp Arg Val Pro Leu Gly Arg Arg Asp Ser Leu Gln
115 120 125

Ala Phe Leu Asp Leu Ala Asn Ala Asn Leu Pro Ala Pro Phe Phe Thr
130 135 140

Leu Pro Gln Leu Lys Asp Ser Phe Arg Asn Val Gly Leu Asn Arg Ser
145 150 155 160

Ser Asp Leu Val Ala Leu Ser Gly Gly His Thr Phe Gly Lys Asn Gln
165 170 175

Cys Arg Phe Ile Met Asp Arg Leu Tyr Asn Phe Ser Asn Thr Gly Leu
180 185 190

Pro Asp Pro Thr Leu Asn Thr Thr Tyr Leu Gln Thr Leu Arg Gly Leu
195 200 205

Cys Pro Leu Asn Gly Asn Leu Ser Ala Leu Val Asp Phe Asp Leu Arg
210 215 220

Thr Pro Thr Ile Phe Asp Asn Lys Tyr Tyr Val Asn Leu Glu Glu Gln
225 230 235 240

Lys Gly Leu Ile Gln Ser Asp Gln Glu Leu Phe Ser Ser Pro Asn Ala
245 250 255

Thr Asp Thr Ile Pro Leu Val Arg Ser Phe Ala Asn Ser Thr Gln Thr
260 265 270

Phe Phe Asn Ala Phe Val Glu Ala Met Asp Arg Met Gly Asn Ile Thr
275 280 285

Pro Leu Thr Gly Thr Gln Gly Gln Ile Arg Leu Asn Cys Arg Val Val
290 295 300

Asn Ser
305

<210> 4
<211> 106
<212> PRT
<213> Escherichia coli

<400> 4

Ala Asp Leu Glu Asp Asn Met Glu Thr Leu Asn Asp Asn Leu Lys Val
1 5 10 15

Ile Glu Lys Ala Asp Asn Ala Ala Gln Val Lys Asp Ala Leu Thr Lys
20 25 30

Met Arg Ala Ala Ala Leu Asp Ala Gln Lys Ala Thr Pro Pro Lys Leu
35 40 45

Glu Asp Lys Ser Pro Asp Ser Pro Glu Met Lys Asp Phe Arg Cys Gly
50 55 60

Phe Asp Ile Leu Val Gly Gln Ile Asp Asp Ala Leu Lys Leu Ala Asn
65 70 75 80

Glu Gly Lys Val Lys Glu Ala Gln Ala Ala Ala Glu Gln Leu Lys Thr
85 90 95

Thr Arg Asn Ala Tyr His Gln Lys Tyr Arg
100 105

<210> 5
<211> 20
<212> DNA
<213> artificial

<220>
<223> primer for mutant insertion

<400> 5
aagatttccg ctgcggtttc 20

<210> 6
<211> 17
<212> DNA
<213> artificial

<220>
<223> M13 primer M4

<400> 6
gttttcccag tcacgac 17

<210> 7
<211> 17
<212> DNA
<213> artificial

<220>
<223> M13 primer RV

<400> 7
caggaaacag ctatgac 17

<210> 8
<211> 20
<212> DNA
<213> artificial

<220>
<223> MUT4 primer

<400> 8
ggccagtgcc tagcttacat 20

<210> 9
<211> 141
<212> PRT
<213> Homo sapiens

<400> 9

Val Leu Ser Pro Ala Asp Lys Thr Asn Val Lys Ala Ala Trp Gly Lys
1 5 10 15

Val Gly Ala His Ala Gly Glu Tyr Gly Ala Glu Ala Leu Glu Arg Met
20 25 30

Phe Leu Ser Phe Pro Thr Thr Lys Thr Tyr Phe Pro His Phe Asp Leu
35 40 45

Ser His Gly Ser Ala Gln Val Lys Gly His Gly Lys Lys Val Ala Asp
50 55 60

Ala Leu Thr Asn Ala Val Ala His Val Asp Asp Met Pro Asn Ala Leu

65 70 75 80

Ser Ala Leu Ser Asp Leu His Ala His Lys Leu Arg Val Asp Pro Val
85 90 95

Asn Phe Lys Leu Leu Ser His Cys Leu Leu Val Thr Leu Ala Ala His
100 105 110

Leu Pro Ala Glu Phe Thr Pro Ala Val His Ala Ser Leu Asp Lys Phe
115 120 125

Leu Ala Ser Val Ser Thr Val Leu Thr Ser Lys Tyr Arg
130 135 140

<210> 10
<211> 146
<212> PRT
<213> Homo sapiens

<400> 10

Val His Leu Thr Pro Glu Glu Lys Ser Ala Val Thr Ala Leu Trp Gly
1 5 10 15

Lys Val Asn Val Asp Glu Val Gly Gly Glu Ala Leu Gly Arg Leu Leu
20 25 30

Val Val Tyr Pro Trp Thr Gln Arg Phe Phe Glu Ser Phe Gly Asp Leu
35 40 45

Ser Thr Pro Asp Ala Val Met Gly Asn Pro Lys Val Lys Ala His Gly
50 55 60

Lys Lys Val Leu Gly Ala Phe Ser Asp Gly Leu Ala His Leu Asp Asn
65 70 75 80

Leu Lys Gly Thr Phe Ala Thr Leu Ser Glu Leu His Cys Asp Lys Leu
85 90 95

His Val Asp Pro Glu Asn Phe Arg Leu Leu Gly Asn Val Leu Val Cys
100 105 110

Val Leu Ala His His Phe Gly Lys Glu Phe Thr Pro Pro Val Gln Ala
115 120 125

Ala Tyr Gln Lys Val Val Ala Gly Val Ala Asn Ala Leu Ala His Lys
130 135 140

Tyr His
145